# EUROPÄISCHE PATENTSCHRIFT

(11) **EP 1 774 954 B1**
(45) Veröffentlichungstag und Bekanntmachung des Hinweises auf die Patenterteilung: **13.08.2014**
(21) Anmeldenummer: 06011570.6
(22) Anmeldetag: 03.06.2006
(51) Int. Cl.: A61K 8/34, A61K 8/42, A61K 8/97, A61Q 11/00

(54) **Mund-und Zahnpflege-und Reinigunsmittel**
Mouthwash and dentifrice and cleansing composition
Collutoire et dentifrice et composition nettoyante

(30) Priorität: 17.10.2005 DE 102005049972
(43) Veröffentlichungstag der Anmeldung: 18.04.2007
(73) Patentinhaber: Henkel AG & Co. KGaA, 40589 Düsseldorf (DE)
(72) Erfinder: Bernecker, Ullrich, 52074 Aachen (DE); Barth, Adolf Peter, 42781 Haan (DE)

(56) Entgegenhaltungen:
- EP-A- 1 323 356
- WO-A-2004/000787
- WO-A-2004/015044
- WO-A-2004/043906
- WO-A-2004/073672
- DE-A- 19 730 651
- US-A- 3 720 762
- US-A- 5 407 665
- US-A- 5 942 211
- US-A1- 2004 166 172
- US-A1- 2004 247 535
- US-B1- 6 190 643

## Beschreibung

Die Erfindung betrifft Zubereitungen zur Mund- und Zahnpflege- und -reinigung, die aufgrund von speziellen Inhaltsstoffen ein besonderes Gefühl im Mund bewirken.

Es ist bekannt, Zubereitungen zur Mund- und Zahnpflege- und -reinigung Geruchs- und/oder Geschmacksstoffe bzw. Aromen zuzusetzen, um ihnen einerseits einen besonderen Geruch bzw. Geschmack zu verleihen und andererseits um ein aromatisches Gefühl von Frische nach der Anwendung zu hinterlassen. Solche Aromen usw. sind im Bereich der Mund- und Zahnpflege und -reinigung weit verbreitet. In speziellen Zubereitungen ist auch der Einsatz von sogenannten Scharfstoffen bereits bekannt, um einen sensorisch interessanten und lang anhaltenden Geschmack und/oder Frischeeffekt zu bewirken. So offenbart die GB 1,438, 205 Zahnpasten oder Mundwässer, welche N-isobutyl-2,6,8-Decatrienamid enthalten, das in Mengen um 0,01 Gew.%, bezogen auf die Zubereitung, eingesetzt wird.

Der Einsatz anderer Scharfstoffe aus der Gruppe der Alkadienamide wird beispielsweise in der DE 103 51 422 A1 offenbart.

Die EP 1 323 356 A1, die WO 2004/00787 A1 sowie die WO 2004/043906 A1 offenbaren Aromastoffe bzw. -mischungen mit Scharfstoffen, welche in Kaugummis oder Mundwässern eingesetzt werden.
Alle drei Dokumente offenbaren weder explizit Zahnpasten, noch den Einsatz von Fluoriden in Zahnpasten, welche bestimmte Scharfstoffe in bestimmten Mengen enthalten.

Es hat sich gezeigt, dass der gewünschte scharfe, kribbelnde, mundwässernde oder wärmeerzeugende Effekt von einem Großteil der Verbraucher als unangenehm und zu stark empfunden wird, das aber die Reduzierung in der Einsatzkonzentration des Scharfstoffes zu verringerter Wahrnehmung des Effektes führt. Es bestand daher nach wie vor der Wunsch nach Zubereitungsformen, die ein sensorisch einzigartiges Empfinden hervorrufen, das vom Verbraucher akzeptiert und nicht als unangenehm empfunden wird.

Überraschenderweise wurde nun festgestellt, dass Zubereitungen, die (einen) mehrwertige(n) Alkohol(e) und Wasser enthalten und bei denen das Verhältnis dieser beiden Stoffe innerhalb spezieller Grenzen liegt, dem Anwender ein angenehmes Gefühl von Wärme und Schärfe vermitteln, ohne dass dieses Gefühl als unangenehm empfunden wird.

Gegenstand der vorliegenden Erfindung sind demnach Mund- und Zahnpflege- und -reinigungsmittel, enthaltend
a) mindestens einen mehrwertigen Alkohol aus der Gruppe Sorbit und/oder Glycerin und/oder 1,2-Propylenglycol;
b) Wasser;
a) mindestens eine scharf schmeckende und/oder ein Gefühl von Wärme erzeugende Substanz aus der Gruppe der N-Alkyl-substituierte Amide von ungesättigten Carbonsäuren
c) 0,01 bis 5 Gew.% Fluorverbindung(en),
wobei das Gewichtsverhältnis von b) zu a) größer als 1,5 : 1 ist.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahn-reinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Mund- und Zahnpflege- und -reinigungsmittel enthalten als ersten wesentlichen Inhaltsstoff mindestens einen mehrwertigen Alkohol aus der Gruppe Sorbit bzw. Glycerin bzw. 1,2-Propylenglycol.

Hier sind erfindungsgemäße Mund- und Zahnpflege- und -reinigungsmittel bevorzugt, die 10 bis 40 Gew.-%, vorzugsweise 12,5 bis 35 Gew.-%, besonders bevorzugt 15 bis 30 Gew.-% und insbesondere 20 bis 27,5 Gew.-% mindestens eines mehrwertigen Alkohols aus der Gruppe Sorbit und/oder Glycerin und/oder 1,2-Propylenglycol.-%, jeweils bezogen auf das Gewichts des gesamten Mittels, enthalten.

Neben Sorbit bzw. Glycerin bzw. 1,2-Propylenglycol, von denen mindestens ein Stoff in den erfindungsgemäßen Mitteln enthalten ist, eignen sich als weitere mehwertige Alkohole solche mit mindestens 2 OH-Gruppen, vorzugsweise Mannit, Xylitol, Polyethylenglycol, Polypropylenglycol und deren Mischungen.

Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind. Zwar werden bei der Berechnung des Verhältnisses von b) zu a) werden gegebenenfalls enthaltene andere mehrwertige Alkohole nicht berücksichtigt, doch sind erfindungsgemäße Mittel besonders bevorzugt, die entweder keine anderen mehrwertigen Alkohole enthalten, oder bei denen das Verhältnis von Wasser zu allen im Mittel enthaltenen mehrwertigen Alkoholen größer als 1,5 : 1 ist.

Als zweiten wesentlichen Bestandteil enthalten die erfindungsgemäßen Zusammensetzungen Wasser. Hier sind erfindungsgemäße Mund- und Zahnpflege- und -reinigungsmittel bevorzugt, die 30 bis 70 Gew.-%, vorzugsweise 36 bis 65 Gew.-%, besonders bevorzugt 41 bis 60 Gew.-% und insbesondere 45 bis 55 Gew.-% Wasser.-%, jeweils bezogen auf das Gewichts des gesamten Mittels, enthalten.

In die Berechnung des Gesamt-Wassergehaltes der Mittel fließen die Wasseranteile wäßriger Lösungen selbstverständlich mit ein. Wird also beispielsweise Sorbit in Form einer 70 Gew.-%-igen Lösung eingesetzt, so beträgt der Sorbit-Anteil das 0,7-fache des eingesetzten Gewichtsanteils, während der Wasseranteil um das 0,3-fache des eingesetzten Gewichtsanteils erhöht wird. Analog ist auch mit wäßrigen Lösungen von Farb- oder Aromastoffen usw. zu verfahren.

Erfindungsgemäß ist das Verhältnis von Wasser zu dem bzw. den Alkohol(en) aus der Gruppe Sorbit und/oder Glycerin und/oder 1,2-Propylenglycol (und vorzugsweise zu allen gegebenenfalls im Mittel enthaltenen mehrwertigen Alkoholen) größer als 1,5 : 1. Bevorzugte Mittel weisen ein Verhältnis von > 1,6 : 1 auf, weiter bevorzugte Mittel ein Verhältnis von > 1,65 : 1, weiter bevorzugte Mittel ein Verhältnis von > 1,7 : 1, weiter bevorzugte Mittel ein Verhältnis von > 1,75 : 1, weiter bevorzugte Mittel ein Verhältnis von > 1,8 : 1, weiter bevorzugte Mittel ein Verhältnis von > 1,85 : 1, weiter bevorzugte Mittel ein Verhältnis von > 1,9 : 1, weiter bevorzugte Mittel ein Verhältnis von > 1,95 : 1, weiter bevorzugte Mittel ein Verhältnis von > 2,0 : 1, weiter bevorzugte Mittel ein Verhältnis von > 2,05 : 1, weiter bevorzugte Mittel ein Verhältnis von > 2,1 : 1 und insbesondere bevorzugte Mittel ein Verhältnis von > 2,15 : 1.

Als dritten wesentlichen Inhaltsstoff enthalten die erfindungsgemäßen Mund- und Zahnpflege- und -reinigungsmittel eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein PrickeIn, Perlen, Kitzeln oder Sprudeln hervor.

Erfindungsgemäß bevorzugte Mund- und Zahnpflege- und -reinigungsmittel enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,5 Gew.-% und insbesondere von 0,01 bis 0,2 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Erfindungsgemäß eingesetzt werden N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1 -Methylpropyl)-amid
- 2E,4E-Decadiensäure-N-Isobutylamid
- 2E,4E-Decadiensäure-N-tert-Butylamid

- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid

- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid

- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid

- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid

- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid

- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid
- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid

- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid

- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid

- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:

2E,6Z,8E-Decatriensäure-N-isobutylamid (N-Isobutyl-2E,6Z,8E-decatrienamid, auch Spilanthol oder Affinin genannt):

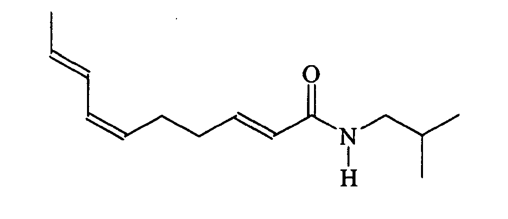

2E,4E,8Z-Decatriensäure-N-isobutylamid (N-Isobutyl-2E,4E,8Z-decatrienamid, auch Isoaffinin genannt):

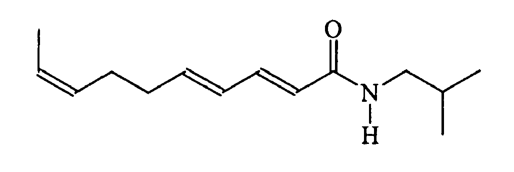

2E,7Z,9E-Undecatriensäure-N-isobutylamid (N-Isobutyl-2E,7Z,9E-undecatrienamid):

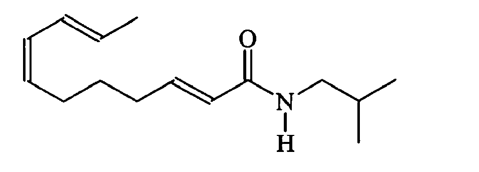

2E,4Z-Decadiensäure-N-isobutylamid (cis-Pellitorin):

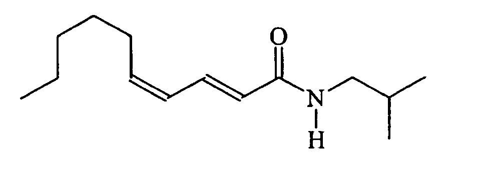

2E,4E-Decadiensäure-N-isobutylamid (trans-Pellitorin):

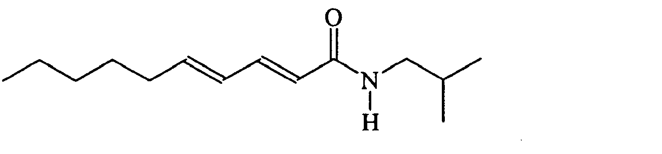

Ferulasäureamide, beispielsweise

Ferulasäure-N-Vanillylamid:

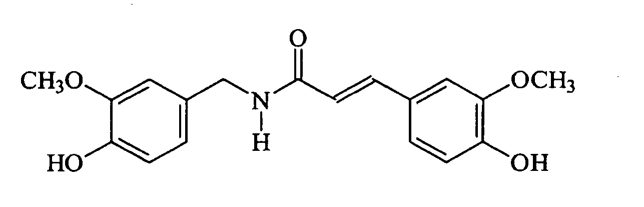

*N*-[2-(4-Hydroxy-3-methoxyphenyl)ethyl]-3-(4-hydroxy-3-methoxy-phenyl)- (2*E*)-propensäureamid (trans-Feruloylmethoxytyramin):

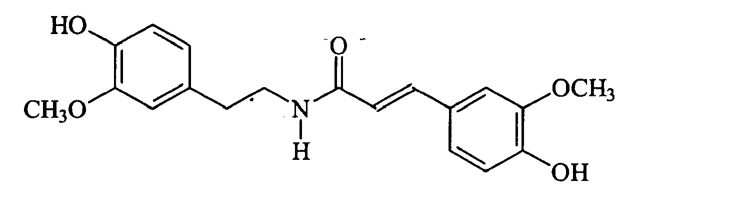

*N*-[2- (4-Hydroxy-3-methoxyphenyl)ethyl]-3-(4-hydroxy-3-methoxy-phenyl)-(2 Z)-propensäureamid (cis-Feruloylmethoxytyramin):

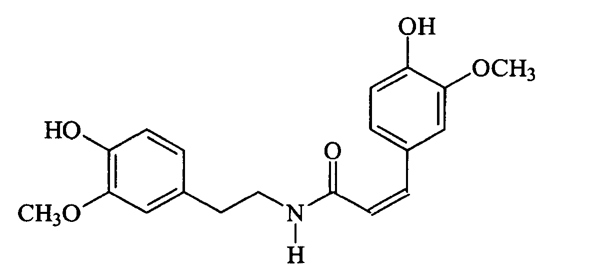

N-[2-(4- Hydroxy-3-methoxyphenyl)ethyl]-3-(4-hydroxy-3-methoxy-phenyl)- propansäureamid (Dihydroferuloylmethoxytyramin):

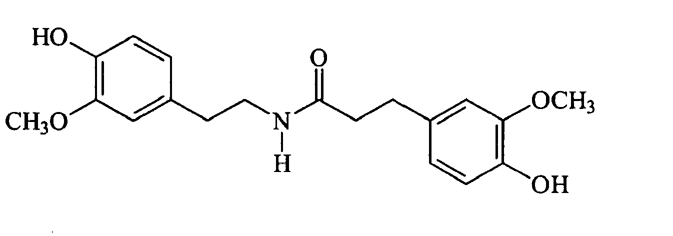

*N*-[2-(3,4- Dihydroxyphenyl)ethyl]-3-(4-hydroxy-3-methoxy-phenyl)-(2*E*)- propensäureamid (trans-Feruloyldopamin):

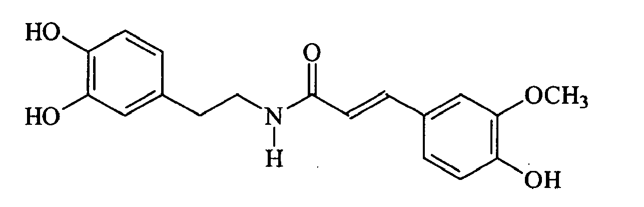

N-[2-(3,4-Dihydroxyphenyl) ethyl]-3-(4-hydroxy-3-methoxy-phenyl)-(2Z)-propensäureamid (cis-Feruloyldopamin):

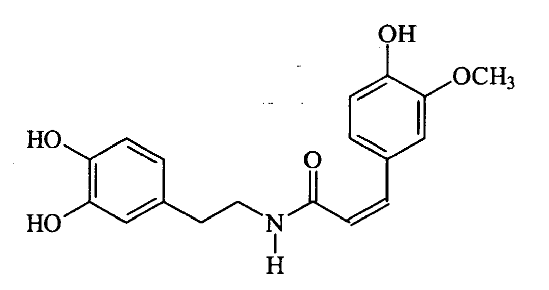

N-[2-(4-Hydroxyphenyl)ethyl]-3-(3,4- dihydroxyphenyl)-(2E)-propensäureamid (trans-Caffeoyltyramin):

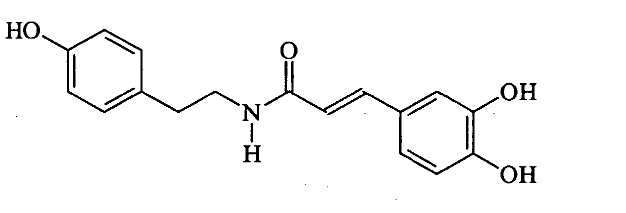

*N*-[2-(4-Hydroxyphenyl)ethyl]-3-(3,4-dihydroxyphenyl)-(2*Z*)- propensäureamid (cis-Caffeoyltyramin):

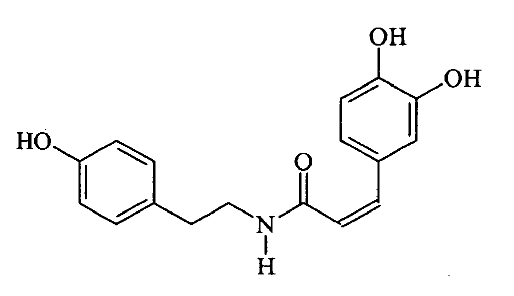

N-[2-(3,4-Dimethoxyphenyl) ethyl]-3-(3,4-dimethoxyphenyl)-(2*E*)-propensäureamid (trans-Rubenamin):

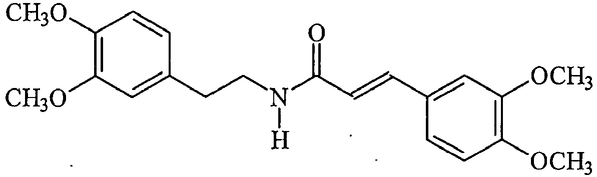

### N-[2-(3,4-dimethoxyphenyl)ethyl]-3-(3,4- dimethoxy-phenyl)-(2Z)-propensäureamid (cis-Rubenamin):

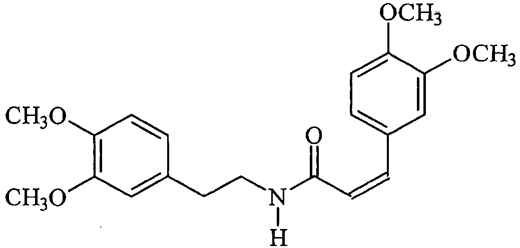

Weitere im Rahmen der vorliegenden Erfindung mit besonderem Vorzug zusätzlich zu den N-Alkyl-substituierte Amiden von ungesättigten Carbonsäuren einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*)*,* Extrakte aus *Allium ssp*.(insbesondere Zwiebel und Knoblauchextrakte), Extrakte aus Rettich (*Raphanus ssp.*)*,* Meerrettichextrakte (*Cochlearia armoracia*)*,* Extrakte aus schwarzem (*Brassica nigra*)*,* wildem oder gelbem Senf (*Sinapis ssp.,* insbesondere *Sinapis arvensis* und *Sinapis alba*)*,* Bertramwurzel-Extrakte (*Anacyclus ssp.,* insbesondere *Anacyclus pyrethrumL*.), Sonnenhutextrakte (*Echinaceae ssp*.), Extrakten aus Szechuan-Pfeffer (*Zanthoxylum ssp.,* insbesondere *Zanthoxylum piperitum*)*,* Spilanthesextrakt (*Spilanthes ssp.,* insbesondere *Spilanthes acmella*)*,* Chiliextrakt (*Capsicum ssp.,* insbesondere *Capsicum frutescens* ), Paradieskörner-Extrakt ( *Aframomum* ssp.,insbesondere *Aframomum melegueta*[Rose] K. Schum.),Ingwerextrakt (*Zingiber* ssp.,insbesondere *Zingiber officinale*) und Galangaextrakt (*Kaempferia galanga* oder *Alpinia galanga*)*.*

Eine besonders geeignete Substanz ist das aus dem Ingwerextrakt stammende Gingerol:

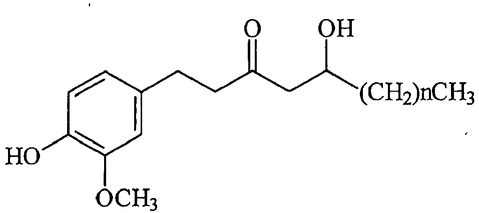

Einsetzbar ist auch N-Ethyl-p-menthan-3-carboxamid (N-Ethyl-5-Methyl-2-isopropylcyclohexancarboxamid):

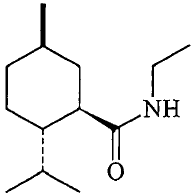

Andere zusätzlich zu den N-Alkyl-substituierte Amiden von ungesättigten Carbonsäuren einsetzbare scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.
Besonders bevorzugte erfindungsgemäße Mund- und Zahnpflege- und -reinigungsmittel sind demnach dadurch gekennzeichnet, dass sie mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- *N*-[2-(4-Hydroxy-3-methoxyphenyl)ethyl]-3-(4-hydroxy-3-methoxy-phenyl)- (2*E*)-propensäureamid (trans-Feruloylmethoxytyramin) und/oder
- *N*-[2- (4-Hydroxy-3-methoxyphenyl)ethyl]-3-(4-hydroxy-3-methoxy-phenyl)-(2 *Z*)-propensäureamid (cis-Feruloylmethoxytyramin) und/oder
- *N*-[2-(4-Hydroxy-3-methoxyphenyl)ethyl]-3-(4-hydroxy-3-methoxy-phenyl)-propansäureamid (Dihydroferuloylmethoxytyramin) und/oder
- *N*-[2-(3,4- Dihydroxyphenyl)ethyl]-3-(4-hydroxy-3-methoxy-phenyl)-(2*E*)-propensäureamid (trans-Feruloyldopamin) und/oder
- *N*-[2-(3,4-Dihydroxyphenyl) ethyl]-3-(4-hydroxy-3-methoxy-phenyl)-(2*Z*)-propensäureamid (cis- Feruloyldopamin) und/oder
- *N*-[2-(4-Hydroxyphenyl)ethyl]-3-(3,4- dihydroxyphenyl)-(*2E*)-propensäureamid (trans-Caffeoyltyramin) und/oder
- *N*-[2-(4-Hydroxyphenyl)ethyl]-3-(3,4-dihydroxyphenyl)-(2*Z*)- propensäureamid (cis-Caffeoyltyramin) und/oder
- *N*-[2-(3,4-Dimethoxyphenyl) ethyl]-3-(3,4-dimethoxyphenyl)-(2*E*)-propensäureamid (trans- Rubenamin) und/oder
- *N*-[2-(3,4-dimethoxyphenyl)ethyl]-3-(3,4- dimethoxy-phenyl)-(2*Z*)-propensäureamid (cis-Rubenamin)
enthalten

Zusätzlich zu den genannten Scharfstoffen können auch weitere scharf schmeckende und/oder ein Gefühl von Wärme erzeugende Substanzen in die erfindungsgemäßen Mund- und Zahnpflege- und -reinigungsmittel eingearbeitet werden.

Als besonders geeignete zusätzlich zu den N-Alkyl-substituierte Amiden von ungesättigten Carbonsäuren einsetzbare Verbindungen haben sich im Rahmen der vorliegenden Erfindung alkylsubstituierte Dioxane der Formel 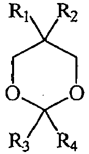 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃,-CH(CH₃)₂. Einzelne Spezies, die der vorstehend genannten Formel genügen, sind hinsichtlich ihrer Reste R1, R2, R3 und R4 in der nachstehenden Tabelle 1 aufgeführt. Diese sind erfindungsgemäß besonders bevorzugt.

**Tabelle 1: Bevorzugte alkylsubstituierte Dioxane:**

| **Nr.** | **R1** | **R2** | **R3** | **R4** |
|---|---|---|---|---|
| 1 | -H | -CH₃ | -H | -H |
| 2 | -H | -CH₂CH₃ | -H | -H |
| 3 | -CH₃ | -CH₃ | -H | -H |
| 4 | -CH₃ | -CH₂CH₃ | -H | -H |
| 5 | -CH₂CH₃ | -CH₂CH₃ | -H | -H |
| 6 | -H | -CH₃ | -H | -CH₃ |
| 7 | -H | -CH₃ | -H | -CH₂CH₃ |
| 8 | -H | -CH₃ | -H | -CH₂CH₂CH₃ |
| 9 | -H | -CH₃ | -H | -CH(CH₃)₂ |
| 10 | -H | -CH₃ | -CH₃ | -CH₃ |
| 11 | -H | -CH₃ | -CH₃ | -CH₂CH₃ |
| 12 | -H | -CH₃ | -CH₃ | -CH₂CH₂CH₃ |
| 13 | -H | -CH₃ | -CH₃ | -CH(CH₃)₂ |
| 14 | -H | -CH₃ | -CH₂CH₃ | -CH₂CH₃ |
| 15 | -H | -CH₃ | -CH₂CH₃ | -CH₂CH₂CH₃ |
| 16 | -H | -CH₃ | -CH₂CH₃ | -CH(CH₃)₂ |
| 17 | -H | -CH₃ | -CH₂CH₂CH₃ | -CH₂CH₂CH₃ |
| 18 | -H | -CH₃ | -CH₂CH₂CH₃ | -CH(CH₃)₂ |
| 19 | -H | -CH₃ | -CH(CH₃)₂ | -CH(CH₃)₂ |
| 20 | -H | -CH₂CH₃ | -H | -CH₃ |
| 21 | -H | -CH₂CH₃ | -H | -CH₂CH₃ |
| 22 | -H | -CH₂CH₃ | -H | -CH₂CH₂CH₃ |
| 23 | -H | -CH₂CH₃ | -H | -CH(CH₃)₂ |
| 24 | -H | -CH₂CH₃ | -CH₃ | -CH₃ |
| 25 | -H | -CH₂CH₃ | -CH₃ | -CH₂CH₃ |
| 26 | -H | -CH₂CH₃ | -CH₃ | -CH₂CH₂CH₃ |
| 27 | -H | -CH₂CH₃ | -CH₃ | -CH(CH₃)₂ |
| 28 | -H | -CH₂CH₃ | -CH₂CH₃ | -CH₂CH₃ |
| 29 | -H | -CH₂CH₃ | -CH₂CH₃ | -CH₂CH₂CH₃ |
| 30 | -H | -CH₂CH₃ | -CH₂CH₃ | -CH(CH₃)₂ |
| 31 | -H | -CH₂CH₃ | -CH₂CH₂CH₃ | -CH₂CH₂CH₃ |
| 32 | -H | -CH₂CH₃ | -CH₂CH₂CH₃ | -CH(CH₃)₂ |
| 33 | -H | -CH₂CH₃ | -CH(CH₃)₂ | -CH(CH₃)₂ |
| 34 | -CH₃ | -CH₃ | -H | -CH₃ |
| 35 | -CH₃ | -CH₃ | -H | -CH₂CH₃ |
| 36 | -CH₃ | -CH₃ | -H | -CH₂CH₂CH₃ |
| 37 | -CH₃ | -CH₃ | -H | -CH(CH₃)₂ |
| 38 | -CH₃ | -CH₃ | -CH₃ | -CH₃ |
| 39 | -CH₃ | -CH₃ | -CH₃ | -CH₂CH₃ |
| 40 | -CH₃ | -CH₃ | -CH₃ | -CH₂CH₂CH₃ |
| 41 | -CH₃ | -CH₃ | -CH₃ | -CH(CH₃)₂ |
| 42 | -CH₃ | -CH₃ | -CH₂CH₃ | -CH₂CH₃ |
| 43 | -CH₃ | -CH₃ | -CH₂CH₃ | -CH₂CH₂CH₃ |
| 44 | -CH₃ | -CH₃ | -CH₂CH₃ | -CH(CH₃)₂ |
| 45 | -CH₃ | -CH₃ | -CH₂CH₂CH₃ | -CH₂CH₂CH₃ |
| 46 | -CH₃ | -CH₃ | -CH₂CH₂CH₃ | -CH(CH₃)₂ |
| 47 | -CH₃ | -CH₃ | -CH(CH₃)₂ | -CH(CH₃)₂ |
| 48 | -CH₃ | -CH₂CH₃ | -H | -CH₃ |
| 49 | -CH₃ | -CH₂CH₃ | -H | -CH₂CH₃ |
| 50 | -CH₃ | -CH₂CH₃ | -H | -CH₂CH₂CH₃ |
| 51 | -CH₃ | -CH₂CH₃ | -H | -CH(CH₃)₂ |
| 52 | -CH₃ | -CH₂CH₃ | -CH₃ | -CH₃ |
| 53 | -CH₃ | -CH₂CH₃ | -CH₃ | -CH₂CH₃ |
| 54 | -CH₃ | -CH₂CH₃ | -CH₃ | -CH₂CH₂CH₃ |
| 55 | -CH₃ | -CH₂CH₃ | -CH₃ | -CH(CH₃)₂ |
| 56 | -CH₃ | -CH₂CH₃ | -CH₂CH₃ | -CH₂CH₃ |
| 57 | -CH₃ | -CH₂CH₃ | -CH₂CH₃ | -CH₂CH₂CH₃ |
| 58 | -CH₃ | -CH₂CH₃ | -CH₂CH₃ | -CH(CH₃)₂ |
| 59 | -CH₃ | -CH₂CH₃ | -CH₂CH₂CH₃ | -CH₂CH₂CH₃ |
| 60 | -CH₃ | -CH₂CH₃ | -CH₂CH₂CH₃ | -CH(CH₃)₂ |
| 61 | -CH₃ | -CH₂CH₃ | -CH(CH₃)₂ | -CH(CH₃)₂ |
| 62 | -CH₂CH₃ | -CH₂CH₃ | -H | -CH₃ |
| 63 | -CH₂CH₃ | -CH₂CH₃ | -H | -CH₂CH₃ |
| 64 | -CH₂CH₃ | -CH₂CH₃ | -H | -CH₂CH₂CH₃ |
| 65 | -CH₂CH₃ | -CH₂CH₃ | -H | -CH(CH₃)₂ |
| 66 | -CH₂CH₃ | -CH₂CH₃ | -CH₃ | -CH₃ |
| 67 | -CH₂CH₃ | -CH₂CH₃ | -CH₃ | -CH₂CH₃ |
| 68 | -CH₂CH₃ | -CH₂CH₃ | -CH₃ | -CH₂CH₂CH₃ |
| 69 | -CH₂CH₃ | -CH₂CH₃ | -CH₃ | -CH(CH₃)₂ |
| 70 | -CH₂CH₃ | -CH₂CH₃ | -CH₂CH₃ | -CH₂CH₃ |
| 71 | -CH₂CH₃ | -CH₂CH₃ | -CH₂CH₃ | -CH₂CH₂CH₃ |
| 72 | -CH₂CH₃ | -CH₂CH₃ | -CH₂CH₃ | -CH(CH₃)₂ |
| 73 | -CH₂CH₃ | -CH₂CH₃ | -CH₂CH₂CH₃ | -CH₂CH₂CH₃ |
| 74 | -CH₂CH₃ | -CH₂CH₃ | -CH₂CH₂CH₃ | -CH(CH₃)₂ |
| 75 | -CH₂CH₃ | -CH₂CH₃ | -CH(CH₃)₂ | -CH(CH₃)₂ |

Als weiterhin besonders geeignete zusätzlich zu den N-Alkyl-substituierte Amiden von ungesättigten Carbonsäuren einsetzbare Verbindungen haben sich im Rahmen der vorliegenden Erfindung Phenylester der Formel 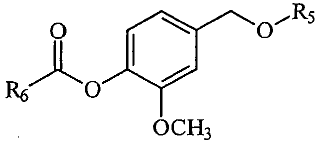 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Einzelne Spezies, die der vorstehend genannten Formel genügen, sind hinsichtlich ihrer Reste R5 und R6 in der nachstehenden Tabelle 2 aufgeführt. Diese sind erfindungsgemäß besonders bevorzugt.

**Tabelle 2: Bevorzugte Phenylester**

| **Nr.** | **R5** | **R6** |
|---|---|---|
| 1 | -CH₃ | -CH₃ |
| 2 | -CH₂CH₃ | -CH₃ |
| 3 | -CH₂CH₂CH₃ | -CH₃ |
| 4 | -CH(CH₃)₂ | -CH₃ |
| 5 | -CH₂CH₂CH₂CH₃ | -CH₃ |
| 6 | -CH(CH₃)CH₂CH₃ | -CH₃ |
| 7 | -CH₂CH(CH₃)₂ | -CH₃ |
| 8 | -C(CH₃)₃ | -CH₃ |
| 9 | -CH₂(CH₂)₃CH₃ | -CH₃ |
| 10 | -CH(CH₃)(CH₂)₂CH₃ | -CH₃ |
| 11 | -CH₂CH(CH₃)CH₂CH₃ | -CH₃ |
| 12 | -(CH₂)₂CH(CH₃)₂ | -CH₃ |
| 13 | -CH₂(CH₂)₄CH₃ | -CH₃ |
| 14 | -CH₂(CH₂)₅CH₃ | -CH₃ |
| 15 | -CH₂(CH₂)₆CH₃ | -CH₃ |
| 16 | -CH=CH₂ | -CH₃ |
| 17 | -CH=CHCH₃ | -CH₃ |
| 18 | -CH=C(CH₃)₂ | -CH₃ |
| 19 | -C(CH₃)=CH₂ | -CH₃ |
| 20 | -C(CH₃)=CHCH₃ | -CH₃ |
| 21 | -C(CH₃)=C(CH₃)₂ | -CH₃ |
| 22 | -CH=CH-CH₂CH₃ | -CH₃ |
| 23 | -CH₂CH=CH-CH₃ | -CH₃ |
| 24 | -(CH₂)₂CH=CH₂ | -CH₃ |
| 25 | -CH=CH-(CH₂)₂CH₃ | -CH₃ |
| 26 | -CH₂CH=CH-CH₂CH₃ | -CH₃ |
| 27 | -(CH₂)₂CH=CH-CH₃ | -CH₃ |
| 28 | -(CH₂)₃CH=CH₂ | -CH₃ |
| 29 | -CH=CH-(CH₂)₃CH₃ | -CH₃ |
| 30 | -CH₂CH=CH-(CH₂)₂CH₃ | -CH₃ |
| 31 | -(CH₂)₂CH=CH-CH₂CH₃ | -CH₃ |
| 32 | -(CH₂)₃CH=CH-CH₃ | -CH₃ |
| 33 | -(CH₂)₄CH=CH₂ | -CH₃ |
| 34 | -CH=CH-(CH₂)₃CH₃ | -CH₃ |
| 35 | -CH₂CH=CH-(CH₂)₃CH₃ | -CH₃ |
| 36 | -(CH₂)₂CH=CH-(CH₂)₂CH₃ | -CH₃ |
| 37 | -(CH₂)₃CH=CH-CH₂CH₃ | -CH₃ |
| 38 | -(CH₂)₄CH=CH-CH₃ | -CH₃ |
| 39 | -(CH₂)₅CH=CH₂ | -CH₃ |
| 40 | -CH=CH-(CH₂)₅CH₃ | -CH₃ |
| 41 | -CH₂CH=CH-(CH₂)₄CH₃ | -CH₃ |
| 42 | -(CH₂)₂CH=CH-(CH₂)₃CH₃ | -CH₃ |
| 43 | -(CH₂)₃CH=CH-(CH₂)₂CH₃ | -CH₃ |
| 44 | -(CH₂)₄CH=CH-CH₂CH₃ | -CH₃ |
| 45 | -(CH₂)₅CH=CH-CH₃ | -CH₃ |
| 46 | -(CH₂)₆CH=CH₂ | -CH₃ |
| 47 | -CH₃ | -CH₂CH₃ |
| 48 | -CH₂CH₃ | -CH₂CH₃ |
| 49 | -CH₂CH₂CH₃ | -CH₂CH₃ |
| 50 | -CH(CH₃)₂ | -CH₂CH₃ |
| 51 | -CH₂CH₂CH₂CH₃ | -CH₂CH₃ |
| 52 | -CH(CH₃)CH₂CH₃ | -CH₂CH₃ |
| 53 | -CH₂CH(CH₃)₂ | -CH₂CH₃ |
| 54 | -C(CH₃)₃ | -CH₂CH₃ |
| 55 | -CH₂(CH₂)₃CH₃ | -CH₂CH₃ |
| 56 | -CH(CH₃)(CH₂)₂CH₃ | -CH₂CH₃ |
| 57 | -CH₂CH(CH₃)CH₂CH₃ | -CH₂CH₃ |
| 58 | -(CH₂)₂CH(CH₃)₂ | -CH₂CH₃ |
| 59 | -CH₂(CH₂)₄CH₃ | -CH₂CH₃ |
| 60 | -CH₂(CH₂)₅CH₃ | -CH₂CH₃ |
| 61 | -CH₂(CH₂)₆CH₃ | -CH₂CH₃ |
| 62 | -CH=CH₂ | -CH₂CH₃ |
| 63 | -CH=CHCH₃ | -CH₂CH₃ |
| 64 | -CH=C(CH₃)₂ | -CH₂CH₃ |
| 65 | -C(CH₃)=CH₂ | -CH₂CH₃ |
| 66 | -C(CH₃)=CHCH₃ | -CH₂CH₃ |
| 67 | -C(CH₃)=C(CH₃)₂ | -CH₂CH₃ |
| 68 | -CH=CH-CH₂CH₃ | -CH₂CH₃ |
| 69 | -CH₂CH=CH-CH₃ | -CH₂CH₃ |
| 70 | -(CH₂)₂CH=CH₂ | -CH₂CH₃ |
| 71 | -CH=CH-(CH₂)₂CH₃ | -CH₂CH₃ |
| 72 | -CH₂CH=CH-CH₂CH₃ | -CH₂CH₃ |
| 73 | -(CH₂)₂CH=CH-CH₃ | -CH₂CH₃ |
| 74 | -(CH₂)₃CH=CH₂ | -CH₂CH₃ |
| 75 | -CH=CH-(CH₂)₃CH₃ | -CH₂CH₃ |
| 76 | -CH₂CH=CH-(CH₂)₂CH₃ | -CH₂CH₃ |
| 77 | -(CH₂)₂CH=CH-CH₂CH₃ | -CH₂CH₃ |
| 78 | -(CH₂)₃CH=CH-CH₃ | -CH₂CH₃ |
| 79 | -(CH₂)₄CH=CH₂ | -CH₂CH₃ |
| 80 | -CH=CH-(CH₂)₃CH₃ | -CH₂CH₃ |
| 81 | -CH₂CH=CH-(CH₂)₃CH₃ | -CH₂CH₃ |
| 82 | -(CH₂)₂CH=CH-(CH₂)₂CH₃ | -CH₂CH₃ |
| 83 | -(CH₂)₃CH=CH-CH₂CH₃ | -CH₂CH₃ |
| 84 | -(CH₂)₄CH=CH-CH₃ | -CH₂CH₃ |
| 85 | -(CH₂)₅CH=CH₂ | -CH₂CH₃ |
| 86 | -CH=CH-(CH₂)₅CH₃ | -CH₂CH₃ |
| 87 | -CH₂CH=CH-(CH₂)₄CH₃ | -CH₂CH₃ |
| 88 | -(CH₂)₂CH=CH-(CH₂)₃CH₃ | -CH₂CH₃ |
| 89 | -(CH₂)₃CH=CH-(CH₂)₂CH₃ | -CH₂CH₃ |
| 90 | -(CH₂)₄CH=CH-CH₂CH₃ | -CH₂CH₃ |
| 91 | -(CH₂)₅CH=CH-CH₃ | -CH₂CH₃ |
| 92 | -(CH₂)₆CH=CH₂ | -CH₂CH₃ |
| 93 | -CH₃ | -CH₂CH₂CH₃ |
| 94 | -CH₂CH₃ | -CH₂CH₂CH₃ |
| 95 | -CH₂CH₂CH₃ | -CH₂CH₂CH₃ |
| 96 | -CH(CH₃)₂ | -CH₂CH₂CH₃ |
| 97 | -CH₂CH₂CH₂CH₃ | -CH₂CH₂CH₃ |
| 98 | -CH(CH₃)CH₂CH₃ | -CH₂CH₂CH₃ |
| 99 | -CH₂CH(CH₃)₂ | -CH₂CH₂CH₃ |
| 100 | -C(CH₃)₃ | -CH₂CH₂CH₃ |
| 101 | -CH₂(CH₂)₃CH₃ | -CH₂CH₂CH₃ |
| 102 | -CH(CH₃)(CH₂)₂CH₃ | -CH₂CH₂CH₃ |
| 103 | -CH₂CH(CH₃)CH₂CH₃ | -CH₂CH₂CH₃ |
| 104 | -(CH₂)₂CH(CH₃)₂ | -CH₂CH₂CH₃ |
| 105 | -CH₂(CH₂)₄CH₃ | -CH₂CH₂CH₃ |
| 106 | -CH₂(CH₂)₅CH₃ | -CH₂CH₂CH₃ |
| 107 | -CH₂(CH₂)₆CH₃ | -CH₂CH₂CH₃ |
| 108 | -CH=CH₂ | -CH₂CH₂CH₃ |
| 109 | -CH=CHCH₃ | -CH₂CH₂CH₃ |
| 110 | -CH=C(CH₃)₂ | -CH₂CH₂CH₃ |
| 111 | -C(CH₃)=CH₂ | -CH₂CH₂CH₃ |
| 112 | -C(CH₃)=CHCH₃ | -CH₂CH₂CH₃ |
| 113 | -C(CH₃)=C(CH₃)₂ | -CH₂CH₂CH₃ |
| 114 | -CH=CH-CH₂CH₃ | -CH₂CH₂CH₃ |
| 115 | -CH₂CH=CH-CH₃ | -CH₂CH₂CH₃ |
| 116 | -(CH₂)₂CH=CH₂ | -CH₂CH₂CH₃ |
| 117 | -CH=CH-(CH₂)₂CH₃ | -CH₂CH₂CH₃ |
| 118 | -CH₂CH=CH-CH₂CH₃ | -CH₂CH₂CH₃ |
| 119 | -(CH₂)₂CH=CH-CH₃ | -CH₂CH₂CH₃ |
| 120 | -(CH₂)₃CH=CH₂ | -CH₂CH₂CH₃ |
| 121 | -CH=CH-(CH₂)₃CH₃ | -CH₂CH₂CH₃ |
| 122 | -CH₂CH=CH-(CH₂)₂CH₃ | -CH₂CH₂CH₃ |
| 123 | -(CH₂)₂CH=CH-CH₂CH₃ | -CH₂CH₂CH₃ |
| 124 | -(CH₂)₃CH=CH-CH₃ | -CH₂CH₂CH₃ |
| 125 | -(CH₂)₄CH=CH₂ | -CH₂CH₂CH₃ |
| 126 | -CH=CH-(CH₂)₃CH₃ | -CH₂CH₂CH₃ |
| 127 | -CH₂CH=CH-(CH₂)₃CH₃ | -CH₂CH₂CH₃ |
| 128 | -(CH₂)₂CH=CH-(CH₂)₂CH₃ | -CH₂CH₂CH₃ |
| 129 | -(CH₂)₃CH=CH-CH₂CH₃ | -CH₂CH₂CH₃ |
| 130 | -(CH₂)₄CH=CH-CH₃ | -CH₂CH₂CH₃ |
| 131 | -(CH₂)₅CH=CH₂ | -CH₂CH₂CH₃ |
| 132 | -CH=CH-(CH₂)₅CH₃ | -CH₂CH₂CH₃ |
| 133 | -CH₂CH=CH-(CH₂)₄CH₃ | -CH₂CH₂CH₃ |
| 134 | -(CH₂)₂CH=CH-(CH₂)₃CH₃ | -CH₂CH₂CH₃ |
| 135 | -(CH₂)₃CH=CH-(CH₂)₂CH₃ | -CH₂CH₂CH₃ |
| 136 | -(CH₂)₄CH=CH-CH₂CH₃ | -CH₂CH₂CH₃ |
| 137 | -(CH₂)₅CH=CH-CH₃ | -CH₂CH₂CH₃ |
| 138 | -(CH₂)₆CH=CH₂ | -CH₂CH₂CH₃ |
| 139 | -CH₃ | -CH(CH₃)₂ |
| 140 | -CH₂CH₃ | -CH(CH₃)₂ |
| 141 | -CH₂CH₂CH₃ | -CH(CH₃)₂ |
| 142 | -CH(CH₃)₂ | -CH(CH₃)₂ |
| 143 | -CH₂CH₂CH₂CH₃ | -CH(CH₃)₂ |
| 144 | -CH(CH₃)CH₂CH₃ | -CH(CH₃)₂ |
| 145 | -CH₂CH(CH₃)₂ | -CH(CH₃)₂ |
| 146 | -C(CH₃)₃ | -CH(CH₃)₂ |
| 147 | -CH₂(CH₂)₃CH₃ | -CH(CH₃)₂ |
| 148 | -CH(CH₃)(CH₂)₂CH₃ | -CH(CH₃)₂ |
| 149 | -CH₂CH(CH₃)CH₂CH₃ | -CH(CH₃)₂ |
| 150 | -(CH₂)₂CH(CH₃)₂ | -CH(CH₃)₂ |
| 151 | -CH₂(CH₂)₄CH₃ | -CH(CH₃)₂ |
| 152 | -CH₂(CH₂)₅CH₃ | -CH(CH₃)₂ |
| 153 | -CH₂(CH₂)₆CH₃ | -CH(CH₃)₂ |
| 154 | -CH=CH₂ | -CH(CH₃)₂ |
| 155 | -CH=CHCH₃ | -CH(CH₃)₂ |
| 156 | -CH=C(CH₃)₂ | -CH(CH₃)₂ |
| 157 | -C(CH₃)=CH₂ | -CH(CH₃)₂ |
| 158 | -C(CH₃)=CHCH₃ | -CH(CH₃)₂ |
| 159 | -C(CH₃)=C(CH₃)₂ | -CH(CH₃)₂ |
| 160 | -CH=CH-CH₂CH₃ | -CH(CH₃)₂ |
| 161 | -CH₂CH=CH-CH₃ | -CH(CH₃)₂ |
| 162 | -(CH₂)₂CH=CH₂ | -CH(CH₃)₂ |
| 163 | -CH=CH-(CH₂)₂CH₃ | -CH(CH₃)₂ |
| 164 | -CH₂CH=CH-CH₂CH₃ | -CH(CH₃)₂ |
| 165 | -(CH₂)₂CH=CH-CH₃ | -CH(CH₃)₂ |
| 166 | -(CH₂)₃CH=CH₂ | -CH(CH₃)₂ |
| 167 | -CH=CH-(CH₂)₃CH₃ | -CH(CH₃)₂ |
| 168 | -CH₂CH=CH-(CH₂)₂CH₃ | -CH(CH₃)₂ |
| 169 | -(CH₂)₂CH=CH-CH₂CH₃ | -CH(CH₃)₂ |
| 170 | -(CH₂)₃CH=CH-CH₃ | -CH(CH₃)₂ |
| 171 | -(CH₂)₄CH=CH₂ | -CH(CH₃)₂ |
| 172 | -CH=CH-(CH₂)₃CH₃ | -CH(CH₃)₂ |
| 173 | -CH₂CH=CH-(CH₂)₃CH₃ | -CH(CH₃)₂ |
| 174 | -(CH₂)₂CH=CH-(CH₂)₂CH₃ | -CH(CH₃)₂ |
| 175 | -(CH₂)₃CH=CH-CH₂CH₃ | -CH(CH₃)₂ |
| 176 | -(CH₂)₄CH=CH-CH₃ | -CH(CH₃)₂ |
| 177 | -(CH₂)₅CH=CH₂ | -CH(CH₃)₂ |
| 178 | -CH=CH-(CH₂)₅CH₃ | -CH(CH₃)₂ |
| 179 | -CH₂CH=CH-(CH₂)₄CH₃ | -CH(CH₃)₂ |
| 180 | -(CH₂)₂CH=CH-(CH₂)₃CH₃ | -CH(CH₃)₂ |
| 181 | -(CH₂)₃CH=CH-(CH₂)₂CH₃ | -CH(CH₃)₂ |
| 182 | -(CH₂)₄CH=CH-CH₂CH₃ | -CH(CH₃)₂ |
| 183 | -(CH₂)₅CH=CH-CH₃ | -CH(CH₃)₂ |
| 184 | -(CH₂)₆CH=CH₂ | -CH(CH₃)₂ |
| 185 | -CH₃ | -CH₂CH₂CH₂CH₃ |
| 186 | -CH₂CH₃ | -CH₂CH₂CH₂CH₃ |
| 187 | -CH₂CH₂CH₃ | -CH₂CH₂CH₂CH₃ |
| 188 | -CH(CH₃)₂ | -CH₂CH₂CH₂CH₃ |
| 189 | -CH₂CH₂CH₂CH₃ | -CH₂CH₂CH₂CH₃ |
| 190 | -CH(CH₃)CH₂CH₃ | -CH₂CH₂CH₂CH₃ |
| 191 | -CH₂CH(CH₃)₂ | -CH₂CH₂CH₂CH₃ |
| 192 | -C(CH₃)₃ | -CH₂CH₂CH₂CH₃ |
| 193 | -CH₂(CH₂)₃CH₃ | -CH₂CH₂CH₂CH₃ |
| 194 | -CH(CH₃)(CH₂)₂CH₃ | -CH₂CH₂CH₂CH₃ |
| 195 | -CH₂CH(CH₃)CH₂CH₃ | -CH₂CH₂CH₂CH₃ |
| 196 | -(CH₂)₂CH(CH₃)₂ | -CH₂CH₂CH₂CH₃ |
| 197 | -CH₂(CH₂)₄CH₃ | -CH₂CH₂CH₂CH₃ |
| 198 | -CH₂(CH₂)₅CH₃ | -CH₂CH₂CH₂CH₃ |
| 199 | -CH₂(CH₂)₆CH₃ | -CH₂CH₂CH₂CH₃ |
| 200 | -CH=CH₂ | -CH₂CH₂CH₂CH₃ |
| 201 | -CH=CHCH₃ | -CH₂CH₂CH₂CH₃ |
| 202 | -CH=C(CH₃)₂ | -CH₂CH₂CH₂CH₃ |
| 203 | -C(CH₃)=CH₂ | -CH₂CH₂CH₂CH₃ |
| 204 | -C(CH₃)=CHCH₃ | -CH₂CH₂CH₂CH₃ |
| 205 | -C(CH₃)=C(CH₃)₂ | -CH₂CH₂CH₂CH₃ |
| 206 | -CH=CH-CH₂CH₃ | -CH₂CH₂CH₂CH₃ |
| 207 | -CH₂CH=CH-CH₃ | -CH₂CH₂CH₂CH₃ |
| 208 | -(CH₂)₂CH=CH₂ | -CH₂CH₂CH₂CH₃ |
| 209 | -CH=CH-(CH₂)₂CH₃ | -CH₂CH₂CH₂CH₃ |
| 210 | -CH₂CH=CH-CH₂CH₃ | -CH₂CH₂CH₂CH₃ |
| 211 | -(CH₂)₂CH=CH-CH₃ | -CH₂CH₂CH₂CH₃ |
| 212 | -(CH₂)₃CH=CH₂ | -CH₂CH₂CH₂CH₃ |
| 213 | -CH=CH-(CH₂)₃CH₃ | -CH₂CH₂CH₂CH₃ |
| 214 | -CH₂CH=CH-(CH₂)₂CH₃ | -CH₂CH₂CH₂CH₃ |
| 215 | -(CH₂)₂CH=CH-CH₂CH₃ | -CH₂CH₂CH₂CH₃ |
| 216 | -(CH₂)₃CH=CH-CH₃ | -CH₂CH₂CH₂CH₃ |
| 217 | -(CH₂)₄CH=CH₂ | -CH₂CH₂CH₂CH₃ |
| 218 | -CH=CH-(CH₂)₃CH₃ | -CH₂CH₂CH₂CH₃ |
| 219 | -CH₂CH=CH-(CH₂)₃CH₃ | -CH₂CH₂CH₂CH₃ |
| 220 | -(CH₂)₂CH=CH-(CH₂)₂CH₃ | -CH₂CH₂CH₂CH₃ |
| 221 | -(CH₂)₃CH=CH-CH₂CH₃ | -CH₂CH₂CH₂CH₃ |
| 222 | -(CH₂)₄CH=CH-CH₃ | -CH₂CH₂CH₂CH₃ |
| 223 | -(CH₂)₅CH=CH₂ | -CH₂CH₂CH₂CH₃ |
| 224 | -CH=CH-(CH₂)₅CH₃ | -CH₂CH₂CH₂CH₃ |
| 225 | -CH₂CH=CH-(CH₂)₄CH₃ | -CH₂CH₂CH₂CH₃ |
| 226 | -(CH₂)₂CH=CH-(CH₂)₃CH₃ | -CH₂CH₂CH₂CH₃ |
| 227 | -(CH₂)₃CH=CH-(CH₂)₂CH₃ | -CH₂CH₂CH₂CH₃ |
| 228 | -(CH₂)₄CH=CH-CH₂CH₃ | -CH₂CH₂CH₂CH₃ |
| 229 | -(CH₂)₅CH=CH-CH₃ | -CH₂CH₂CH₂CH₃ |
| 230 | -(CH₂)₆CH=CH₂ | -CH₂CH₂CH₂CH₃ |
| 231 | -CH₃ | -CH(CH₃)CH₂CH₃ |
| 232 | -CH₂CH₃ | -CH(CH₃)CH₂CH₃ |
| 233 | -CH₂CH₂CH₃ | -CH(CH₃)CH₂CH₃ |
| 234 | -CH(CH₃)₂ | -CH(CH₃)CH₂CH₃ |
| 235 | -CH₂CH₂CH₂CH₃ | -CH(CH₃)CH₂CH₃ |
| 236 | -CH(CH₃)CH₂CH₃ | -CH(CH₃)CH₂CH₃ |
| 237 | -CH₂CH(CH₃)₂ | -CH(CH₃)CH₂CH₃ |
| 238 | -C(CH₃)₃ | -CH(CH₃)CH₂CH₃ |
| 239 | -CH₂(CH₂)₃CH₃ | -CH(CH₃)CH₂CH₃ |
| 240 | -CH(CH₃)(CH₂)₂CH₃ | -CH(CH₃)CH₂CH₃ |
| 241 | -CH₂CH(CH₃)CH₂CH₃ | -CH(CH₃)CH₂CH₃ |
| 242 | -(CH₂)₂CH(CH₃)₂ | -CH(CH₃)CH₂CH₃ |
| 243 | -CH₂(CH₂)₄CH₃ | -CH(CH₃)CH₂CH₃ |
| 244 | -CH₂(CH₂)₅CH₃ | -CH(CH₃)CH₂CH₃ |
| 245 | -CH₂(CH₂)₆CH₃ | -CH(CH₃)CH₂CH₃ |
| 246 | -CH=CH₂ | -CH(CH₃)CH₂CH₃ |
| 247 | -CH=CHCH₃ | -CH(CH₃)CH₂CH₃ |
| 248 | -CH=C(CH₃)₂ | -CH(CH₃)CH₂CH₃ |
| 249 | -C(CH₃)=CH₂ | -CH(CH₃)CH₂CH₃ |
| 250 | -C(CH₃)=CHCH₃ | -CH(CH₃)CH₂CH₃ |
| 251 | -C(CH₃)=C(CH₃)₂ | -CH(CH₃)CH₂CH₃ |
| 252 | -CH=CH-CH₂CH₃ | -CH(CH₃)CH₂CH₃ |
| 253 | -CH₂CH=CH-CH₃ | -CH(CH₃)CH₂CH₃ |
| 254 | -(CH₂)₂CH=CH₂ | -CH(CH₃)CH₂CH₃ |
| 255 | -CH=CH-(CH₂)₂CH₃ | -CH(CH₃)CH₂CH₃ |
| 256 | -CH₂CH=CH-CH₂CH₃ | -CH(CH₃)CH₂CH₃ |
| 257 | -(CH₂)₂CH=CH-CH₃ | -CH(CH₃)CH₂CH₃ |
| 258 | -(CH₂)₃CH=CH₂ | -CH(CH₃)CH₂CH₃ |
| 259 | -CH=CH-(CH₂)₃CH₃ | -CH(CH₃)CH₂CH₃ |
| 260 | -CH₂CH=CH-(CH₂)₂CH₃ | -CH(CH₃)CH₂CH₃ |
| 261 | -(CH₂)₂CH=CH-CH₂CH₃ | -CH(CH₃)CH₂CH₃ |
| 262 | -(CH₂)₃CH=CH-CH₃ | -CH(CH₃)CH₂CH₃ |
| 263 | -(CH₂)₄CH=CH₂ | -CH(CH₃)CH₂CH₃ |
| 264 | -CH=CH-(CH₂)₃CH₃ | -CH(CH₃)CH₂CH₃ |
| 265 | -CH₂CH=CH-(CH₂)₃CH₃ | -CH(CH₃)CH₂CH₃ |
| 266 | -(CH₂)₂CH=CH-(CH₂)₂CH₃ | -CH(CH₃)CH₂CH₃ |
| 267 | -(CH₂)₃CH=CH-CH₂CH₃ | -CH(CH₃)CH₂CH₃ |
| 268 | -(CH₂)₄CH=CH-CH₃ | -CH(CH₃)CH₂CH₃ |
| 269 | -(CH₂)₅CH=CH₂ | -CH(CH₃)CH₂CH₃ |
| 270 | -CH=CH-(CH₂)₅CH₃ | -CH(CH₃)CH₂CH₃ |
| 271 | -CH₂CH=CH-(CH₂)₄CH₃ | -CH(CH₃)CH₂CH₃ |
| 272 | -(CH₂)₂CH=CH-(CH₂)₃CH₃ | -CH(CH₃)CH₂CH₃ |
| 273 | -(CH₂)₃CH=CH-(CH₂)₂CH₃ | -CH(CH₃)CH₂CH₃ |
| 274 | -(CH₂)₄CH=CH-CH₂CH₃ | -CH(CH₃)CH₂CH₃ |
| 275 | -(CH₂)₅CH=CH-CH₃ | -CH(CH₃)CH₂CH₃ |
| 276 | -(CH₂)₆CH=CH₂ | -CH(CH₃)CH₂CH₃ |
| 277 | -CH₃ | -CH₂CH(CH₃)₂ |
| 278 | -CH₂CH₃ | -CH₂CH(CH₃)₂ |
| 279 | -CH₂CH₂CH₃ | -CH₂CH(CH₃)₂ |
| 280 | -CH(CH₃)₂ | -CH₂CH(CH₃)₂ |
| 281 | -CH₂CH₂CH₂CH₃ | -CH₂CH(CH₃)₂ |
| 282 | -CH(CH₃)CH₂CH₃ | -CH₂CH(CH₃)₂ |
| 283 | -CH₂CH(CH₃)₂ | -CH₂CH(CH₃)₂ |
| 284 | -C(CH₃)₃ | -CH₂CH(CH₃)₂ |
| 285 | -CH₂(CH₂)₃CH₃ | -CH₂CH(CH₃)₂ |
| 286 | -CH(CH₃)(CH₂)₂CH₃ | -CH₂CH(CH₃)₂ |
| 287 | -CH₂CH(CH₃)CH₂CH₃ | -CH₂CH(CH₃)₂ |
| 288 | -(CH₂)₂CH(CH₃)₂ | -CH₂CH(CH₃)₂ |
| 289 | -CH₂(CH₂)₄CH₃ | -CH₂CH(CH₃)₂ |
| 290 | -CH₂(CH₂)₅CH₃ | -CH₂CH(CH₃)₂ |
| 291 | -CH₂(CH₂)₆CH₃ | -CH₂CH(CH₃)₂ |
| 292 | -CH=CH₂ | -CH₂CH(CH₃)₂ |
| 293 | -CH=CHCH₃ | -CH₂CH(CH₃)₂ |
| 294 | -CH=C(CH₃)₂ | -CH₂CH(CH₃)₂ |
| 295 | -C(CH₃)=CH₂ | -CH₂CH(CH₃)₂ |
| 296 | -C(CH₃)=CHCH₃ | -CH₂CH(CH₃)₂ |
| 297 | -C(CH₃)=C(CH₃)₂ | -CH₂CH(CH₃)₂ |
| 298 | -CH=CH-CH₂CH₃ | -CH₂CH(CH₃)₂ |
| 299 | -CH₂CH=CH-CH₃ | -CH₂CH(CH₃)₂ |
| 300 | -(CH₂)₂CH=CH₂ | -CH₂CH(CH₃)₂ |
| 301 | -CH=CH-(CH₂)₂CH₃ | -CH₂CH(CH₃)₂ |
| 302 | -CH₂CH=CH-CH₂CH₃ | -CH₂CH(CH₃)₂ |
| 303 | -(CH₂)₂CH=CH-CH₃ | -CH₂CH(CH₃)₂ |
| 304 | -(CH₂)₃CH=CH₂ | -CH₂CH(CH₃)₂ |
| 305 | -CH=CH-(CH₂)₃CH₃ | -CH₂CH(CH₃)₂ |
| 306 | -CH₂CH=CH-(CH₂)₂CH₃ | -CH₂CH(CH₃)₂ |
| 307 | -(CH₂)₂CH=CH-CH₂CH₃ | -CH₂CH(CH₃)₂ |
| 308 | -(CH₂)₃CH=CH-CH₃ | -CH₂CH(CH₃)₂ |
| 309 | -(CH₂)₄CH=CH₂ | -CH₂CH(CH₃)₂ |
| 310 | -CH=CH-(CH₂)₃CH₃ | -CH₂CH(CH₃)₂ |
| 311 | -CH₂CH=CH-(CH₂)₃CH₃ | -CH₂CH(CH₃)₂ |
| 312 | -(CH₂)₂CH=CH-(CH₂)₂CH₃ | -CH₂CH(CH₃)₂ |
| 313 | -(CH₂)₃CH=CH-CH₂CH₃ | -CH₂CH(CH₃)₂ |
| 314 | -(CH₂)₄CH=CH-CH₃ | -CH₂CH(CH₃)₂ |
| 315 | -(CH₂)₅CH=CH₂ | -CH₂CH(CH₃)₂ |
| 316 | -CH=CH-(CH₂)₅CH₃ | -CH₂CH(CH₃)₂ |
| 317 | -CH₂CH=CH-(CH₂)₄CH₃ | -CH₂CH(CH₃)₂ |
| 318 | -(CH₂)₂CH=CH-(CH₂)₃CH₃ | -CH₂CH(CH₃)₂ |
| 319 | -(CH₂)₃CH=CH-(CH₂)₂CH₃ | -CH₂CH(CH₃)₂ |
| 320 | -(CH₂)₄CH=CH-CH₂CH₃ | -CH₂CH(CH₃)₂ |
| 321 | -(CH₂)₅CH=CH-CH₃ | -CH₂CH(CH₃)₂ |
| 322 | -(CH₂)₆CH=CH₂ | -CH₂CH(CH₃)₂ |
| 323 | -CH₃ | -C(CH₃)₃ |
| 324 | -CH₂CH₃ | -C(CH₃)₃ |
| 325 | -CH₂CH₂CH₃ | -C(CH₃)₃ |
| 326 | -CH(CH₃)₂ | -C(CH₃)₃ |
| 327 | -CH₂CH₂CH₂CH₃ | -C(CH₃)₃ |
| 328 | -CH(CH₃)CH₂CH₃ | -C(CH₃)₃ |
| 329 | -CH₂CH(CH₃)₂ | -C(CH₃)₃ |
| 330 | -C(CH₃)₃ | -C(CH₃)₃ |
| 331 | -CH₂(CH₂)₃CH₃ | -C(CH₃)₃ |
| 332 | -CH(CH₃)(CH₂)₂CH₃ | -C(CH₃)₃ |
| 333 | -CH₂CH(CH₃)CH₂CH₃ | -C(CH₃)₃ |
| 334 | -(CH₂)₂CH(CH₃)₂ | -C(CH₃)₃ |
| 335 | -CH₂(CH₂)₄CH₃ | -C(CH₃)₃ |
| 336 | -CH₂(CH₂)₅CH₃ | -C(CH₃)₃ |
| 337 | -CH₂(CH₂)₆CH₃ | -C(CH₃)₃ |
| 338 | -CH=CH₂ | -C(CH₃)₃ |
| 339 | -CH=CHCH₃ | -C(CH₃)₃ |
| 340 | -CH=C(CH₃)₂ | -C(CH₃)₃ |
| 341 | -C(CH₃)=CH₂ | -C(CH₃)₃ |
| 342 | -C(CH₃)=CHCH₃ | -C(CH₃)₃ |
| 343 | -C(CH₃)=C(CH₃)₂ | -C(CH₃)₃ |
| 344 | -CH=CH-CH₂CH₃ | -C(CH₃)₃ |
| 345 | -CH₂CH=CH-CH₃ | -C(CH₃)₃ |
| 346 | -(CH₂)₂CH=CH₂ | -C(CH₃)₃ |
| 347 | -CH=CH-(CH₂)₂CH₃ | -C(CH₃)₃ |
| 348 | -CH₂CH=CH-CH₂CH₃ | -C(CH₃)₃ |
| 349 | -(CH₂)₂CH=CH-CH₃ | -C(CH₃)₃ |
| 350 | -(CH₂)₃CH=CH₂ | -C(CH₃)₃ |
| 351 | -CH=CH-(CH₂)₃CH₃ | -C(CH₃)₃ |
| 352 | -CH₂CH=CH-(CH₂)₂CH₃ | -C(CH₃)₃ |
| 353 | -(CH₂)₂CH=CH-CH₂CH₃ | -C(CH₃)₃ |
| 354 | -(CH₂)₃CH=CH-CH₃ | -C(CH₃)₃ |
| 355 | -(CH₂)₄CH=CH₂ | -C(CH₃)₃ |
| 356 | -CH=CH-(CH₂)₃CH₃ | -C(CH₃)₃ |
| 357 | -CH₂CH=CH-(CH₂)₃CH₃ | -C(CH₃)₃ |
| 358 | -(CH₂)₂CH=CH-(CH₂)₂CH₃ | -C(CH₃)₃ |
| 359 | -(CH₂)₃CH=CH-CH₂CH₃ | -C(CH₃)₃ |
| 360 | -(CH₂)₄CH=CH-CH₃ | -C(CH₃)₃ |
| 361 | -(CH₂)₅CH=CH₂ | -C(CH₃)₃ |
| 362 | -CH=CH-(CH₂)₅CH₃ | -C(CH₃)₃ |
| 363 | -CH₂CH=CH-(CH₂)₄CH₃ | -C(CH₃)₃ |
| 364 | -(CH₂)₂CH=CH-(CH₂)₃CH₃ | -C(CH₃)₃ |
| 365 | -(CH₂)₃CH=CH-(CH₂)₂CH₃ | -C(CH₃)₃ |
| 366 | -(CH₂)₄CH=CH-CH₂CH₃ | -C(CH₃)₃ |
| 367 | -(CH₂)₅CH=CH-CH₃ | -C(CH₃)₃ |
| 368 | -(CH₂)₆CH=CH₂ | -C(CH₃)₃ |
| 369 | -CH₃ | -CH₂(CH₂)₃CH₃ |
| 370 | -CH₂CH₃ | -CH₂(CH₂)₃CH₃ |
| 371 | -CH₂CH₂CH₃ | -CH₂(CH₂)₃CH₃ |
| 372 | -CH(CH₃)₂ | -CH₂(CH₂)₃CH₃ |
| 373 | -CH₂CH₂CH₂CH₃ | -CH₂(CH₂)₃CH₃ |
| 374 | -CH(CH₃)CH₂CH₃ | -CH₂(CH₂)₃CH₃ |
| 375 | -CH₂CH(CH₃)₂ | -CH₂(CH₂)₃CH₃ |
| 376 | -C(CH₃)₃ | -CH₂(CH₂)₃CH₃ |
| 377 | -CH₂(CH₂)₃CH₃ | -CH₂(CH₂)₃CH₃ |
| 378 | -CH(CH₃)(CH₂)₂CH₃ | -CH₂(CH₂)₃CH₃ |
| 379 | -CH₂CH(CH₃)CH₂CH₃ | -CH₂(CH₂)₃CH₃ |
| 380 | -(CH₂)₂CH(CH₃)₂ | -CH₂(CH₂)₃CH₃ |
| 381 | -CH₂(CH₂)₄CH₃ | -CH₂(CH₂)₃CH₃ |
| 382 | -CH₂(CH₂)₅CH₃ | -CH₂(CH₂)₃CH₃ |
| 383 | -CH₂(CH₂)₆CH₃ | -CH₂(CH₂)₃CH₃ |
| 384 | -CH=CH₂ | -CH₂(CH₂)₃CH₃ |
| 385 | -CH=CHCH₃ | -CH₂(CH₂)₃CH₃ |
| 386 | -CH=C(CH₃)₂ | -CH₂(CH₂)₃CH₃ |
| 387 | -C(CH₃)=CH₂ | -CH₂(CH₂)₃CH₃ |
| 388 | -C(CH₃)=CHCH₃ | -CH₂(CH₂)₃CH₃ |
| 389 | -C(CH₃)=C(CH₃)₂ | -CH₂(CH₂)₃CH₃ |
| 390 | -CH=CH-CH₂CH₃ | -CH₂(CH₂)₃CH₃ |
| 391 | -CH₂CH=CH-CH₃ | -CH₂(CH₂)₃CH₃ |
| 392 | -(CH₂)₂CH=CH₂ | -CH₂(CH₂)₃CH₃ |
| 393 | -CH=CH-(CH₂)₂CH₃ | -CH₂(CH₂)₃CH₃ |
| 394 | -CH₂CH=CH-CH₂CH₃ | -CH₂(CH₂)₃CH₃ |
| 395 | -(CH₂)₂CH=CH-CH₃ | -CH₂(CH₂)₃CH₃ |
| 396 | -(CH₂)₃CH=CH₂ | -CH₂(CH₂)₃CH₃ |
| 397 | -CH=CH-(CH₂)₃CH₃ | -CH₂(CH₂)₃CH₃ |
| 398 | -CH₂CH=CH-(CH₂)₂CH₃ | -CH₂(CH₂)₃CH₃ |
| 399 | -(CH₂)₂CH=CH-CH₂CH₃ | -CH₂(CH₂)₃CH₃ |
| 400 | -(CH₂)₃CH=CH-CH₃ | -CH₂(CH₂)₃CH₃ |
| 401 | -(CH₂)₄CH=CH₂ | -CH₂(CH₂)₃CH₃ |
| 402 | -CH=CH-(CH₂)₃CH₃ | -CH₂(CH₂)₃CH₃ |
| 403 | -CH₂CH=CH-(CH₂)₃CH₃ | -CH₂(CH₂)₃CH₃ |
| 404 | -(CH₂)₂CH=CH-(CH₂)₂CH₃ | -CH₂(CH₂)₃CH₃ |
| 405 | -(CH₂)₃CH=CH-CH₂CH₃ | -CH₂(CH₂)₃CH₃ |
| 406 | -(CH₂)₄CH=CH-CH₃ | -CH₂(CH₂)₃CH₃ |
| 407 | -(CH₂)₅CH=CH₂ | -CH₂(CH₂)₃CH₃ |
| 408 | -CH=CH-(CH₂)₅CH₃ | -CH₂(CH₂)₃CH₃ |
| 409 | -CH₂CH=CH-(CH₂)₄CH₃ | -CH₂(CH₂)₃CH₃ |
| 410 | -(CH₂)₂CH=CH-(CH₂)₃CH₃ | -CH₂(CH₂)₃CH₃ |
| 411 | -(CH₂)₃CH=CH-(CH₂)₂CH₃ | -CH₂(CH₂)₃CH₃ |
| 412 | -(CH₂)₄CH=CH-CH₂CH₃ | -CH₂(CH₂)₃CH₃ |
| 413 | -(CH₂)₅CH=CH-CH₃ | -CH₂(CH₂)₃CH₃ |
| 414 | -(CH₂)₆CH=CH₂ | -CH₂(CH₂)₃CH₃ |
| 415 | -CH₃ | -CH(CH₃)(CH₂)₂CH₃ |
| 416 | -CH₂CH₃ | -CH(CH₃)(CH₂)₂CH₃ |
| 417 | -CH₂CH₂CH₃ | -CH(CH₃)(CH₂)₂CH₃ |
| 418 | -CH(CH₃)₂ | -CH(CH₃)(CH₂)₂CH₃ |
| 419 | -CH₂CH₂CH₂CH₃ | -CH(CH₃)(CH₂)₂CH₃ |
| 420 | -CH(CH₃)CH₂CH₃ | -CH(CH₃)(CH₂)₂CH₃ |
| 421 | -CH₂CH(CH₃)₂ | -CH(CH₃)(CH₂)₂CH₃ |
| 422 | -C(CH₃)₃ | -CH(CH₃)(CH₂)₂CH₃ |
| 423 | -CH₂(CH₂)₃CH₃ | -CH(CH₃)(CH₂)₂CH₃ |
| 424 | -CH(CH₃)(CH₂)₂CH₃ | -CH(CH₃)(CH₂)₂CH₃ |
| 425 | -CH₂CH(CH₃)CH₂CH₃ | -CH(CH₃)(CH₂)₂CH₃ |
| 426 | -(CH₂)₂CH(CH₃)₂ | -CH(CH₃)(CH₂)₂CH₃ |
| 427 | -CH₂(CH₂)₄CH₃ | -CH(CH₃)(CH₂)₂CH₃ |
| 428 | -CH₂(CH₂)₅CH₃ | -CH(CH₃)(CH₂)₂CH₃ |
| 429 | -CH₂(CH₂)₆CH₃ | -CH(CH₃)(CH₂)₂CH₃ |
| 430 | -CH=CH₂ | -CH(CH₃)(CH₂)₂CH₃ |
| 431 | -CH=CHCH₃ | -CH(CH₃)(CH₂)₂CH₃ |
| 432 | -CH=C(CH₃)₂ | -CH(CH₃)(CH₂)₂CH₃ |
| 433 | -C(CH₃)=CH₂ | -CH(CH₃)(CH₂)₂CH₃ |
| 434 | -C(CH₃)=CHCH₃ | -CH(CH₃)(CH₂)₂CH₃ |
| 435 | -C(CH₃)=C(CH₃)₂ | -CH(CH₃)(CH₂)₂CH₃ |
| 436 | -CH=CH-CH₂CH₃ | -CH(CH₃)(CH₂)₂CH₃ |
| 437 | -CH₂CH=CH-CH₃ | -CH(CH₃)(CH₂)₂CH₃ |
| 438 | -(CH₂)₂CH=CH₂ | -CH(CH₃)(CH₂)₂CH₃ |
| 439 | -CH=CH-(CH₂)₂CH₃ | -CH(CH₃)(CH₂)₂CH₃ |
| 440 | -CH₂CH=CH-CH₂CH₃ | -CH(CH₃)(CH₂)₂CH₃ |
| 441 | -(CH₂)₂CH=CH-CH₃ | -CH(CH₃)(CH₂)₂CH₃ |
| 442 | -(CH₂)₃CH=CH₂ | -CH(CH₃)(CH₂)₂CH₃ |
| 443 | -CH=CH-(CH₂)₃CH₃ | -CH(CH₃)(CH₂)₂CH₃ |
| 444 | -CH₂CH=CH-(CH₂)₂CH₃ | -CH(CH₃)(CH₂)₂CH₃ |
| 445 | -(CH₂)₂CH=CH-CH₂CH₃ | -CH(CH₃)(CH₂)₂CH₃ |
| 446 | -(CH₂)₃CH=CH-CH₃ | -CH(CH₃)(CH₂)₂CH₃ |
| 447 | -(CH₂)₄CH=CH₂ | -CH(CH₃)(CH₂)₂CH₃ |
| 448 | -CH=CH-(CH₂)₃CH₃ | -CH(CH₃)(CH₂)₂CH₃ |
| 449 | -CH₂CH=CH-(CH₂)₃CH₃ | -CH(CH₃)(CH₂)₂CH₃ |
| 450 | -(CH₂)₂CH=CH-(CH₂)₂CH₃ | -CH(CH₃)(CH₂)₂CH₃ |
| 451 | -(CH₂)₃CH=CH-CH₂CH₃ | -CH(CH₃)(CH₂)₂CH₃ |
| 452 | -(CH₂)₄CH=CH-CH₃ | -CH(CH₃)(CH₂)₂CH₃ |
| 453 | -(CH₂)₅CH=CH₂ | -CH(CH₃)(CH₂)₂CH₃ |
| 454 | -CH=CH-(CH₂)₅CH₃ | -CH(CH₃)(CH₂)₂CH₃ |
| 455 | -CH₂CH=CH-(CH₂)₄CH₃ | -CH(CH₃)(CH₂)₂CH₃ |
| 456 | -(CH₂)₂CH=CH-(CH₂)₃CH₃ | -CH(CH₃)(CH₂)₂CH₃ |
| 457 | -(CH₂)₃CH=CH-(CH₂)₂CH₃ | -CH(CH₃)(CH₂)₂CH₃ |
| 458 | -(CH₂)₄CH=CH-CH₂CH₃ | -CH(CH₃)(CH₂)₂CH₃ |
| 459 | -(CH₂)₅CH=CH-CH₃ | -CH(CH₃)(CH₂)₂CH₃ |
| 460 | -(CH₂)₆CH=CH₂ | -CH(CH₃)(CH₂)₂CH₃ |
| 461 | -CH₃ | -CH₂CH(CH₃)CH₂CH₃ |
| 462 | -CH₂CH₃ | -CH₂CH(CH₃)CH₂CH₃ |
| 463 | -CH₂CH₂CH₃ | -CH₂CH(CH₃)CH₂CH₃ |
| 464 | -CH(CH₃)₂ | -CH₂CH(CH₃)CH₂CH₃ |
| 465 | -CH₂CH₂CH₂CH₃ | -CH₂CH(CH₃)CH₂CH₃ |
| 466 | -CH(CH₃)CH₂CH₃ | -CH₂CH(CH₃)CH₂CH₃ |
| 467 | -CH₂CH(CH₃)₂ | -CH₂CH(CH₃)CH₂CH₃ |
| 468 | -C(CH₃)₃ | -CH₂CH(CH₃)CH₂CH₃ |
| 469 | -CH₂(CH₂)₃CH₃ | -CH₂CH(CH₃)CH₂CH₃ |
| 470 | -CH(CH₃)(CH₂)₂CH₃ | -CH₂CH(CH₃)CH₂CH₃ |
| 471 | -CH₂CH(CH₃)CH₂CH₃ | -CH₂CH(CH₃)CH₂CH₃ |
| 472 | -(CH₂)₂CH(CH₃)₂ | -CH₂CH(CH₃)CH₂CH₃ |
| 473 | -CH₂(CH₂)₄CH₃ | -CH₂CH(CH₃)CH₂CH₃ |
| 474 | -CH₂(CH₂)₅CH₃ | -CH₂CH(CH₃)CH₂CH₃ |
| 475 | -CH₂(CH₂)₆CH₃ | -CH₂CH(CH₃)CH₂CH₃ |
| 476 | -CH=CH₂ | -CH₂CH(CH₃)CH₂CH₃ |
| 477 | -CH=CHCH₃ | -CH₂CH(CH₃)CH₂CH₃ |
| 478 | -CH=C(CH₃)₂ | -CH₂CH(CH₃)CH₂CH₃ |
| 479 | -C(CH₃)=CH₂ | -CH₂CH(CH₃)CH₂CH₃ |
| 480 | -C(CH₃)=CHCH₃ | -CH₂CH(CH₃)CH₂CH₃ |
| 481 | -C(CH₃)=C(CH₃)₂ | -CH₂CH(CH₃)CH₂CH₃ |
| 482 | -CH=CH-CH₂CH₃ | -CH₂CH(CH₃)CH₂CH₃ |
| 483 | -CH₂CH=CH-CH₃ | -CH₂CH(CH₃)CH₂CH₃ |
| 484 | -(CH₂)₂CH=CH₂ | -CH₂CH(CH₃)CH₂CH₃ |
| 485 | -CH=CH-(CH₂)₂CH₃ | -CH₂CH(CH₃)CH₂CH₃ |
| 486 | -CH₂CH=CH-CH₂CH₃ | -CH₂CH(CH₃)CH₂CH₃ |
| 487 | -(CH₂)₂CH=CH-CH₃ | -CH₂CH(CH₃)CH₂CH₃ |
| 488 | -(CH₂)₃CH=CH₂ | -CH₂CH(CH₃)CH₂CH₃ |
| 489 | -CH=CH-(CH₂)₃CH₃ | -CH₂CH(CH₃)CH₂CH₃ |
| 490 | -CH₂CH=CH-(CH₂)₂CH₃ | -CH₂CH(CH₃)CH₂CH₃ |
| 491 | -(CH₂)₂CH=CH-CH₂CH₃ | -CH₂CH(CH₃)CH₂CH₃ |
| 492 | -(CH₂)₃CH=CH-CH₃ | -CH₂CH(CH₃)CH₂CH₃ |
| 493 | -(CH₂)₄CH=CH₂ | -CH₂CH(CH₃)CH₂CH₃ |
| 494 | -CH=CH-(CH₂)₃CH₃ | -CH₂CH(CH₃)CH₂CH₃ |
| 495 | -CH₂CH=CH-(CH₂)₃CH₃ | -CH₂CH(CH₃)CH₂CH₃ |
| 496 | -(CH₂)₂CH=CH-(CH₂)₂CH₃ | -CH₂CH(CH₃)CH₂CH₃ |
| 497 | -(CH₂)₃CH=CH-CH₂CH₃ | -CH₂CH(CH₃)CH₂CH₃ |
| 498 | -(CH₂)₄CH=CH-CH₃ | -CH₂CH(CH₃)CH₂CH₃ |
| 499 | -(CH₂)₅CH=CH₂ | -CH₂CH(CH₃)CH₂CH₃ |
| 500 | -CH=CH-(CH₂)₅CH₃ | -CH₂CH(CH₃)CH₂CH₃ |
| 501 | -CH₂CH=CH-(CH₂)₄CH₃ | -CH₂CH(CH₃)CH₂CH₃ |
| 502 | -(CH₂)₂CH=CH-(CH₂)₃CH₃ | -CH₂CH(CH₃)CH₂CH₃ |
| 503 | -(CH₂)₃CH=CH-(CH₂)₂CH₃ | -CH₂CH(CH₃)CH₂CH₃ |
| 504 | -(CH₂)₄CH=CH-CH₂CH₃ | -CH₂CH(CH₃)CH₂CH₃ |
| 505 | -(CH₂)₅CH=CH-CH₃ | -CH₂CH(CH₃)CH₂CH₃ |
| 506 | -(CH₂)₆CH=CH₂ | -CH₂CH(CH₃)CH₂CH₃ |
| 507 | -CH₃ | -(CH₂)₂CH(CH₃)₂ |
| 508 | -CH₂CH₃ | -(CH₂)₂CH(CH₃)₂ |
| 509 | -CH₂CH₂CH₃ | -(CH₂)₂CH(CH₃)₂ |
| 510 | -CH(CH₃)₂ | -(CH₂)₂CH(CH₃)₂ |
| 511 | -CH₂CH₂CH₂CH₃ | -(CH₂)₂CH(CH₃)₂ |
| 512 | -CH(CH₃)CH₂CH₃ | -(CH₂)₂CH(CH₃)₂ |
| 513 | -CH₂CH(CH₃)₂ | -(CH₂)₂CH(CH₃)₂ |
| 514 | -C(CH₃)₃ | -(CH₂)₂CH(CH₃)₂ |
| 515 | -CH₂(CH₂)₃CH₃ | -(CH₂)₂CH(CH₃)₂ |
| 516 | -CH(CH₃)(CH₂)₂CH₃ | -(CH₂)₂CH(CH₃)₂ |
| 517 | -CH₂CH(CH₃)CH₂CH₃ | -(CH₂)₂CH(CH₃)₂ |
| 518 | -(CH₂)₂CH(CH₃)₂ | -(CH₂)₂CH(CH₃)₂ |
| 519 | -CH₂(CH₂)₄CH₃ | -(CH₂)₂CH(CH₃)₂ |
| 520 | -CH₂(CH₂)₅CH₃ | -(CH₂)₂CH(CH₃)₂ |
| 521 | -CH₂(CH₂)₆CH₃ | -(CH₂)₂CH(CH₃)₂ |
| 522 | -CH=CH₂ | -(CH₂)₂CH(CH₃)₂ |
| 523 | -CH=CHCH₃ | -(CH₂)₂CH(CH₃)₂ |
| 524 | -CH=C(CH₃)₂ | -(CH₂)₂CH(CH₃)₂ |
| 525 | -C(CH₃)=CH₂ | -(CH₂)₂CH(CH₃)₂ |
| 526 | -C(CH₃)=CHCH₃ | -(CH₂)₂CH(CH₃)₂ |
| 527 | -C(CH₃)=C(CH₃)₂ | -(CH₂)₂CH(CH₃)₂ |
| 528 | -CH=CH-CH₂CH₃ | -(CH₂)₂CH(CH₃)₂ |
| 529 | -CH₂CH=CH-CH₃ | -(CH₂)₂CH(CH₃)₂ |
| 530 | -(CH₂)₂CH=CH₂ | -(CH₂)₂CH(CH₃)₂ |
| 531 | -CH=CH-(CH₂)₂CH₃ | -(CH₂)₂CH(CH₃)₂ |
| 532 | -CH₂CH=CH-CH₂CH₃ | -(CH₂)₂CH(CH₃)₂ |
| 533 | -(CH₂)₂CH=CH-CH₃ | -(CH₂)₂CH(CH₃)₂ |
| 534 | -(CH₂)₃CH=CH₂ | -(CH₂)₂CH(CH₃)₂ |
| 535 | -CH=CH-(CH₂)₃CH₃ | -(CH₂)₂CH(CH₃)₂ |
| 536 | -CH₂CH=CH-(CH₂)₂CH₃ | -(CH₂)₂CH(CH₃)₂ |
| 537 | -(CH₂)₂CH=CH-CH₂CH₃ | -(CH₂)₂CH(CH₃)₂ |
| 538 | -(CH₂)₃CH=CH-CH₃ | -(CH₂)₂CH(CH₃)₂ |
| 539 | -(CH₂)₄CH=CH₂ | -(CH₂)₂CH(CH₃)₂ |
| 540 | -CH=CH-(CH₂)₃CH₃ | -(CH₂)₂CH(CH₃)₂ |
| 541 | -CH₂CH=CH-(CH₂)₃CH₃ | -(CH₂)₂CH(CH₃)₂ |
| 542 | -(CH₂)₂CH=CH-(CH₂)₂CH₃ | -(CH₂)₂CH(CH₃)₂ |
| 543 | -(CH₂)₃CH=CH-CH₂CH₃ | -(CH₂)₂CH(CH₃)₂ |
| 544 | -(CH₂)₄CH=CH-CH₃ | -(CH₂)₂CH(CH₃)₂ |
| 545 | -(CH₂)₅CH=CH₂ | -(CH₂)₂CH(CH₃)₂ |
| 546 | -CH=CH-(CH₂)₅CH₃ | -(CH₂)₂CH(CH₃)₂ |
| 547 | -CH₂CH=CH-(CH₂)₄CH₃ | -(CH₂)₂CH(CH₃)₂ |
| 548 | -(CH₂)₂CH=CH-(CH₂)₃CH₃ | -(CH₂)₂CH(CH₃)₂ |
| 549 | -(CH₂)₃CH₌CH-(CH₂)₂CH₃ | -(CH₂)₂CH(CH₃)₂ |
| 550 | -(CH₂)₄CH=CH-CH₂CH₃ | -(CH₂)₂CH(CH₃)₂ |
| 551 | -(CH₂)₅CH=CH-CH₃ | -(CH₂)₂CH(CH₃)₂ |
| 552 | -(CH₂)₆CH=CH₂ | -(CH₂)₂CH(CH₃)₂ |
| 553 | -CH₃ | -CH₂(CH₂)₄CH₃ |
| 554 | -CH₂CH₃ | -CH₂(CH₂)₄CH₃ |
| 555 | -CH₂CH₂CH₃ | -CH₂(CH₂)₄CH₃ |
| 556 | -CH(CH₃)₂ | -CH₂(CH₂)₄CH₃ |
| 557 | -CH₂CH₂CH₂CH₃ | -CH₂(CH₂)₄CH₃ |
| 558 | -CH(CH₃)CH₂CH₃ | -CH₂(CH₂)₄CH₃ |
| 559 | -CH₂CH(CH₃)₂ | -CH₂(CH₂)₄CH₃ |
| 560 | -C(CH₃)₃ | -CH₂(CH₂)₄CH₃ |
| 561 | -CH₂(CH₂)₃CH₃ | -CH₂(CH₂)₄CH₃ |
| 562 | -CH(CH₃)(CH₂)₂CH₃ | -CH₂(CH₂)₄CH₃ |
| 563 | -CH₂CH(CH₃)CH₂CH₃ | -CH₂(CH₂)₄CH₃ |
| 564 | -(CH₂)₂CH(CH₃)₂ | -CH₂(CH₂)₄CH₃ |
| 565 | -CH₂(CH₂)₄CH₃ | -CH₂(CH₂)₄CH₃ |
| 566 | -CH₂(CH₂)₅CH₃ | -CH₂(CH₂)₄CH₃ |
| 567 | -CH₂(CH₂)₆CH₃ | -CH₂(CH₂)₄CH₃ |
| 568 | -CH=CH₂ | -CH₂(CH₂)₄CH₃ |
| 569 | -CH=CHCH₃ | -CH₂(CH₂)₄CH₃ |
| 570 | -CH=C(CH₃)₂ | -CH₂(CH₂)₄CH₃ |
| 571 | -C(CH₃)=CH₂ | -CH₂(CH₂)₄CH₃ |
| 572 | -C(CH₃)=CHCH₃ | -CH₂(CH₂)₄CH₃ |
| 573 | -C(CH₃)=C(CH₃)₂ | -CH₂(CH₂)₄CH₃ |
| 574 | -CH=CH-CH₂CH₃ | -CH₂(CH₂)₄CH₃ |
| 575 | -CH₂CH=CH-CH₃ | -CH₂(CH₂)₄CH₃ |
| 576 | -(CH₂)₂CH=CH₂ | -CH₂(CH₂)₄CH₃ |
| 577 | -CH=CH-(CH₂)₂CH₃ | -CH₂(CH₂)₄CH₃ |
| 578 | -CH₂CH=CH-CH₂CH₃ | -CH₂(CH₂)₄CH₃ |
| 579 | -(CH₂)₂CH=CH-CH₃ | -CH₂(CH₂)₄CH₃ |
| 580 | -(CH₂)₃CH=CH₂ | -CH₂(CH₂)₄CH₃ |
| 581 | -CH=CH-(CH₂)₃CH₃ | -CH₂(CH₂)₄CH₃ |
| 582 | -CH₂CH=CH-(CH₂)₂CH₃ | -CH₂(CH₂)₄CH₃ |
| 583 | -(CH₂)₂CH=CH-CH₂CH₃ | -CH₂(CH₂)₄CH₃ |
| 584 | -(CH₂)₃CH=CH-CH₃ | -CH₂(CH₂)₄CH₃ |
| 585 | -(CH₂)₄CH=CH₂ | -CH₂(CH₂)₄CH₃ |
| 586 | -CH=CH-(CH₂)₃CH₃ | -CH₂(CH₂)₄CH₃ |
| 587 | -CH₂CH=CH-(CH₂)₃CH₃ | -CH₂(CH₂)₄CH₃ |
| 588 | -(CH₂)₂CH=CH-(CH₂)₂CH₃ | -CH₂(CH₂)₄CH₃ |
| 589 | -(CH₂)₃CH=CH-CH₂CH₃ | -CH₂(CH₂)₄CH₃ |
| 590 | -(CH₂)₄CH=CH-CH₃ | -CH₂(CH₂)₄CH₃ |
| 591 | -(CH₂)₅CH=CH₂ | -CH₂(CH₂)₄CH₃ |
| 592 | -CH=CH-(CH₂)₅CH₃ | -CH₂(CH₂)₄CH₃ |
| 593 | -CH₂CH=CH-(CH₂)₄CH₃ | -CH₂(CH₂)₄CH₃ |
| 594 | -(CH₂)₂CH=CH-(CH₂)₃CH₃ | -CH₂(CH₂)₄CH₃ |
| 595 | -(CH₂)₃CH=CH-(CH₂)₂CH₃ | -CH₂(CH₂)₄CH₃ |
| 596 | -(CH₂)₄CH=CH-CH₂CH₃ | -CH₂(CH₂)₄CH₃ |
| 597 | -(CH₂)₅CH=CH-CH₃ | -CH₂(CH₂)₄CH₃ |
| 598 | -(CH₂)₆CH=CH₂ | -CH₂(CH₂)₄CH₃ |
| 599 | -CH₃ | -CH₂(CH₂)₅CH₃ |
| 600 | -CH₂CH₃ | -CH₂(CH₂)₅CH₃ |
| 601 | -CH₂CH₂CH₃ | -CH₂(CH₂)₅CH₃ |
| 602 | -CH(CH₃)₂ | -CH₂(CH₂)₅CH₃ |
| 603 | -CH₂CH₂CH₂CH₃ | -CH₂(CH₂)₅CH₃ |
| 604 | -CH(CH₃)CH₂CH₃ | -CH₂(CH₂)₅CH₃ |
| 605 | -CH₂CH(CH₃)₂ | -CH₂(CH₂)₅CH₃ |
| 606 | -C(CH₃)₃ | -CH₂(CH₂)₅CH₃ |
| 607 | -CH₂(CH₂)₃CH₃ | -CH₂(CH₂)₅CH₃ |
| 608 | -CH(CH₃)(CH₂)₂CH₃ | -CH₂(CH₂)₅CH₃ |
| 609 | -CH₂CH(CH₃)CH₂CH₃ | -CH₂(CH₂)₅CH₃ |
| 610 | -(CH₂)₂CH(CH₃)₂ | -CH₂(CH₂)₅CH₃ |
| 611 | -CH₂(CH₂)₄CH₃ | -CH₂(CH₂)₅CH₃ |
| 612 | -CH₂(CH₂)₅CH₃ | -CH₂(CH₂)₅CH₃ |
| 613 | -CH₂(CH₂)₆CH₃ | -CH₂(CH₂)₅CH₃ |
| 614 | -CH=CH₂ | -CH₂(CH₂)₅CH₃ |
| 615 | -CH=CHCH₃ | -CH₂(CH₂)₅CH₃ |
| 616 | -CH=C(CH₃)₂ | -CH₂(CH₂)₅CH₃ |
| 617 | -C(CH₃)=CH₂ | -CH₂(CH₂)₅CH₃ |
| 618 | -C(CH₃)=CHCH₃ | -CH₂(CH₂)₅CH₃ |
| 619 | -C(CH₃)=C(CH₃)₂ | -CH₂(CH₂)₅CH₃ |
| 620 | -CH=CH-CH₂CH₃ | -CH₂(CH₂)₅CH₃ |
| 621 | -CH₂CH=CH-CH₃ | -CH₂(CH₂)₅CH₃ |
| 622 | -(CH₂)₂CH=CH₂ | -CH₂(CH₂)₅CH₃ |
| 623 | -CH=CH-(CH₂)₂CH₃ | -CH₂(CH₂)₅CH₃ |
| 624 | -CH₂CH=CH-CH₂CH₃ | -CH₂(CH₂)₅CH₃ |
| 625 | -(CH₂)₂CH=CH-CH₃ | -CH₂(CH₂)₅CH₃ |
| 626 | -(CH₂)₃CH=CH₂ | -CH₂(CH₂)₅CH₃ |
| 627 | -CH=CH-(CH₂)₃CH₃ | -CH₂(CH₂)₅CH₃ |
| 628 | -CH₂CH=CH-(CH₂)₂CH₃ | -CH₂(CH₂)₅CH₃ |
| 629 | -(CH₂)₂CH=CH-CH₂CH₃ | -CH₂(CH₂)₅CH₃ |
| 630 | -(CH₂)₃CH=CH-CH₃ | -CH₂(CH₂)₅CH₃ |
| 631 | -(CH₂)₄CH=CH₂ | -CH₂(CH₂)₅CH₃ |
| 632 | -CH=CH-(CH₂)₃CH₃ | -CH₂(CH₂)₅CH₃ |
| 633 | -CH₂CH=CH-(CH₂)₃CH₃ | -CH₂(CH₂)₅CH₃ |
| 634 | -(CH₂)₂CH=CH-(CH₂)₂CH₃ | -CH₂(CH₂)₅CH₃ |
| 635 | -(CH₂)₃CH=CH-CH₂CH₃ | -CH₂(CH₂)₅CH₃ |
| 636 | -(CH₂)₄CH=CH-CH₃ | -CH₂(CH₂)₅CH₃ |
| 637 | -(CH₂)₅CH=CH₂ | -CH₂(CH₂)₅CH₃ |
| 638 | -CH=CH-(CH₂)₅CH₃ | -CH₂(CH₂)₅CH₃ |
| 639 | -CH₂CH=CH-(CH₂)₄CH₃ | -CH₂(CH₂)₅CH₃ |
| 640 | -(CH₂)₂CH=CH-(CH₂)₃CH₃ | -CH₂(CH₂)₅CH₃ |
| 641 | -(CH₂)₃CH=CH-(CH₂)₂CH₃ | -CH₂(CH₂)₅CH₃ |
| 642 | -(CH₂)₄CH=CH-CH₂CH₃ | -CH₂(CH₂)₅CH₃ |
| 643 | -(CH₂)₅CH=CH-CH₃ | -CH₂(CH₂)₅CH₃ |
| 644 | -(CH₂)₆CH=CH₂ | -CH₂(CH₂)₅CH₃ |
| 645 | -CH₃ | -CH₂(CH₂)₆CH₃ |
| 646 | -CH₂CH₃ | -CH₂(CH₂)₆CH₃ |
| 647 | -CH₂CH₂CH₃ | -CH₂(CH₂)₆CH₃ |
| 648 | -CH(CH₃)₂ | -CH₂(CH₂)₆CH₃ |
| 649 | -CH₂CH₂CH₂CH₃ | -CH₂(CH₂)₆CH₃ |
| 650 | -CH(CH₃)CH₂CH₃ | -CH₂(CH₂)₆CH₃ |
| 651 | -CH₂CH(CH₃)₂ | -CH₂(CH₂)₆CH₃ |
| 652 | -C(CH₃)₃ | -CH₂(CH₂)₆CH₃ |
| 653 | -CH₂(CH₂)₃CH₃ | -CH₂(CH₂)₆CH₃ |
| 654 | -CH(CH₃)(CH₂)₂CH₃ | -CH₂(CH₂)₆CH₃ |
| 655 | -CH₂CH(CH₃)CH₂CH₃ | -CH₂(CH₂)₆CH₃ |
| 656 | -(CH₂)₂CH(CH₃)₂ | -CH₂(CH₂)₆CH₃ |
| 657 | -CH₂(CH₂)₄CH₃ | -CH₂(CH₂)₆CH₃ |
| 658 | -CH₂(CH₂)₅CH₃ | -CH₂(CH₂)₆CH₃ |
| 59 | -CH₂(CH₂)₆CH₃ | -CH₂(CH₂)₆CH₃ |
| 660 | -CH=CH₂ | -CH₂(CH₂)₆CH₃ |
| 661 | -CH=CHCH₃ | -CH₂(CH₂)₆CH₃ |
| 662 | -CH=C(CH₃)₂ | -CH₂(CH₂)₆CH₃ |
| 663 | -C(CH₃)=CH₂ | -CH₂(CH₂)₆CH₃ |
| 664 | -C(CH₃)=CHCH₃ | -CH₂(CH₂)₆CH₃ |
| 665 | -C(CH₃)=C(CH₃)₂ | -CH₂(CH₂)₆CH₃ |
| 666 | -CH=CH-CH₂CH₃ | -CH₂(CH₂)₆CH₃ |
| 667 | -CH₂CH=CH-CH₃ | -CH₂(CH₂)₆CH₃ |
| 668 | -(CH₂)₂CH=CH₂ | -CH₂(CH₂)₆CH₃ |
| 669 | -CH=CH-(CH₂)₂CH₃ | -CH₂(CH₂)₆CH₃ |
| 670 | -CH₂CH=CH-CH₂CH₃ | -CH₂(CH₂)₆CH₃ |
| 671 | -(CH₂)₂CH=CH-CH₃ | -CH₂(CH₂)₆CH₃ |
| 672 | -(CH₂)₃CH=CH₂ | -CH₂(CH₂)₆CH₃ |
| 673 | -CH=CH-(CH₂)₃CH₃ | -CH₂(CH₂)₆CH₃ |
| 674 | -CH₂CH=CH-(CH₂)₂CH₃ | -CH₂(CH₂)₆CH₃ |
| 675 | -(CH₂)₂CH=CH-CH₂CH₃ | -CH₂(CH₂)₆CH₃ |
| 676 | -(CH₂)₃CH=CH-CH₃ | -CH₂(CH₂)₆CH₃ |
| 677 | -(CH₂)₄CH=CH₂ | -CH₂(CH₂)₆CH₃ |
| 678 | -CH=CH-(CH₂)₃CH₃ | -CH₂(CH₂)₆CH₃ |
| 679 | -CH₂CH=CH-(CH₂)₃CH₃ | -CH₂(CH₂)₆CH₃ |
| 680 | -(CH₂)₂CH=CH-(CH₂)₂CH₃ | -CH₂(CH₂)₆CH₃ |
| 681 | -(CH₂)₃CH=CH-CH₂CH₃ | -CH₂(CH₂)₆CH₃ |
| 682 | -(CH₂)₄CH=CH-CH₃ | -CH₂(CH₂)₆CH₃ |
| 683 | -(CH₂)₅CH=CH₂ | -CH₂(CH₂)₆CH₃ |
| 684 | -CH=CH-(CH₂)₅CH₃ | -CH₂(CH₂)₆CH₃ |
| 685 | -CH₂CH=CH-(CH₂)₄CH₃ | -CH₂(CH₂)₆CH₃ |
| 686 | -(CH₂)₂CH=CH-(CH₂)₃CH₃ | -CH₂(CH₂)₆CH₃ |
| 687 | -(CH₂)₃CH=CH-(CH₂)₂CH₃ | -CH₂(CH₂)₆CH₃ |
| 688 | -(CH₂)₄CH=CH-CH₂CH₃ | -CH₂(CH₂)₆CH₃ |
| 689 | -(CH₂)₅CH=CH-CH₃ | -CH₂(CH₂)₆CH₃ |
| 690 | -(CH₂)₆CH=CH₂ | -CH₂(CH₂)₆CH₃ |
| 691 | -CH₃ | -CH=CH₂ |
| 692 | -CH₂CH₃ | -CH=CH₂ |
| 693 | -CH₂CH₂CH₃ | -CH=CH₂ |
| 694 | -CH(CH₃)₂ | -CH=CH₂ |
| 695 | -CH₂CH₂CH₂CH₃ | -CH=CH₂ |
| 696 | -CH(CH₃)CH₂CH₃ | -CH=CH₂ |
| 697 | -CH₂CH(CH₃)₂ | -CH=CH₂ |
| 698 | -C(CH₃)₃ | -CH=CH₂ |
| 699 | -CH₂(CH₂)₃CH₃ | -CH=CH₂ |
| 700 | -CH(CH₃)(CH₂)₂CH₃ | -CH=CH₂ |
| 701 | -CH₂CH(CH₃)CH₂CH₃ | -CH=CH₂ |
| 702 | -(CH₂)₂CH(CH₃)₂ | -CH=CH₂ |
| 703 | -CH₂(CH₂)₄CH₃ | -CH=CH₂ |
| 704 | -CH₂(CH₂)₅CH₃ | -CH=CH₂ |
| 705 | -CH₂(CH₂)₆CH₃ | -CH=CH₂ |
| 706 | -CH=CH₂ | -CH=CH₂ |
| 707 | -CH=CHCH₃ | -CH=CH₂ |
| 708 | -CH=C(CH₃)₂ | -CH=CH₂ |
| 709 | -C(CH₃)=CH₂ | -CH=CH₂ |
| 710 | -C(CH₃)=CHCH₃ | -CH=CH₂ |
| 711 | -C(CH₃)=C(CH₃)₂ | -CH=CH₂ |
| 712 | -CH=CH-CH₂CH₃ | -CH=CH₂ |
| 713 | -CH₂CH=CH-CH₃ | -CH=CH₂ |
| 714 | -(CH₂)₂CH=CH₂ | -CH=CH₂ |
| 715 | -CH=CH-(CH₂)₂CH₃ | -CH=CH₂ |
| 716 | -CH₂CH=CH-CH₂CH₃ | -CH=CH₂ |
| 717 | -(CH₂)₂CH=CH-CH₃ | -CH=CH₂ |
| 718 | -(CH₂)₃CH=CH₂ | -CH=CH₂ |
| 719 | -CH=CH-(CH₂)₃CH₃ | -CH=CH₂ |
| 720 | -CH₂CH=CH-(CH₂)₂CH₃ | -CH=CH₂ |
| 721 | -(CH₂)₂CH=CH-CH₂CH₃ | -CH=CH₂ |
| 722 | -(CH₂)₃CH=CH-CH₃ | -CH=CH₂ |
| 723 | -(CH₂)₄CH=CH₂ | -CH=CH₂ |
| 724 | -CH=CH-(CH₂)₃CH₃ | -CH=CH₂ |
| 725 | -CH₂CH=CH-(CH₂)₃CH₃ | -CH=CH₂ |
| 726 | -(CH₂)₂CH=CH-(CH₂)₂CH₃ | -CH=CH₂ |
| 727 | -(CH₂)₃CH=CH-CH₂CH₃ | -CH=CH₂ |
| 728 | -(CH₂)₄CH=CH-CH₃ | -CH=CH₂ |
| 729 | -(CH₂)₅CH=CH₂ | -CH=CH₂ |
| 730 | -CH=CH-(CH₂)₅CH₃ | -CH=CH₂ |
| 731 | -CH₂CH=CH-(CH₂)₄CH₃ | -CH=CH₂ |
| 732 | -(CH₂)₂CH=CH-(CH₂)₃CH₃ | -CH=CH₂ |
| 733 | -(CH₂)₃CH=CH-(CH₂)₂CH₃ | -CH=CH₂ |
| 734 | -(CH₂)₄CH=CH-CH₂CH₃ | -CH=CH₂ |
| 735 | -(CH₂)₅CH=CH-CH₃ | -CH=CH₂ |
| 736 | -(CH₂)₆CH=CH₂ | -CH=CH₂ |
| 737 | -CH₃ | -C(CH₃)=CH₂ |
| 738 | -CH₂CH₃ | -C(CH₃)=CH₂ |
| 739 | -CH₂CH₂CH₃ | -C(CH₃)=CH₂ |
| 740 | -CH(CH₃)₂ | -C(CH₃)=CH₂ |
| 741 | -CH₂CH₂CH₂CH₃ | -C(CH₃)=CH₂ |
| 742 | -CH(CH₃)CH₂CH₃ | -C(CH₃)=CH₂ |
| 743 | -CH₂CH(CH₃)₂ | -C(CH₃)=CH₂ |
| 744 | -C(CH₃)₃ | -C(CH₃)=CH₂ |
| 745 | -CH₂(CH₂)₃CH₃ | -C(CH₃)=CH₂ |
| 746 | -CH(CH₃)(CH₂)₂CH₃ | -C(CH₃)=CH₂ |
| 747 | -CH₂CH(CH₃)CH₂CH₃ | -C(CH₃)=CH₂ |
| 748 | -(CH₂)₂CH(CH₃)₂ | -C(CH₃)=CH₂ |
| 749 | -CH₂(CH₂)₄CH₃ | -C(CH₃)=CH₂ |
| 750 | -CH₂(CH₂)₅CH₃ | -C(CH₃)=CH₂ |
| 751 | -CH₂(CH₂)₆CH₃ | -C(CH₃)=CH₂ |
| 752 | -CH=CH₂ | -C(CH₃)=CH₂ |
| 753 | -CH=CHCH₃ | -C(CH₃)=CH₂ |
| 754 | -CH=C(CH₃)₂ | -C(CH₃)=CH₂ |
| 755 | -C(CH₃)=CH₂ | -C(CH₃)=CH₂ |
| 756 | -C(CH₃)=CHCH₃ | -C(CH₃)=CH₂ |
| 757 | -C(CH₃)=C(CH₃)₂ | -C(CH₃)=CH₂ |
| 758 | -CH=CH-CH₂CH₃ | -C(CH₃)=CH₂ |
| 759 | -CH₂CH=CH-CH₃ | -C(CH₃)=CH₂ |
| 760 | -(CH₂)₂CH=CH₂ | -C(CH₃)=CH₂ |
| 761 | -CH=CH-(CH₂)₂CH₃ | -C(CH₃)=CH₂ |
| 762 | -CH₂CH=CH-CH₂CH₃ | -C(CH₃)=CH₂ |
| 763 | -(CH₂)₂CH=CH-CH₃ | -C(CH₃)=CH₂ |
| 764 | -(CH₂)₃CH=CH₂ | -C(CH₃)=CH₂ |
| 765 | -CH=CH-(CH₂)₃CH₃ | -C(CH₃)=CH₂ |
| 766 | -CH₂CH=CH-(CH₂)₂CH₃ | -C(CH₃)=CH₂ |
| 767 | -(CH₂)₂CH=CH-CH₂CH₃ | -C(CH₃)=CH₂ |
| 768 | -(CH₂)₃CH=CH-CH₃ | -C(CH₃)=CH₂ |
| 769 | -(CH₂)₄CH=CH₂ | -C(CH₃)=CH₂ |
| 770 | -CH=CH-(CH₂)₃CH₃ | -C(CH₃)=CH₂ |
| 771 | -CH₂CH=CH-(CH₂)₃CH₃ | -C(CH₃)=CH₂ |
| 772 | -(CH₂)₂CH=CH-(CH₂)₂CH₃ | -C(CH₃)=CH₂ |
| 773 | -(CH₂)₃CH=CH-CH₂CH₃ | -C(CH₃)=CH₂ |
| 774 | -(CH₂)₄CH=CH-CH₃ | -C(CH₃)=CH₂ |
| 775 | -(CH₂)₅CH=CH₂ | -C(CH₃)=CH₂ |
| 776 | -CH=CH-(CH₂)₅CH₃ | -C(CH₃)=CH₂ |
| 777 | -CH₂CH=CH-(CH₂)₄CH₃ | -C(CH₃)=CH₂ |
| 778 | -(CH₂)₂CH=CH-(CH₂)₃CH₃ | -C(CH₃)=CH₂ |
| 779 | -(CH₂)₃CH=CH-(CH₂)₂CH₃ | -C(CH₃)=CH₂ |
| 780 | -(CH₂)₄CH=CH-CH₂CH₃ | -C(CH₃)=CH₂ |
| 781 | -(CH₂)₅CH=CH-CH₃ | -C(CH₃)=CH₂ |
| 782 | -(CH₂)₆CH=CH₂ | -C(CH₃)=CH₂ |
| 783 | -CH₃ | -O-CH₃ |
| 784 | -CH₂CH₃ | -O-CH₃ |
| 785 | -CH₂CH₂CH₃ | -O-CH₃ |
| 786 | -CH(CH₃)₂ | -O-CH₃ |
| 787 | -CH₂CH₂CH₂CH₃ | -O-CH₃ |
| 788 | -CH(CH₃)CH₂CH₃ | -O-CH₃ |
| 789 | -CH₂CH(CH₃)₂ | -O-CH₃ |
| 790 | -C(CH₃)₃ | -O-CH₃ |
| 791 | -CH₂(CH₂)₃CH₃ | -O-CH₃ |
| 792 | -CH(CH₃)(CH₂)₂CH₃ | -O-CH₃ |
| 793 | -CH₂CH(CH₃)CH₂CH₃ | -O-CH₃ |
| 794 | -(CH₂)₂CH(CH₃)₂ | -O-CH₃ |
| 795 | -CH₂(CH₂)₄CH₃ | -O-CH₃ |
| 796 | -CH₂(CH₂)₅CH₃ | -O-CH₃ |
| 797 | -CH₂(CH₂)₆CH₃ | -O-CH₃ |
| 798 | -CH=CH₂ | -O-CH₃ |
| 799 | -CH=CHCH₃ | -O-CH₃ |
| 800 | -CH=C(CH₃)₂ | -O-CH₃ |
| 801 | -C(CH₃)=CH₂ | -O-CH₃ |
| 802 | -C(CH₃)=CHCH₃ | -O-CH₃ |
| 803 | -C(CH₃)=C(CH₃)₂ | -O-CH₃ |
| 804 | -CH=CH-CH₂CH₃ | -O-CH₃ |
| 805 | -CH₂CH=CH-CH₃ | -O-CH₃ |
| 806 | -(CH₂)₂CH=CH₂ | -O-CH₃ |
| 807 | -CH=CH-(CH₂)₂CH₃ | -O-CH₃ |
| 808 | -CH₂CH=CH-CH₂CH₃ | -O-CH₃ |
| 809 | -(CH₂)₂CH=CH-CH₃ | -O-CH₃ |
| 810 | -(CH₂)₃CH=CH₂ | -O-CH₃ |
| 811 | -CH=CH-(CH₂)₃CH₃ | -O-CH₃ |
| 812 | -CH₂CH=CH-(CH₂)₂CH₃ | -O-CH₃ |
| 813 | -(CH₂)₂CH=CH-CH₂CH₃ | -O-CH₃ |
| 814 | -(CH₂)₃CH=CH-CH₃ | -O-CH₃ |
| 815 | -(CH₂)₄CH=CH₂ | -O-CH₃ |
| 816 | -CH=CH-(CH₂)₃CH₃ | -O-CH₃ |
| 817 | -CH₂CH=CH-(CH₂)₃CH₃ | -O-CH₃ |
| 818 | -(CH₂)₂CH=CH-(CH₂)₂CH₃ | -O-CH₃ |
| 819 | -(CH₂)₃CH=CH-CH₂CH₃ | -O-CH₃ |
| 820 | -(CH₂)₄CH=CH-CH₃ | -O-CH₃ |
| 821 | -(CH₂)₅CH=CH₂ | -O-CH₃ |
| 822 | -CH=CH-(CH₂)₅CH₃ | -O-CH₃ |
| 823 | -CH₂CH=CH-(CH₂)₄CH₃ | -O-CH₃ |
| 824 | -(CH₂)₂CH=CH-(CH₂)₃CH₃ | -O-CH₃ |
| 825 | -(CH₂)₃CH=CH-(CH₂)₂CH₃ | -O-CH₃ |
| 826 | -(CH₂)₄CH=CH-CH₂CH₃ | -O-CH₃ |
| 827 | -(CH₂)₅CH=CH-CH₃ | -O-CH₃ |
| 828 | -(CH₂)₆CH=CH₂ | -O-CH₃ |
| 829 | -CH₃ | -O-CH₂CH₃ |
| 830 | -CH₂CH₃ | -O-CH₂CH₃ |
| 831 | -CH₂CH₂CH₃ | -O-CH₂CH₃ |
| 832 | -CH(CH₃)₂ | -O-CH₂CH₃ |
| 833 | -CH₂CH₂CH₂CH₃ | -O-CH₂CH₃ |
| 834 | -CH(CH₃)CH₂CH₃ | -O-CH₂CH₃ |
| 835 | -CH₂CH(CH₃)₂ | -O-CH₂CH₃ |
| 836 | -C(CH₃)₃ | -O-CH₂CH₃ |
| 837 | -CH₂(CH₂)₃CH₃ | -O-CH₂CH₃ |
| 838 | -CH(CH₃)(CH₂)₂CH₃ | -O-CH₂CH₃ |
| 839 | -CH₂CH(CH₃)CH₂CH₃ | -O-CH₂CH₃ |
| 840 | -(CH₂)₂CH(CH₃)₂ | -O-CH₂CH₃ |
| 841 | -CH₂(CH₂)₄CH₃ | -O-CH₂CH₃ |
| 842 | -CH₂(CH₂)₅CH₃ | -O-CH₂CH₃ |
| 843 | -CH₂(CH₂)₆CH₃ | -O-CH₂CH₃ |
| 844 | -CH=CH₂ | -O-CH₂CH₃ |
| 845 | -CH=CHCH₃ | -O-CH₂CH₃ |
| 846 | -CH=C(CH₃)₂ | -O-CH₂CH₃ |
| 847 | -C(CH₃)=CH₂ | -O-CH₂CH₃ |
| 848 | -C(CH₃)=CHCH₃ | -O-CH₂CH₃ |
| 849 | -C(CH₃)=C(CH₃)₂ | -O-CH₂CH₃ |
| 850 | -CH=CH-CH₂CH₃ | -O-CH₂CH₃ |
| 851 | -CH₂CH=CH-CH₃ | -O-CH₂CH₃ |
| 852 | -(CH₂)₂CH=CH₂ | -O-CH₂CH₃ |
| 853 | -CH=CH-(CH₂)₂CH₃ | -O-CH₂CH₃ |
| 854 | -CH₂CH=CH-CH₂CH₃ | -O-CH₂CH₃ |
| 855 | -(CH₂)₂CH=CH-CH₃ | -O-CH₂CH₃ |
| 856 | -(CH₂)₃CH=CH₂ | -O-CH₂CH₃ |
| 857 | -CH=CH-(CH₂)₃CH₃ | -O-CH₂CH₃ |
| 858 | -CH₂CH=CH-(CH₂)₂CH₃ | -O-CH₂CH₃ |
| 859 | -(CH₂)₂CH=CH-CH₂CH₃ | -O-CH₂CH₃ |
| 860 | -(CH₂)₃CH=CH-CH₃ | -O-CH₂CH₃ |
| 861 | -(CH₂)₄CH=CH₂ | -O-CH₂CH₃ |
| 862 | -CH=CH-(CH₂)₃CH₃ | -O-CH₂CH₃ |
| 863 | -CH₂CH=CH-(CH₂)₃CH₃ | -O-CH₂CH₃ |
| 864 | -(CH₂)₂CH=CH-(CH₂)₂CH₃ | -O-CH₂CH₃ |
| 865 | -(CH₂)₃CH=CH-CH₂CH₃ | -O-CH₂CH₃ |
| 866 | -(CH₂)₄CH=CH-CH₃ | -O-CH₂CH₃ |
| 867 | -(CH₂)₅CH=CH₂ | -O-CH₂CH₃ |
| 868 | -CH=CH-(CH₂)₅CH₃ | -O-CH₂CH₃ |
| 869 | -CH₂CH=CH-(CH₂)₄CH₃ | -O-CH₂CH₃ |
| 870 | -(CH₂)₂CH=CH-(CH₂)₃CH₃ | -O-CH₂CH₃ |
| 871 | -(CH₂)₃CH=CH-(CH₂)₂CH₃ | -O-CH₂CH₃ |
| 872 | -(CH₂)₄CH=CH-CH₂CH₃ | -O-CH₂CH₃ |
| 873 | -(CH₂)₅CH=CH-CH₃ | -O-CH₂CH₃ |
| 874 | -(CH₂)₆CH=CH₂ | -O-CH₂CH₃ |
| 875 | -CH₃ | -O-CH₂CH₂CH₃ |
| 876 | -CH₂CH₃ | -O-CH₂CH₂CH₃ |
| 877 | -CH₂CH₂CH₃ | -O-CH₂CH₂CH₃ |
| 878 | -CH(CH₃)₂ | -O-CH₂CH₂CH₃ |
| 879 | -CH₂CH₂CH₂CH₃ | -O-CH₂CH₂CH₃ |
| 880 | -CH(CH₃)CH₂CH₃ | -O-CH₂CH₂CH₃ |
| 881 | -CH₂CH(CH₃)₂ | -O-CH₂CH₂CH₃ |
| 882 | -C(CH₃)₃ | -O-CH₂CH₂CH₃ |
| 883 | -CH₂(CH₂)₃CH₃ | -O-CH₂CH₂CH₃ |
| 884 | -CH(CH₃)(CH₂)₂CH₃ | -O-CH₂CH₂CH₃ |
| 885 | -CH₂CH(CH₃)CH₂CH₃ | -O-CH₂CH₂CH₃ |
| 886 | -(CH₂)₂CH(CH₃)₂ | -O-CH₂CH₂CH₃ |
| 887 | -CH₂(CH₂)₄CH₃ | -O-CH₂CH₂CH₃ |
| 888 | -CH₂(CH₂)₅CH₃ | -O-CH₂CH₂CH₃ |
| 889 | -CH₂(CH₂)₆CH₃ | -O-CH₂CH₂CH₃ |
| 890 | -CH=CH₂ | -O-CH₂CH₂CH₃ |
| 891 | -CH=CHCH₃ | -O-CH₂CH₂CH₃ |
| 892 | -CH=C(CH₃)₂ | -O-CH₂CH₂CH₃ |
| 893 | -C(CH₃)=CH₂ | -O-CH₂CH₂CH₃ |
| 894 | -C(CH₃)=CHCH₃ | -O-CH₂CH₂CH₃ |
| 895 | -C(CH₃)=C(CH₃)₂ | -O-CH₂CH₂CH₃ |
| 896 | -CH=CH-CH₂CH₃ | -O-CH₂CH₂CH₃ |
| 897 | -CH₂CH=CH-CH₃ | -O-CH₂CH₂CH₃ |
| 898 | -(CH₂)₂CH=CH₂ | -O-CH₂CH₂CH₃ |
| 899 | -CH=CH-(CH₂)₂CH₃ | -O-CH₂CH₂CH₃ |
| 900 | -CH₂CH=CH-CH₂CH₃ | -O-CH₂CH₂CH₃ |
| 901 | -(CH₂)₂CH=CH-CH₃ | -O-CH₂CH₂CH₃ |
| 902 | -(CH₂)₃CH=CH₂ | -O-CH₂CH₂CH₃ |
| 903 | -CH=CH-(CH₂)₃CH₃ | -O-CH₂CH₂CH₃ |
| 904 | -CH₂CH=CH-(CH₂)₂CH₃ | -O-CH₂CH₂CH₃ |
| 905 | -(CH₂)₂CH=CH-CH₂CH₃ | -O-CH₂CH₂CH₃ |
| 906 | -(CH₂)₃CH=CH-CH₃ | -O-CH₂CH₂CH₃ |
| 907 | -(CH₂)₄CH=CH₂ | -O-CH₂CH₂CH₃ |
| 908 | -CH=CH-(CH₂)₃CH₃ | -O-CH₂CH₂CH₃ |
| 909 | -CH₂CH=CH-(CH₂)₃CH₃ | -O-CH₂CH₂CH₃ |
| 910 | -(CH₂)₂CH=CH-(CH₂)₂CH₃ | -O-CH₂CH₂CH₃ |
| 911 | -(CH₂)₃CH=CH-CH₂CH₃ | -O-CH₂CH₂CH₃ |
| 912 | -(CH₂)₄CH=CH-CH₃ | -O-CH₂CH₂CH₃ |
| 913 | -(CH₂)₅CH=CH₂ | -O-CH₂CH₂CH₃ |
| 914 | -CH=CH-(CH₂)₅CH₃ | -O-CH₂CH₂CH₃ |
| 915 | -CH₂CH=CH-(CH₂)₄CH₃ | -O-CH₂CH₂CH₃ |
| 916 | -(CH₂)₂CH=CH-(CH₂)₃CH₃ | -O-CH₂CH₂CH₃ |
| 917 | -(CH₂)₃CH=CH-(CH₂)₂CH₃ | -O-CH₂CH₂CH₃ |
| 918 | -(CH₂)₄CH=CH-CH₂CH₃ | -O-CH₂CH₂CH₃ |
| 919 | -(CH₂)₅CH=CH-CH₃ | -O-CH₂CH₂CH₃ |
| 920 | -(CH₂)₆CH=CH₂ | -O-CH₂CH₂CH₃ |
| 921 | -CH₃ | -O-CH(CH₃)₂ |
| 922 | -CH₂CH₃ | -O-CH(CH₃)₂ |
| 923 | -CH₂CH₂CH₃ | -O-CH(CH₃)₂ |
| 924 | -CH(CH₃)₂ | -O-CH(CH₃)₂ |
| 925 | -CH₂CH₂CH₂CH₃ | -O-CH(CH₃)₂ |
| 926 | -CH(CH₃)CH₂CH₃ | -O-CH(CH₃)₂ |
| 927 | -CH₂CH(CH₃)₂ | -O-CH(CH₃)₂ |
| 928 | -C(CH₃)₃ | -O-CH(CH₃)₂ |
| 929 | -CH₂(CH₂)₃CH₃ | -O-CH(CH₃)₂ |
| 930 | -CH(CH₃)(CH₂)₂CH₃ | -O-CH(CH₃)₂ |
| 931 | -CH₂CH(CH₃)CH₂CH₃ | -O-CH(CH₃)₂ |
| 932 | -(CH₂)₂CH(CH₃)₂ | -O-CH(CH₃)₂ |
| 933 | -CH₂(CH₂)₄CH₃ | -O-CH(CH₃)₂ |
| 934 | -CH₂(CH₂)₅CH₃ | -O-CH(CH₃)₂ |
| 935 | -CH₂(CH₂)₆CH₃ | -O-CH(CH₃)₂ |
| 936 | -CH=CH₂ | -O-CH(CH₃)₂ |
| 937 | -CH=CHCH₃ | -O-CH(CH₃)₂ |
| 938 | -CH=C(CH₃)₂ | -O-CH(CH₃)₂ |
| 939 | -C(CH₃)=CH₂ | -O-CH(CH₃)₂ |
| 940 | -C(CH₃)=CHCH₃ | -O-CH(CH₃)₂ |
| 941 | -C(CH₃)=C(CH₃)₂ | -O-CH(CH₃)₂ |
| 942 | -CH=CH-CH₂CH₃ | -O-CH(CH₃)₂ |
| 943 | -CH₂CH=CH-CH₃ | -O-CH(CH₃)₂ |
| 944 | -(CH₂)₂CH=CH₂ | -O-CH(CH₃)₂ |
| 945 | -CH=CH-(CH₂)₂CH₃ | -O-CH(CH₃)₂ |
| 946 | -CH₂CH=CH-CH₂CH₃ | -O-CH(CH₃)₂ |
| 947 | -(CH₂)₂CH=CH-CH₃ | -O-CH(CH₃)₂ |
| 948 | -(CH₂)₃CH=CH₂ | -O-CH(CH₃)₂ |
| 949 | -CH=CH-(CH₂)₃CH₃ | -O-CH(CH₃)₂ |
| 950 | -CH₂CH=CH-(CH₂)₂CH₃ | -O-CH(CH₃)₂ |
| 951 | -(CH₂)₂CH=CH-CH₂CH₃ | -O-CH(CH₃)₂ |
| 952 | -(CH₂)₃CH=CH-CH₃ | -O-CH(CH₃)₂ |
| 953 | -(CH₂)₄CH=CH₂ | -O-CH(CH₃)₂ |
| 954 | -CH=CH-(CH₂)₃CH₃ | -O-CH(CH₃)₂ |
| 955 | -CH₂CH=CH-(CH₂)₃CH₃ | -O-CH(CH₃)₂ |
| 956 | -(CH₂)₂CH=CH-(CH₂)₂CH₃ | -O-CH(CH₃)₂ |
| 957 | -(CH₂)₃CH=CH-CH₂CH₃ | -O-CH(CH₃)₂ |
| 958 | -(CH₂)₄CH=CH-CH₃ | -O-CH(CH₃)₂ |
| 959 | -(CH₂)₅CH=CH₂ | -O-CH(CH₃)₂ |
| 960 | -CH=CH-(CH₂)₅CH₃ | -O-CH(CH₃)₂ |
| 961 | -CH₂CH=CH-(CH₂)₄CH₃ | -O-CH(CH₃)₂ |
| 962 | -(CH₂)₂CH=CH-(CH₂)₃CH₃ | -O-CH(CH₃)₂ |
| 963 | -(CH₂)₃CH=CH-(CH₂)₂CH₃ | -O-CH(CH₃)₂ |
| 964 | -(CH₂)₄CH=CH-CH₂CH₃ | -O-CH(CH₃)₂ |
| 965 | -(CH₂)₅CH=CH-CH₃ | -O-CH(CH₃)₂ |
| 966 | -(CH₂)₆CH=CH₂ | -O-CH(CH₃)₂ |

Als weiterhin besonders geeignet haben sich im Rahmen der vorliegenden Erfindung Carvonacetale der Formel 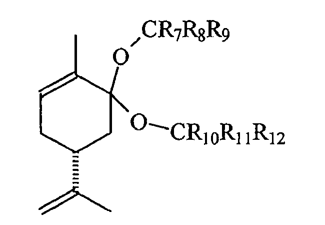 erwiesen, in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, - CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, C(CH₃)₃

Einzelne Spezies, die der vorstehend genannten Formel genügen, sind hinsichtlich ihrer Reste R7 bis R12 in der nachstehenden Tabelle 3 aufgeführt. Diese sind erfindungsgemäß besonders bevorzugt.

**Tabelle 3: Bevorzugte Carvonacetale**

| **Nr.** | **R7** | **R8** | **R9** | **R10** | **R11** | **R12** |
|---|---|---|---|---|---|---|
| 1 | -H | -H | -H | -H | -H | -H |
| 2 | -CH₃ | -H | -H | -H | -H | -H |
| 3 | -CH₂CH₃ | -H | -H | -H | -H | -H |
| 4 | -CH₂CH₂CH₃ | -H | -H | -H | -H | -H |
| 5 | -CH(CH₃)₂ | -H | -H | -H | -H | -H |
| 6 | -CH₂CH₂CH₂CH₃ | -H | -H | -H | -H | -H |
| 7 | -CH(CH₃)CH₂CH₃ | -H | -H | -H | -H | -H |
| 8 | -CH₂CH(CH₃)₂ | -H | -H | -H | -H | -H |
| 9 | -C(CH₃)₃ | -H | -H | -H | -H | -H |
| 10 | -H | -CH₃ | -H | -H | -H | -H |
| 11 | -CH₃ | -CH₃ | -H | -H | -H | -H |
| 12 | -CH₂CH₃ | -CH₃ | -H | -H | -H | -H |
| 13 | -CH₂CH₂CH₃ | -CH₃ | -H | -H | -H | -H |
| 14 | -CH(CH₃)₂ | -CH₃ | -H | -H | -H | -H |
| 15 | -CH₂CH₂CH₂CH₃ | -CH₃ | -H | -H | -H | -H |
| 16 | -CH(CH₃)CH₂CH₃ | -CH₃ | -H | -H | -H | -H |
| 17 | -CH₂CH(CH₃)₂ | -CH₃ | -H | -H | -H | -H |
| 18 | -C(CH₃)₃ | -CH₃ | -H | -H | -H | -H |
| 19 | -H | -CH₃ | -CH₃ | -H | -H | -H |
| 20 | -CH₃ | -CH₃ | -CH₃ | -H | -H | -H |
| 21 | -CH₂CH₃ | -CH₃ | -CH₃ | -H | -H | -H |
| 22 | -CH₂CH₂CH₃ | -CH₃ | -CH₃ | -H | -H | -H |
| 23 | -CH(CH₃)₂ | -CH₃ | -CH₃ | -H | -H | -H |
| 24 | -CH₂CH₂CH₂CH₃ | -CH₃ | -CH₃ | -H | -H | -H |
| 25 | -CH(CH₃)CH₂CH₃ | -CH₃ | -CH₃ | -H | -H | -H |
| 26 | -CH₂CH(CH₃)₂ | -CH₃ | -CH₃ | -H | -H | -H |
| 27 | -C(CH₃)₃ | -CH₃ | -CH₃ | -H | -H | -H |
| 28 | -H | -CH₂CH₃ | -H | -H | -H | -H |
| 29 | -CH₃ | -CH₂CH₃ | -H | -H | -H | -H |
| 30 | -CH₂CH₃ | -CH₂CH₃ | -H | -H | -H | -H |
| 31 | -CH₂CH₂CH₃ | -CH₂CH₃ | -H | -H | -H | -H |
| 32 | -CH(CH₃)₂ | -CH₂CH₃ | -H | -H | -H | -H |
| 33 | -CH₂CH₂CH₂CH₃ | -CH₂CH₃ | -H | -H | -H | -H |
| 34 | -CH(CH₃)CH₂CH₃ | -CH₂CH₃ | -H | -H | -H | -H |
| 35 | -CH₂CH(CH₃)₂ | -CH₂CH₃ | -H | -H | -H | -H |
| 36 | -C(CH₃)₃ | -CH₂CH₃ | -H | -H | -H | -H |
| 37 | -H | -CH₂CH₃ | -CH₃ | -H | -H | -H |
| 38 | -CH₃ | -CH₂CH₃ | -CH₃ | -H | -H | -H |
| 39 | -CH₂CH₃ | -CH₂CH₃ | -CH₃ | -H | -H | -H |
| 40 | -CH₂CH₂CH₃ | -CH₂CH₃ | -CH₃ | -H | -H | -H |
| 41 | -CH(CH₃)₂ | -CH₂CH₃ | -CH₃ | -H | -H | -H |
| 42 | -CH₂CH₂CH₂CH₃ | -CH₂CH₃ | -CH₃ | -H | -H | -H |
| 43 | -CH(CH₃)CH₂CH₃ | -CH₂CH₃ | -CH₃ | -H | -H | -H |
| 44 | -CH₂CH(CH₃)₂ | -CH₂CH₃ | -CH₃ | -H | -H | -H |
| 45 | -C(CH₃)₃ | -CH₂CH₃ | -CH₃ | -H | -H | -H |
| 46 | -H | -CH₂CH₃ | -CH₂CH₃ | -H | -H | -H |
| 47 | -CH₃ | -CH₂CH₃ | -CH₂CH₃ | -H | -H | -H |
| 48 | -CH₂CH₃ | -CH₂CH₃ | -CH₂CH₃ | -H | -H | -H |
| 49 | -CH₂CH₂CH₃ | -CH₂CH₃ | -CH₂CH₃ | -H | -H | -H |
| 50 | -CH(CH₃)₂ | -CH₂CH₃ | -CH₂CH₃ | -H | -H | -H |
| 51 | -CH₂CH₂CH₂CH₃ | -CH₂CH₃ | -CH₂CH₃ | -H | -H | -H |
| 52 | -CH(CH₃)CH₂CH₃ | -CH₂CH₃ | -CH₂CH₃ | -H | -H | -H |
| 53 | -CH₂CH(CH₃)₂ | -CH₂CH₃ | -CH₂CH₃ | -H | -H | -H |
| 54 | -C(CH₃)₃ | -CH₂CH₃ | -CH₂CH₃ | -H | -H | -H |
| 55 | -H | -CH₂CH₂CH₃ | -H | -H | -H | -H |
| 56 | -CH₃ | -CH₂CH₂CH₃ | -H | -H | -H | -H |
| 57 | -CH₂CH₃ | -CH₂CH₂CH₃ | -H | -H | -H | -H |
| 58 | -CH₂CH₂CH₃ | -CH₂CH₂CH₃ | -H | -H | -H | -H |
| 59 | -CH(CH₃)₂ | -CH₂CH₂CH₃ | -H | -H | -H | -H |
| 60 | -CH₂CH₂CH₂CH₃ | -CH₂CH₂CH₃ | -H | -H | -H | -H |
| 61 | -CH(CH₃)CH₂CH₃ | -CH₂CH₂CH₃ | -H | -H | -H | -H |
| 62 | -CH₂CH(CH₃)₂ | -CH₂CH₂CH₃ | -H | -H | -H | -H |
| 63 | -C(CH₃)₃ | -CH₂CH₂CH₃ | -H | -H | -H | -H |
| 64 | -H | -CH₂CH₂CH₃ | -CH₃ | -H | -H | -H |
| 65 | -CH₃ | -CH₂CH₂CH₃ | -CH₃ | -H | -H | -H |
| 66 | -CH₂CH₃ | -CH₂CH₂CH₃ | -CH₃ | -H | -H | -H |
| 67 | -CH₂CH₂CH₃ | -CH₂CH₂CH₃ | -CH₃ | -H | -H | -H |
| 68 | -CH(CH₃)₂ | -CH₂CH₂CH₃ | -CH₃ | -H | -H | -H |
| 69 | -CH₂CH₂CH₂CH₃ | -CH₂CH₂CH₃ | -CH₃ | -H | -H | -H |
| 70 | -CH(CH₃)CH₂CH₃ | -CH₂CH₂CH₃ | -CH₃ | -H | -H | -H |
| 71 | -CH₂CH(CH₃)₂ | -CH₂CH₂CH₃ | -CH₃ | -H | -H | -H |
| 72 | -C(CH₃)₃ | -CH₂CH₂CH₃ | -CH₃ | -H | -H | -H |
| 73 | -H | -CH₂CH₂CH₃ | -CH₂CH₃ | -H | -H | -H |
| 74 | -CH₃ | -CH₂CH₂CH₃ | -CH₂CH₃ | -H | -H | -H |
| 75 | -CH₂CH₃ | -CH₂CH₂CH₃ | -CH₂CH₃ | -H | -H | -H |
| 76 | -CH₂CH₂CH₃ | -CH₂CH₂CH₃ | -CH₂CH₃ | -H | -H | -H |
| 77 | -CH(CH₃)₂ | -CH₂CH₂CH₃ | -CH₂CH₃ | -H | -H | -H |
| 78 | -CH₂CH₂CH₂CH₃ | -CH₂CH₂CH₃ | -CH₂CH₃ | -H | -H | -H |
| 79 | -CH(CH₃)CH₂CH₃ | -CH₂CH₂CH₃ | -CH₂CH₃ | -H | -H | -H |
| 80 | -CH₂CH(CH₃)₂ | -CH₂CH₂CH₃ | -CH₂CH₃ | -H | -H | -H |
| 81 | -C(CH₃)₃ | -CH₂CH₂CH₃ | -CH₂CH₃ | -H | -H | -H |
| 82 | -H | -CH₂CH₂CH₃ | -CH₂CH₂CH₃ | -H | -H | -H |
| 83 | -CH₃ | -CH₂CH₂CH₃ | -CH₂CH₂CH₃ | -H | -H | -H |
| 84 | -CH₂CH₃ | -CH₂CH₂CH₃ | -CH₂CH₂CH₃ | -H | -H | -H |
| 85 | -CH₂CH₂CH₃ | -CH₂CH₂CH₃ | -CH₂CH₂CH₃ | -H | -H | -H |
| 86 | -CH(CH₃)₂ | -CH₂CH₂CH₃ | -CH₂CH₂CH₃ | -H | -H | -H |
| 87 | -CH₂CH₂CH₂CH₃ | -CH₂CH₂CH₃ | -CH₂CH₂CH₃ | -H | -H | -H |
| 88 | -CH(CH₃)CH₂CH₃ | -CH₂CH₂CH₃ | -CH₂CH₂CH₃ | -H | -H | -H |
| 89 | -CH₂CH(CH₃)₂ | -CH₂CH₂CH₃ | -CH₂CH₂CH₃ | -H | -H | -H |
| 90 | -C(CH₃)₃ | -CH₂CH₂CH₃ | -CH₂CH₂CH₃ | -H | -H | -H |
| 91 | -H | -CH(CH₃)₂ | -H | -H | -H | -H |
| 92 | -CH₃ | -CH(CH₃)₂ | -H | -H | -H | -H |
| 93 | -CH₂CH₃ | -CH(CH₃)₂ | -H | -H | -H | -H |
| 94 | -CH₂CH₂CH₃ | -CH(CH₃)₂ | -H | -H | -H | -H |
| 95 | -CH(CH₃)₂ | -CH(CH₃)₂ | -H | -H | -H | -H |
| 96 | -CH₂CH₂CH₂CH₃ | -CH(CH₃)₂ | -H | -H | -H | -H |
| 97 | -CH(CH₃)CH₂CH₃ | -CH(CH₃)₂ | -H | -H | -H | -H |
| 98 | -CH₂CH(CH₃)₂ | -CH(CH₃)₂ | -H | -H | -H | -H |
| 99 | -C(CH₃)₃ | -CH(CH₃)₂ | -H | -H | -H | -H |
| 100 | -H | -CH(CH₃)₂ | -CH₃ | -H | -H | -H |
| 101 | -CH₃ | -CH(CH₃)₂ | -CH₃ | -H | -H | -H |
| 102 | -CH₂CH₃ | -CH(CH₃)₂ | -CH₃ | -H | -H | -H |
| 103 | -CH₂CH₂CH₃ | -CH(CH₃)₂ | -CH₃ | -H | -H | -H |
| 104 | -CH(CH₃)₂ | -CH(CH₃)₂ | -CH₃ | -H | -H | -H |
| 105 | -CH₂CH₂CH₂CH₃ | -CH(CH₃)₂ | -CH₃ | -H | -H | -H |
| 106 | -CH(CH₃)CH₂CH₃ | -CH(CH₃)₂ | -CH₃ | -H | -H | -H |
| 107 | -CH₂CH(CH₃)₂ | -CH(CH₃)₂ | -CH₃ | -H | -H | -H |
| 108 | -C(CH₃)₃ | -CH(CH₃)₂ | -CH₃ | -H | -H | -H |
| 109 | -H | -CH(CH₃)₂ | -CH₂CH₃ | -H | -H | -H |
| 110 | -CH₃ | -CH(CH₃)₂ | -CH₂CH₃ | -H | -H | -H |
| 111 | -CH₂CH₃ | -CH(CH₃)₂ | -CH₂CH₃ | -H | -H | -H |
| 112 | -CH₂CH₂CH₃ | -CH(CH₃)₂ | -CH₂CH₃ | -H | -H | -H |
| 113 | -CH(CH₃)₂ | -CH(CH₃)₂ | -CH₂CH₃ | -H | -H | -H |
| 114 | -CH₂CH₂CH₂CH₃ | -CH(CH₃)₂ | -CH₂CH₃ | -H | -H | -H |
| 115 | -CH(CH₃)CH₂CH₃ | -CH(CH₃)₂ | -CH₂CH₃ | -H | -H | -H |
| 116 | -CH₂CH(CH₃)₂ | -CH(CH₃)₂ | -CH₂CH₃ | -H | -H | -H |
| 117 | -C(CH₃)₃ | -CH(CH₃)₂ | -CH₂CH₃ | -H | -H | -H |
| 118 | -H | -CH(CH₃)₂ | -CH₂CH₂CH₃ | -H | -H | -H |
| 119 | -CH₃ | -CH(CH₃)₂ | -CH₂CH₂CH₃ | -H | -H | -H |
| 120 | -CH₂CH₃ | -CH(CH₃)₂ | -CH₂CH₂CH₃ | -H | -H | -H |
| 121 | -CH₂CH₂CH₃ | -CH(CH₃)₂ | -CH₂CH₂CH₃ | -H | -H | -H |
| 122 | -CH(CH₃)₂ | -CH(CH₃)₂ | -CH₂CH₂CH₃ | -H | -H | -H |
| 123 | -CH₂CH₂CH₂CH₃ | -CH(CH₃)₂ | -CH₂CH₂CH₃ | -H | -H | -H |
| 124 | -CH(CH₃)CH₂CH₃ | -CH(CH₃)₂ | -CH₂CH₂CH₃ | -H | -H | -H |
| 125 | -CH₂CH(CH₃)₂ | -CH(CH₃)₂ | -CH₂CH₂CH₃ | -H | -H | -H |
| 126 | -C(CH₃)₃ | -CH(CH₃)₂ | -CH₂CH₂CH₃ | -H | -H | -H |
| 127 | -H | -CH(CH₃)₂ | -CH(CH₃)₂ | -H | -H | -H |
| 128 | -CH₃ | -CH(CH₃)₂ | -CH(CH₃)₂ | -H | -H | -H |
| 129 | -CH₂CH₃ | -CH(CH₃)₂ | -CH(CH₃)₂ | -H | -H | -H |
| 130 | -CH₂CH₂CH₃ | -CH(CH₃)₂ | -CH(CH₃)₂ | -H | -H | -H |
| 131 | -CH(CH₃)₂ | -CH(CH₃)₂ | -CH(CH₃)₂ | -H | -H | -H |
| 132 | -CH₂CH₂CH₂CH₃ | -CH(CH₃)₂ | -CH(CH₃)₂ | -H | -H | -H |
| 133 | -CH(CH₃)CH₂CH₃ | -CH(CH₃)₂ | -CH(CH₃)₂ | -H | -H | -H |
| 134 | -CH₂CH(CH₃)₂ | -CH(CH₃)₂ | -CH(CH₃)₂ | -H | -H | -H |
| 135 | -C(CH₃)₃ | -CH(CH₃)₂ | -CH(CH₃)₂ | -H | -H | -H |
| | | | | | | |

| **Nr.** | **R10** | **R11** | **R12** | **R7** | **R8** | **R9** |
|---|---|---|---|---|---|---|
| 136 | -H | -H | -H | -H | -H | -H |
| 137 | -CH₃ | -H | -H | -H | -H | -H |
| 138 | -CH₂CH₃ | -H | -H | -H | -H | -H |
| 139 | -CH₂CH₂CH₃ | -H | -H | -H | -H | -H |
| 140 | -CH(CH₃)₂ | -H | -H | -H | -H | -H |
| 141 | -CH₂CH₂CH₂CH₃ | -H | -H | -H | -H | -H |
| 142 | -CH(CH₃)CH₂CH₃ | -H | -H | -H | -H | -H |
| 143 | -CH₂CH(CH₃)₂ | -H | -H | -H | -H | -H |
| 144 | -C(CH₃)₃ | -H | -H | -H | -H | -H |
| 145 | -H | -CH₃ | -H | -H | -H | -H |
| 146 | -CH₃ | -CH₃ | -H | -H | -H | -H |
| 147 | -CH₂CH₃ | -CH₃ | -H | -H | -H | -H |
| 148 | -CH₂CH₂CH₃ | -CH₃ | -H | -H | -H | -H |
| 149 | -CH(CH₃)₂ | -CH₃ | -H | -H | -H | -H |
| 150 | -CH₂CH₂CH₂CH₃ | -CH₃ | -H | -H | -H | -H |
| 151 | -CH(CH₃)CH₂CH₃ | -CH₃ | -H | -H | -H | -H |
| 152 | -CH₂CH(CH₃)₂ | -CH₃ | -H | -H | -H | -H |
| 153 | -C(CH₃)₃ | -CH₃ | -H | -H | -H | -H |
| 154 | -H | -CH₃ | -CH₃ | -H | -H | -H |
| 155 | -CH₃ | -CH₃ | -CH₃ | -H | -H | -H |
| 156 | -CH₂CH₃ | -CH₃ | -CH₃ | -H | -H | -H |
| 157 | -CH₂CH₂CH₃ | -CH₃ | -CH₃ | -H | -H | -H |
| 158 | -CH(CH₃)₂ | -CH₃ | -CH₃ | -H | -H | -H |
| 159 | -CH₂CH₂CH₂CH₃ | -CH₃ | -CH₃ | -H | -H | -H |
| 160 | -CH(CH₃)CH₂CH₃ | -CH₃ | -CH₃ | -H | -H | -H |
| 161 | -CH₂CH(CH₃)₂ | -CH₃ | -CH₃ | -H | -H | -H |
| 162 | -C(CH₃)₃ | -CH₃ | -CH₃ | -H | -H | -H |
| 163 | -H | -CH₂CH₃ | -H | -H | -H | -H |
| 164 | -CH₃ | -CH₂CH₃ | -H | -H | -H | -H |
| 165 | -CH₂CH₃ | -CH₂CH₃ | -H | -H | -H | -H |
| 166 | -CH₂CH₂CH₃ | -CH₂CH₃ | -H | -H | -H | -H |
| 167 | -CH(CH₃)₂ | -CH₂CH₃ | -H | -H | -H | -H |
| 168 | -CH₂CH₂CH₂CH₃ | -CH₂CH₃ | -H | -H | -H | -H |
| 169 | -CH(CH₃)CH₂CH₃ | -CH₂CH₃ | -H | -H | -H | -H |
| 170 | -CH₂CH(CH₃)₂ | -CH₂CH₃ | -H | -H | -H | -H |
| 171 | -C(CH₃)₃ | -CH₂CH₃ | -H | -H | -H | -H |
| 172 | -H | -CH₂CH₃ | -CH₃ | -H | -H | -H |
| 173 | -CH₃ | -CH₂CH₃ | -CH₃ | -H | -H | -H |
| 174 | -CH₂CH₃ | -CH₂CH₃ | -CH₃ | -H | -H | -H |
| 175 | -CH₂CH₂CH₃ | -CH₂CH₃ | -CH₃ | -H | -H | -H |
| 176 | -CH(CH₃)₂ | -CH₂CH₃ | -CH₃ | -H | -H | -H |
| 177 | -CH₂CH₂CH₂CH₃ | -CH₂CH₃ | -CH₃ | -H | -H | -H |
| 178 | -CH(CH₃)CH₂CH₃ | -CH₂CH₃ | -CH₃ | -H | -H | -H |
| 179 | -CH₂CH(CH₃)₂ | -CH₂CH₃ | -CH₃ | -H | -H | -H |
| 180 | -C(CH₃)₃ | -CH₂CH₃ | -CH₃ | -H | -H | -H |
| 181 | -H | -CH₂CH₃ | -CH₂CH₃ | -H | -H | -H |
| 182 | -CH₃ | -CH₂CH₃ | -CH₂CH₃ | -H | -H | -H |
| 183 | -CH₂CH₃ | -CH₂CH₃ | -CH₂CH₃ | -H | -H | -H |
| 184 | -CH₂CH₂CH₃ | -CH₂CH₃ | -CH₂CH₃ | -H | -H | -H |
| 185 | -CH(CH₃)₂ | -CH₂CH₃ | -CH₂CH₃ | -H | -H | -H |
| 186 | -CH₂CH₂CH₂CH₃ | -CH₂CH₃ | -CH₂CH₃ | -H | -H | -H |
| 187 | -CH(CH₃)CH₂CH₃ | -CH₂CH₃ | -CH₂CH₃ | -H | -H | -H |
| 188 | -CH₂CH(CH₃)₂ | -CH₂CH₃ | -CH₂CH₃ | -H | -H | -H |
| 189 | -C(CH₃)₃ | -CH₂CH₃ | -CH₂CH₃ | -H | -H | -H |
| 190 | -H | -CH₂CH₂CH₃ | -H | -H | -H | -H |
| 191 | -CH₃ | -CH₂CH₂CH₃ | -H | -H | -H | -H |
| 192 | -CH₂CH₃ | -CH₂CH₂CH₃ | -H | -H | -H | -H |
| 193 | -CH₂CH₂CH₃ | -CH₂CH₂CH₃ | -H | -H | -H | -H |
| 194 | -CH(CH₃)₂ | -CH₂CH₂CH₃ | -H | -H | -H | -H |
| 195 | -CH₂CH₂CH₂CH₃ | -CH₂CH₂CH₃ | -H | -H | -H | -H |
| 196 | -CH(CH₃)CH₂CH₃ | -CH₂CH₂CH₃ | -H | -H | -H | -H |
| 197 | -CH₂CH(CH₃)₂ | -CH₂CH₂CH₃ | -H | -H | -H | -H |
| 198 | -C(CH₃)₃ | -CH₂CH₂CH₃ | -H | -H | -H | -H |
| 199 | -H | -CH₂CH₂CH₃ | -CH₃ | -H | -H | -H |
| 200 | -CH₃ | -CH₂CH₂CH₃ | -CH₃ | -H | -H | -H |
| 201 | -CH₂CH₃ | -CH₂CH₂CH₃ | -CH₃ | -H | -H | -H |
| 202 | -CH₂CH₂CH₃ | -CH₂CH₂CH₃ | -CH₃ | -H | -H | -H |
| 203 | -CH(CH₃)₂ | -CH₂CH₂CH₃ | -CH₃ | -H | -H | -H |
| 204 | -CH₂CH₂CH₂CH₃ | -CH₂CH₂CH₃ | -CH₃ | -H | -H | -H |
| 205 | -CH(CH₃)CH₂CH₃ | -CH₂CH₂CH₃ | -CH₃ | -H | -H | -H |
| 206 | -CH₂CH(CH₃)₂ | -CH₂CH₂CH₃ | -CH₃ | -H | -H | -H |
| 207 | -C(CH₃)₃ | -CH₂CH₂CH₃ | -CH₃ | -H | -H | -H |
| 208 | -H | -CH₂CH₂CH₃ | -CH₂CH₃ | -H | -H | -H |
| 209 | -CH₃ | -CH₂CH₂CH₃ | -CH₂CH₃ | -H | -H | -H |
| 210 | -CH₂CH₃ | -CH₂CH₂CH₃ | -CH₂CH₃ | -H | -H | -H |
| 211 | -CH₂CH₂CH₃ | -CH₂CH₂CH₃ | -CH₂CH₃ | -H | -H | -H |
| 212 | -CH(CH₃)₂ | -CH₂CH₂CH₃ | -CH₂CH₃ | -H | -H | -H |
| 213 | -CH₂CH₂CH₂CH₃ | -CH₂CH₂CH₃ | -CH₂CH₃ | -H | -H | -H |
| 214 | -CH(CH₃)CH₂CH₃ | -CH₂CH₂CH₃ | -CH₂CH₃ | -H | -H | -H |
| 215 | -CH₂CH(CH₃)₂ | -CH₂CH₂CH₃ | -CH₂CH₃ | -H | -H | -H |
| 216 | -C(CH₃)₃ | -CH₂CH₂CH₃ | -CH₂CH₃ | -H | -H | -H |
| 217 | -H | -CH₂CH₂CH₃ | -CH₂CH₂CH₃ | -H | -H | -H |
| 218 | -CH₃ | -CH₂CH₂CH₃ | -CH₂CH₂CH₃ | -H | -H | -H |
| 219 | -CH₂CH₃ | -CH₂CH₂CH₃ | -CH₂CH₂CH₃ | -H | -H | -H |
| 220 | -CH₂CH₂CH₃ | -CH₂CH₂CH₃ | -CH₂CH₂CH₃ | -H | -H | -H |
| 221 | -CH(CH₃)₂ | -CH₂CH₂CH₃ | -CH₂CH₂CH₃ | -H | -H | -H |
| 222 | -CH₂CH₂CH₂CH₃ | -CH₂CH₂CH₃ | -CH₂CH₂CH₃ | -H | -H | -H |
| 223 | -CH(CH₃)CH₂CH₃ | -CH₂CH₂CH₃ | -CH₂CH₂CH₃ | -H | -H | -H |
| 224 | -CH₂CH(CH₃)₂ | -CH₂CH₂CH₃ | -CH₂CH₂CH₃ | -H | -H | -H |
| 225 | -C(CH₃)₃ | -CH₂CH₂CH₃ | -CH₂CH₂CH₃ | -H | -H | -H |
| 226 | -H | -CH(CH₃)₂ | -H | -H | -H | -H |
| 227 | -CH₃ | -CH(CH₃)₂ | -H | -H | -H | -H |
| 228 | -CH₂CH₃ | -CH(CH₃)₂ | -H | -H | -H | -H |
| 229 | -CH₂CH₂CH₃ | -CH(CH₃)₂ | -H | -H | -H | -H |
| 230 | -CH(CH₃)₂ | -CH(CH₃)₂ | -H | -H | -H | -H |
| 231 | -CH₂CH₂CH₂CH₃ | -CH(CH₃)₂ | -H | -H | -H | -H |
| 232 | -CH(CH₃)CH₂CH₃ | -CH(CH₃)₂ | -H | -H | -H | -H |
| 233 | -CH₂CH(CH₃)₂ | -CH(CH₃)₂ | -H | -H | -H | -H |
| 234 | -C(CH₃)₃ | -CH(CH₃)₂ | -H | -H | -H | -H |
| 235 | -H | -CH(CH₃)₂ | -CH₃ | -H | -H | -H |
| 236 | -CH₃ | -CH(CH₃)₂ | -CH₃ | -H | -H | -H |
| 237 | -CH₂CH₃ | -CH(CH₃)₂ | -CH₃ | -H | -H | -H |
| 238 | -CH₂CH₂CH₃ | -CH(CH₃)₂ | -CH₃ | -H | -H | -H |
| 239 | -CH(CH₃)₂ | -CH(CH₃)₂ | -CH₃ | -H | -H | -H |
| 240 | -CH₂CH₂CH₂CH₃ | -CH(CH₃)₂ | -CH₃ | -H | -H | -H |
| 241 | -CH(CH₃)CH₂CH₃ | -CH(CH₃)₂ | -CH₃ | -H | -H | -H |
| 242 | -CH₂CH(CH₃)₂ | -CH(CH₃)₂ | -CH₃ | -H | -H | -H |
| 243 | -C(CH₃)₃ | -CH(CH₃)₂ | -CH₃ | -H | -H | -H |
| 244 | -H | -CH(CH₃)₂ | -CH₂CH₃ | -H | -H | -H |
| 245 | -CH₃ | -CH(CH₃)₂ | -CH₂CH₃ | -H | -H | -H |
| 246 | -CH₂CH₃ | -CH(CH₃)₂ | -CH₂CH₃ | -H | -H | -H |
| 247 | -CH₂CH₂CH₃ | -CH(CH₃)₂ | -CH₂CH₃ | -H | -H | -H |
| 248 | -CH(CH₃)₂ | -CH(CH₃)₂ | -CH₂CH₃ | -H | -H | -H |
| 249 | -CH₂CH₂CH₂CH₃ | -CH(CH₃)₂ | -CH₂CH₃ | -H | -H | -H |
| 250 | -CH(CH₃)CH₂CH₃ | -CH(CH₃)₂ | -CH₂CH₃ | -H | -H | -H |
| 251 | -CH₂CH(CH₃)₂ | -CH(CH₃)₂ | -CH₂CH₃ | -H | -H | -H |
| 252 | -C(CH₃)₃ | -CH(CH₃)₂ | -CH₂CH₃ | -H | -H | -H |
| 253 | -H | -CH(CH₃)₂ | -CH₂CH₂CH₃ | -H | -H | -H |
| 254 | -CH₃ | -CH(CH₃)₂ | -CH₂CH₂CH₃ | -H | -H | -H |
| 255 | -CH₂CH₃ | -CH(CH₃)₂ | -CH₂CH₂CH₃ | -H | -H | -H |
| 256 | -CH₂CH₂CH₃ | -CH(CH₃)₂ | -CH₂CH₂CH₃ | -H | -H | -H |
| 257 | -CH(CH₃)₂ | -CH(CH₃)₂ | -CH₂CH₂CH₃ | -H | -H | -H |
| 258 | -CH₂CH₂CH₂CH₃ | -CH(CH₃)₂ | -CH₂CH₂CH₃ | -H | -H | -H |
| 259 | -CH(CH₃)CH₂CH₃ | -CH(CH₃)₂ | -CH₂CH₂CH₃ | -H | -H | -H |
| 260 | -CH₂CH(CH₃)₂ | -CH(CH₃)₂ | -CH₂CH₂CH₃ | -H | -H | -H |
| 261 | -C(CH₃)₃ | -CH(CH₃)₂ | -CH₂CH₂CH₃ | -H | -H | -H |
| 262 | -H | -CH(CH₃)₂ | -CH(CH₃)₂ | -H | -H | -H |
| 263 | -CH₃ | -CH(CH₃)₂ | -CH(CH₃)₂ | -H | -H | -H |
| 264 | -CH₂CH₃ | -CH(CH₃)₂ | -CH(CH₃)₂ | -H | -H | -H |
| 265 | -CH₂CH₂CH₃ | -CH(CH₃)₂ | -CH(CH₃)₂ | -H | -H | -H |
| 266 | -CH(CH₃)₂ | -CH(CH₃)₂ | -CH(CH₃)₂ | -H | -H | -H |
| 267 | -CH₂CH₂CH₂CH₃ | -CH(CH₃)₂ | -CH(CH₃)₂ | -H | -H | -H |
| 268 | -CH(CH₃)CH₂CH₃ | -CH(CH₃)₂ | -CH(CH₃)₂ | -H | -H | -H |
| 269 | -CH₂CH(CH₃)₂ | -CH(CH₃)₂ | -CH(CH₃)₂ | -H | -H | -H |
| 270 | -C(CH₃)₃ | -CH(CH₃)₂ | -CH(CH₃)₂ | -H | -H | -H |
| | | | | | | |

| **Nr.** | **R7** | **R8** | **R9** | **R10** | **R11** | **R12** |
|---|---|---|---|---|---|---|
| 271 | -H | -H | -H | -H | -H | -H |
| 272 | -CH₃ | -H | -H | -CH₃ | -H | -H |
| 273 | -CH₂CH₃ | -H | -H | -CH₂CH₃ | -H | -H |
| 274 | -CH₂CH₂CH₃ | -H | -H | -CH₂CH₂CH₃ | -H | -H |
| 275 | -CH(CH₃)₂ | -H | -H | -CH(CH₃)₂ | -H | -H |
| 276 | -CH₂CH₂CH₂CH₃ | -H | -H | -CH₂CH₂CH₂CH₃ | -H | -H |
| 277 | -CH(CH₃)CH₂CH₃ | -H | -H | -CH(CH₃)CH₂CH₃ | -H | -H |
| 278 | -CH₂CH(CH₃)₂ | -H | -H | -CH₂CH(CH₃)₂ | -H | -H |
| 279 | -C(CH₃)₃ | -H | -H | -C(CH₃)₃ | -H | -H |
| 280 | -H | -CH₃ | -H | -H | -CH₃ | -H |
| 281 | -CH₃ | -CH₃ | -H | -CH₃ | -CH₃ | -H |
| 282 | -CH₂CH₃ | -CH₃ | -H | -CH₂CH₃ | -CH₃ | -H |
| 283 | -CH₂CH₂CH₃ | -CH₃ | -H | -CH₂CH₂CH₃ | -CH₃ | -H |
| 284 | -CH(CH₃)₂ | -CH₃ | -H | -CH(CH₃)₂ | -CH₃ | -H |
| 285 | -CH₂CH₂CH₂CH₃ | -CH₃ | -H | -CH₂CH₂CH₂CH₃ | -CH₃ | -H |
| 286 | -CH(CH₃)CH₂CH₃ | -CH₃ | -H | -CH(CH₃)CH₂CH₃ | -CH₃ | -H |
| 287 | -CH₂CH(CH₃)₂ | -CH₃ | -H | -CH₂CH(CH₃)₂ | -CH₃ | -H |
| 288 | -C(CH₃)₃ | -CH₃ | -H | -C(CH₃)₃ | -CH₃ | -H |
| 289 | -H | -CH₃ | -CH₃ | -H | -CH₃ | -CH₃ |
| 290 | -CH₃ | -CH₃ | -CH₃ | -CH₃ | -CH₃ | -CH₃ |
| 291 | -CH₂CH₃ | -CH₃ | -CH₃ | -CH₂CH₃ | -CH₃ | -CH₃ |
| 292 | -CH₂CH₂CH₃ | -CH₃ | -CH₃ | -CH₂CH₂CH₃ | -CH₃ | -CH₃ |
| 293 | -CH(CH₃)₂ | -CH₃ | -CH₃ | -CH(CH₃)₂ | -CH₃ | -CH₃ |
| 294 | -CH₂CH₂CH₂CH₃ | -CH₃ | -CH₃ | -CH₂CH₂CH₂CH₃ | -CH₃ | -CH₃ |
| 295 | -CH(CH₃)CH₂CH₃ | -CH₃ | -CH₃ | -CH(CH₃)CH₂CH₃ | -CH₃ | -CH₃ |
| 296 | -CH₂CH(CH₃)₂ | -CH₃ | -CH₃ | -CH₂CH(CH₃)₂ | -CH₃ | -CH₃ |
| 297 | -C(CH₃)₃ | -CH₃ | -CH₃ | -C(CH₃)₃ | -CH₃ | -CH₃ |
| 298 | -H | -CH₂CH₃ | -H | -H | -CH₂CH₃ | -H |
| 299 | -CH₃ | -CH₂CH₃ | -H | -CH₃ | -CH₂CH₃ | -H |
| 300 | -CH₂CH₃ | -CH₂CH₃ | -H | -CH₂CH₃ | -CH₂CH₃ | -H |
| 301 | -CH₂CH₂CH₃ | -CH₂CH₃ | -H | -CH₂CH₂CH₃ | -CH₂CH₃ | -H |
| 302 | -CH(CH₃)₂ | -CH₂CH₃ | -H | -CH(CH₃)₂ | -CH₂CH₃ | -H |
| 303 | -CH₂CH₂CH₂CH₃ | -CH₂CH₃ | -H | -CH₂CH₂CH₂CH₃ | -CH₂CH₃ | -H |
| 304 | -CH(CH₃)CH₂CH₃ | -CH₂CH₃ | -H | -CH(CH₃)CH₂CH₃ | -CH₂CH₃ | -H |
| 305 | -CH₂CH(CH₃)₂ | -CH₂CH₃ | -H | -CH₂CH(CH₃)₂ | -CH₂CH₃ | -H |
| 306 | -C(CH₃)₃ | -CH₂CH₃ | -H | -C(CH₃)₃ | -CH₂CH₃ | -H |
| 307 | -H | -CH₂CH₃ | -CH₃ | -H | -CH₂CH₃ | -CH₃ |
| 308 | -CH₃ | -CH₂CH₃ | -CH₃ | -CH₃ | -CH₂CH₃ | -CH₃ |
| 309 | -CH₂CH₃ | -CH₂CH₃ | -CH₃ | -CH₂CH₃ | -CH₂CH₃ | -CH₃ |
| 310 | -CH₂CH₂CH₃ | -CH₂CH₃ | -CH₃ | -CH₂CH₂CH₃ | -CH₂CH₃ | -CH₃ |
| 311 | -CH(CH₃)₂ | -CH₂CH₃ | -CH₃ | -CH(CH₃)₂ | -CH₂CH₃ | -CH₃ |
| 312 | -CH₂CH₂CH₂CH₃ | -CH₂CH₃ | -CH₃ | -CH₂CH₂CH₂CH₃ | -CH₂CH₃ | -CH₃ |
| 313 | -CH(CH₃)CH₂CH₃ | -CH₂CH₃ | -CH₃ | -CH(CH₃)CH₂CH₃ | -CH₂CH₃ | -CH₃ |
| 314 | -CH₂CH(CH₃)₂ | -CH₂CH₃ | -CH₃ | -CH₂CH(CH₃)₂ | -CH₂CH₃ | -CH₃ |
| 315 | -C(CH₃)₃ | -CH₂CH₃ | -CH₃ | -C(CH₃)₃ | -CH₂CH₃ | -CH₃ |
| 316 | -H | -CH₂CH₃ | -CH₂CH₃ | -H | -CH₂CH₃ | -CH₂CH₃ |
| 317 | -CH₃ | -CH₂CH₃ | -CH₂CH₃ | -CH₃ | -CH₂CH₃ | -CH₂CH₃ |
| 318 | -CH₂CH₃ | -CH₂CH₃ | -CH₂CH₃ | -CH₂CH₃ | -CH₂CH₃ | -CH₂CH₃ |
| 319 | -CH₂CH₂CH₃ | -CH₂CH₃ | -CH₂CH₃ | -CH₂CH₂CH₃ | -CH₂CH₃ | -CH₂CH₃ |
| 320 | -CH(CH₃)₂ | -CH₂CH₃ | -CH₂CH₃ | -CH(CH₃)₂ | -CH₂CH₃ | -CH₂CH₃ |
| 321 | -CH₂CH₂CH₂CH₃ | -CH₂CH₃ | -CH₂CH₃ | -CH₂CH₂CH₂CH₃ | -CH₂CH₃ | -CH₂CH₃ |
| 322 | -CH(CH₃)CH₂CH₃ | -CH₂CH₃ | -CH₂CH₃ | -CH(CH₃)CH₂CH₃ | -CH₂CH₃ | -CH₂CH₃ |
| 323 | -CH₂CH(CH₃)₂ | -CH₂CH₃ | -CH₂CH₃ | -CH₂CH(CH₃)₂ | -CH₂CH₃ | -CH₂CH₃ |
| 324 | -C(CH₃)₃ | -CH₂CH₃ | -CH₂CH₃ | -C(CH₃)₃ | -CH₂CH₃ | -CH₂CH₃ |
| 325 | -H | -CH₂CH₂CH₃ | -H | -H | -CH₂CH₂CH₃ | -H |
| 326 | -CH₃ | -CH₂CH₂CH₃ | -H | -CH₃ | -CH₂CH₂CH₃ | -H |
| 327 | -CH₂CH₃ | -CH₂CH₂CH₃ | -H | -CH₂CH₃ | -CH₂CH₂CH₃ | -H |
| 328 | -CH₂CH₂CH₃ | -CH₂CH₂CH₃ | -H | -CH₂CH₂CH₃ | -CH₂CH₂CH₃ | -H |
| 329 | -CH(CH₃)₂ | -CH₂CH₂CH₃ | -H | -CH(CH₃)₂ | -CH₂CH₂CH₃ | -H |
| 330 | -CH₂CH₂CH₂CH₃ | -CH₂CH₂CH₃ | -H | -CH₂CH₂CH₂CH₃ | -CH₂CH₂CH₃ | -H |
| 331 | -CH(CH₃)CH₂CH₃ | -CH₂CH₂CH₃ | -H | -CH(CH₃)CH₂CH₃ | -CH₂CH₂CH₃ | -H |
| 332 | -CH₂CH(CH₃)₂ | -CH₂CH₂CH₃ | -H | -CH₂CH(CH₃)₂ | -CH₂CH₂CH₃ | -H |
| 333 | -C(CH₃)₃ | -CH₂CH₂CH₃ | -H | -C(CH₃)₃ | -CH₂CH₂CH₃ | -H |
| 334 | -H | -CH₂CH₂CH₃ | -CH₃ | -H | -CH₂CH₂CH₃ | -CH₃ |
| 335 | -CH₃ | -CH₂CH₂CH₃ | -CH₃ | -CH₃ | -CH₂CH₂CH₃ | -CH₃ |
| 336 | -CH₂CH₃ | -CH₂CH₂CH₃ | -CH₃ | -CH₂CH₃ | -CH₂CH₂CH₃ | -CH₃ |
| 337 | -CH₂CH₂CH₃ | -CH₂CH₂CH₃ | -CH₃ | -CH₂CH₂CH₃ | -CH₂CH₂CH₃ | -CH₃ |
| 338 | -CH(CH₃)₂ | -CH₂CH₂CH₃ | -CH₃ | -CH(CH₃)₂ | -CH₂CH₂CH₃ | -CH₃ |
| 339 | -CH₂CH₂CH₂CH₃ | -CH₂CH₂CH₃ | -CH₃ | -CH₂CH₂CH₂CH₃ | -CH₂CH₂CH₃ | -CH₃ |
| 340 | -CH(CH₃)CH₂CH₃ | -CH₂CH₂CH₃ | -CH₃ | -CH(CH₃)CH₂CH₃ | -CH₂CH₂CH₃ | -CH₃ |
| 341 | -CH₂CH(CH₃)₂ | -CH₂CH₂CH₃ | -CH₃ | -CH₂CH(CH₃)₂ | -CH₂CH₂CH₃ | -CH₃ |
| 342 | -C(CH₃)₃ | -CH₂CH₂CH₃ | -CH₃ | -C(CH₃)₃ | -CH₂CH₂CH₃ | -CH₃ |
| 343 | -H | -CH₂CH₂CH₃ | -CH₂CH₃ | -H | -CH₂CH₂CH₃ | -CH₂CH₃ |
| 344 | -CH₃ | -CH₂CH₂CH₃ | -CH₂CH₃ | -CH₃ | -CH₂CH₂CH₃ | -CH₂CH₃ |
| 345 | -CH₂CH₃ | -CH₂CH₂CH₃ | -CH₂CH₃ | -CH₂CH₃ | -CH₂CH₂CH₃ | -CH₂CH₃ |
| 346 | -CH₂CH₂CH₃ | -CH₂CH₂CH₃ | -CH₂CH₃ | -CH₂CH₂CH₃ | -CH₂CH₂CH₃ | -CH₂CH₃ |
| 347 | -CH(CH₃)₂ | -CH₂CH₂CH₃ | -CH₂CH₃ | -CH(CH₃)₂ | -CH₂CH₂CH₃ | -CH₂CH₃ |
| 348 | -CH₂CH₂CH₂CH₃ | -CH₂CH₂CH₃ | -CH₂CH₃ | -CH₂CH₂CH₂CH₃ | -CH₂CH₂CH₃ | -CH₂CH₃ |
| 349 | -CH(CH₃)CH₂CH₃ | -CH₂CH₂CH₃ | -CH₂CH₃ | -CH(CH₃)CH₂CH₃ | -CH₂CH₂CH₃ | -CH₂CH₃ |
| 350 | -CH₂CH(CH₃)₂ | -CH₂CH₂CH₃ | -CH₂CH₃ | -CH₂CH(CH₃)₂ | -CH₂CH₂CH₃ | -CH₂CH₃ |
| 351 | -C(CH₃)₃ | -CH₂CH₂CH₃ | -CH₂CH₃ | -C(CH₃)₃ | -CH₂CH₂CH₃ | -CH₂CH₃ |
| 352 | -H | -CH₂CH₂CH₃ | -CH₂CH₂CH₃ | -H | -CH₂CH₂CH₃ | -CH₂CH₂CH₃ |
| 353 | -CH₃ | -CH₂CH₂CH₃ | -CH₂CH₂CH₃ | -CH₃ | -CH₂CH₂CH₃ | -CH₂CH₂CH₃ |
| 354 | -CH₂CH₃ | -CH₂CH₂CH₃ | -CH₂CH₂CH₃ | -CH₂CH₃ | -CH₂CH₂CH₃ | -CH₂CH₂CH₃ |
| 355 | -CH₂CH₂CH₃ | -CH₂CH₂CH₃ | -CH₂CH₂CH₃ | -CH₂CH₂CH₃ | -CH₂CH₂CH₃ | -CH₂CH₂CH₃ |
| 356 | -CH(CH₃)₂ | -CH₂CH₂CH₃ | -CH₂CH₂CH₃ | -CH(CH₃)₂ | -CH₂CH₂CH₃ | -CH₂CH₂CH₃ |
| 357 | -CH₂CH₂CH₂CH₃ | -CH₂CH₂CH₃ | -CH₂CH₂CH₃ | -CH₂CH₂CH₂CH₃ | -CH₂CH₂CH₃ | -CH₂CH₂CH₃ |
| 358 | -CH(CH₃)CH₂CH₃ | -CH₂CH₂CH₃ | -CH₂CH₂CH₃ | -CH(CH₃)CH₂CH₃ | -CH₂CH₂CH₃ | -CH₂CH₂CH₃ |
| 359 | -CH₂CH(CH₃)₂ | -CH₂CH₂CH₃ | -CH₂CH₂CH₃ | -CH₂CH(CH₃)₂ | -CH₂CH₂CH₃ | -CH₂CH₂CH₃ |
| 360 | -C(CH₃)₃ | -CH₂CH₂CH₃ | -CH₂CH₂CH₃ | -C(CH₃)₃ | -CH₂CH₂CH₃ | -CH₂CH₂CH₃ |
| 361 | -H | -CH(CH₃)₂ | -H | -H | -CH(CH₃)₂ | -H |
| 362 | -CH₃ | -CH(CH₃)₂ | -H | -CH₃ | -CH(CH₃)₂ | -H |
| 363 | -CH₂CH₃ | -CH(CH₃)₂ | -H | -CH₂CH₃ | -CH(CH₃)₂ | -H |
| 364 | -CH₂CH₂CH₃ | -CH(CH₃)₂ | -H | -CH₂CH₂CH₃ | -CH(CH₃)₂ | -H |
| 365 | -CH(CH₃)₂ | -CH(CH₃)₂ | -H | -CH(CH₃)₂ | -CH(CH₃)₂ | -H |
| 366 | -CH₂CH₂CH₂CH₃ | -CH(CH₃)₂ | -H | -CH₂CH₂CH₂CH₃ | -CH(CH₃)₂ | -H |
| 367 | -CH(CH₃)CH₂CH₃ | -CH(CH₃)₂ | -H | -CH(CH₃)CH₂CH₃ | -CH(CH₃)₂ | -H |
| 368 | -CH₂CH(CH₃)₂ | -CH(CH₃)₂ | -H | -CH₂CH(CH₃)₂ | -CH(CH₃)₂ | -H |
| 369 | -C(CH₃)₃ | -CH(CH₃)₂ | -H | -C(CH₃)₃ | -CH(CH₃)₂ | -H |
| 370 | -H | -CH(CH₃)₂ | -CH₃ | -H | -CH(CH₃)₂ | -CH₃ |
| 371 | -CH₃ | -CH(CH₃)₂ | -CH₃ | -CH₃ | -CH(CH₃)₂ | -CH₃ |
| 372 | -CH₂CH₃ | -CH(CH₃)₂ | -CH₃ | -CH₂CH₃ | -CH(CH₃)₂ | -CH₃ |
| 373 | -CH₂CH₂CH₃ | -CH(CH₃)₂ | -CH₃ | -CH₂CH₂CH₃ | -CH(CH₃)₂ | -CH₃ |
| 374 | -CH(CH₃)₂ | -CH(CH₃)₂ | -CH₃ | -CH(CH₃)₂ | -CH(CH₃)₂ | -CH₃ |
| 375 | -CH₂CH₂CH₂CH₃ | -CH(CH₃)₂ | -CH₃ | -CH₂CH₂CH₂CH₃ | -CH(CH₃)₂ | -CH₃ |
| 376 | -CH(CH₃)CH₂CH₃ | -CH(CH₃)₂ | -CH₃ | -CH(CH₃)CH₂CH₃ | -CH(CH₃)₂ | -CH₃ |
| 377 | -CH₂CH(CH₃)₂ | -CH(CH₃)₂ | -CH₃ | -CH₂CH(CH₃)₂ | -CH(CH₃)₂ | -CH₃ |
| 378 | -C(CH₃)₃ | -CH(CH₃)₂ | -CH₃ | -C(CH₃)₃ | -CH(CH₃)₂ | -CH₃ |
| 379 | -H | -CH(CH₃)₂ | -CH₂CH₃ | -H | -CH(CH₃)₂ | -CH₂CH₃ |
| 380 | -CH₃ | -CH(CH₃)₂ | -CH₂CH₃ | -CH₃ | -CH(CH₃)₂ | -CH₂CH₃ |
| 381 | -CH₂CH₃ | -CH(CH₃)₂ | -CH₂CH₃ | -CH₂CH₃ | -CH(CH₃)₂ | -CH₂CH₃ |
| 382 | -CH₂CH₂CH₃ | -CH(CH₃)₂ | -CH₂CH₃ | -CH₂CH₂CH₃ | -CH(CH₃)₂ | -CH₂CH₃ |
| 383 | -CH(CH₃)₂ | -CH(CH₃)₂ | -CH₂CH₃ | -CH(CH₃)₂ | -CH(CH₃)₂ | -CH₂CH₃ |
| 384 | -CH₂CH₂CH₂CH₃ | -CH(CH₃)₂ | -CH₂CH₃ | -CH₂CH₂CH₂CH₃ | -CH(CH₃)₂ | -CH₂CH₃ |
| 385 | -CH(CH₃)CH₂CH₃ | -CH(CH₃)₂ | -CH₂CH₃ | -CH(CH₃)CH₂CH₃ | -CH(CH₃)₂ | -CH₂CH₃ |
| 386 | -CH₂CH(CH₃)₂ | -CH(CH₃)₂ | -CH₂CH₃ | -CH₂CH(CH₃)₂ | -CH(CH₃)₂ | -CH₂CH₃ |
| 387 | -C(CH₃)₃ | -CH(CH₃)₂ | -CH₂CH₃ | -C(CH₃)₃ | -CH(CH₃)₂ | -CH₂CH₃ |
| 388 | -H | -CH(CH₃)₂ | -CH₂CH₂CH₃ | -H | -CH(CH₃)₂ | -CH₂CH₂CH₃ |
| 389 | -CH₃ | -CH(CH₃)₂ | -CH₂CH₂CH₃ | -CH₃ | -CH(CH₃)₂ | -CH₂CH₂CH₃ |
| 390 | -CH₂CH₃ | -CH(CH₃)₂ | -CH₂CH₂CH₃ | -CH₂CH₃ | -CH(CH₃)₂ | -CH₂CH₂CH₃ |
| 391 | -CH₂CH₂CH₃ | -CH(CH₃)₂ | -CH₂CH₂CH₃ | -CH₂CH₂CH₃ | -CH(CH₃)₂ | -CH₂CH₂CH₃ |
| 392 | -CH(CH₃)₂ | -CH(CH₃)₂ | -CH₂CH₂CH₃ | -CH(CH₃)₂ | -CH(CH₃)₂ | -CH₂CH₂CH₃ |
| 393 | -CH₂CH₂CH₂CH₃ | -CH(CH₃)₂ | -CH₂CH₂CH₃ | -CH₂CH₂CH₂CH₃ | -CH(CH₃)₂ | -CH₂CH₂CH₃ |
| 394 | -CH(CH₃)CH₂CH₃ | -CH(CH₃)₂ | -CH₂CH₂CH₃ | -CH(CH₃)CH₂CH₃ | -CH(CH₃)₂ | -CH₂CH₂CH₃ |
| 395 | -CH₂CH(CH₃)₂ | -CH(CH₃)₂ | -CH₂CH₂CH₃ | -CH₂CH(CH₃)₂ | -CH(CH₃)₂ | -CH₂CH₂CH₃ |
| 396 | -C(CH₃)₃ | -CH(CH₃)₂ | -CH₂CH₂CH₃ | -C(CH₃)₃ | -CH(CH₃)₂ | -CH₂CH₂CH₃ |
| 397 | -H | -CH(CH₃)₂ | -CH(CH₃)₂ | -H | -CH(CH₃)₂ | -CH(CH₃)₂ |
| 398 | -CH₃ | -CH(CH₃)₂ | -CH(CH₃)₂ | -CH₃ | -CH(CH₃)₂ | -CH(CH₃)₂ |
| 399 | -CH₂CH₃ | -CH(CH₃)₂ | -CH(CH₃)₂ | -CH₂CH₃ | -CH(CH₃)₂ | -CH(CH₃)₂ |
| 400 | -CH₂CH₂CH₃ | -CH(CH₃)₂ | -CH(CH₃)₂ | -CH₂CH₂CH₃ | -CH(CH₃)₂ | -CH(CH₃)₂ |
| 401 | -CH(CH₃)₂ | -CH(CH₃)₂ | -CH(CH₃)₂ | -CH(CH₃)₂ | -CH(CH₃)₂ | -CH(CH₃)₂ |
| 402 | -CH₂CH₂CH₂CH₃ | -CH(CH₃)₂ | -CH(CH₃)₂ | -CH₂CH₂CH₂CH₃ | -CH(CH₃)₂ | -CH(CH₃)₂ |
| 403 | CH(CH₃)CH₂CH₃ | -CH(CH₃)₂ | -CH(CH₃)₂ | -CH(CH₃)CH₂CH₃ | -CH(CH₃)₂ | -CH(CH₃)₂ |
| 404 | -CH₂CH(CH₃)₂ | -CH(CH₃)₂ | -CH(CH₃)₂ | -CH₂CH(CH₃)₂ | -CH(CH₃)₂ | -CH(CH₃)₂ |
| 405 | -C(CH₃)₃ | -CH(CH₃)₂ | -CH(CH₃)₂ | -C(CH₃)₃ | -CH(CH₃)₂ | -CH(CH₃)₂ |

Zusammenfassend sind erfindungsgemäße Mund- und Zahnpflege- und -reinigungsmittel bevorzugt, die mindestens einen Scharfstoff aus den Gruppen der
- alkylsubstituierten Dioxane der Formel 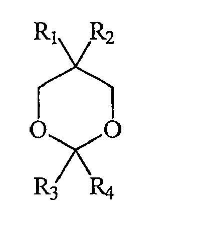 in der R1 und R2 unabhängig voneinander ausgewählt sind aus -H, -CH₃, - CH₂CH₃ und R3 und R4 unabhängig voneinander ausgewählt sind aus -H, -CH₃, - CH₂CH₃, -CH₂CH₂CH₃, -CH(CH₃)₂
   und/oder
- Phenylestern der Formel 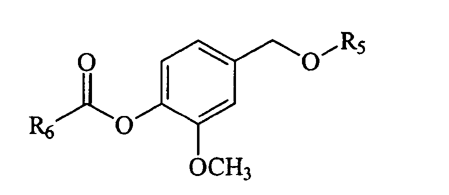 in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht
   und/oder
- Carvonacetalen der Formel 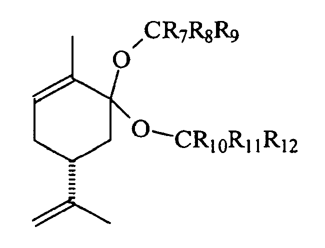 in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, - C(CH₃)₃ oder R9 und R10 zusammen eine chemiche Bindung oder eine Gruppe - (CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, - CH(CH₃)₂, -CH₂ CH₂CH₂CH₃, -CH₂CH(CH₃)₂, -CH(CH₃)CH₂CH₃, C(CH₃)₃
enthalten.

Die erfindungsgemäßen Mund- und Zahnpflege- und -reinigungsmittel können zusätzlich zu den vorstehend genannten wesentlichen Inhaltsstoffen weitere Inhaltsstoffe von Mundreinigungsmitteln, Mundpflegemitteln, Zahnreinigungsmitteln und/oder Zahnpflegemitteln enthalten. Die bevorzugten weiteren Inhaltsstoffe werden nachstehend beschrieben.

Die erfindungsgemäßen Mittel, insbesondere die Zahnpasten oder flüssigen Zahncremes enthalten vorzugsweise ein oder mehrere Poliermittel, üblicherweise in einer Menge von 5 bis 50 Gew.-%.

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Polier-mittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m2/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m2/g (nach BET) aus und eignen sich besonders gut für flüssige Zahncremes. Diese sollten eine Viskosität (25°C, Scherrate D = 10 s-1) von 10 - 100 Pa.s aufweisen.

Zahnpasten, die eine deutlich höhere Viskosität von mehr als 100 Pa.s (25° C, D = 10 s-1) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungs-kieselsäuren mit einer BET-Oberfläche von 150 -250 m2/g zu, z.B. die Handelsprodukte Sipernat 22 LS oder Sipernat 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 20 Gew.%, jeweils bezogen auf das gesamte Mittel, enthalten.

Die erfindungsgemäßen Mund- und Zahnpflege- und reinigungsmittel, insbesondere die Zahnpasten, können z. B. auch antimikrobielle Stoffe als Konservierungsmittel oder als Antiplaque-Wirkstoffe enthalten. Derartige Stoffe können z. B. ausgewählt sein aus p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol usw.. Erfindungsgemäß bevorzugte Mund- und Zahnpflege- und -reinigungsmittel sind dadurch gekennzeichnet, daß sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthalten.

Erfindungsgemäße Mund- und Zahnpflege- und -reinigungsmittel enthalten zusätzlich Fluoridverbindung(en) als Antikaries-Wirkstoffe, insbesondere Natriumfluorid, Kaliumfluorid, Natriummonofluorphosphat, Zinkfluorid, Zinnfluorid und Natriumfluorosilikat, in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel.

Bevorzugt sollte eine Menge von 0,01-0,2 Gew,-% Fluor in Form der genannten Verbindungen enthalten sein.

Die Mund- und Zahnpflege- und reinigungsmittel, insbesondere die Zahnpasten, können z.B. auch Substanzen enthalten, die gegen Zahnstein wirksam sind. Derartige Substanzen können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1-5 Gew.-% enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-β-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.
Bevorzugte Zahnpflegemittel gemäß der vorliegenden Erfindung enthalten neben Poliermitteln, Fluorverbindungen, Feuchthaltemitteln und Bindemitteln bevorzugt

| | |
|---|---|
| 0,01 - 1 Gew.-% | eines halogenierten Diphenylethers |
| 0,05 - 5 Gew.-% | Panthenol oder ein Salz der Pantothensäure und |
| 0,01 - 0,1 Gew.-% | eines Retinol-Esters, bevorzugt Retinol-Palmitat. |

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nicht- ionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die sogenannten bioaktiven Gläser.

Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.

Erfindungsgemäß bsonders bevorzugte Mund- und Zahnpflege- und -reinigungsmittel sind dadurch gekennzeichnet, daß sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Die erfindungsgemäßen Mund- und Zahnpflege- und -reinigungsmittel dieser Ausführungsform enthalten bioaktives Glas oder Glaspulver oder Glaskeramikpulver oder Kompositmaterialien, welche ein solches bioaktives Glas umfassen. Unter Glaspulvern werden im Rahmen der vorliegenden Anmeldung auch Granulate und Glaskügelchen verstanden.

Aufgrund der Anforderungen an die toxikologische Unbedenklichkeit des Glases sowie deren Eignung zum Verzehr soll das Glaspulver besonders rein sein. Die Belastung durch Schwermetalle ist vorzugsweise gering. So beträgt die Maximalkonzentration im Bereich der kosmetischen Formulierungen vorzugsweise für Pb < 20 ppm, Cd < 5 ppm, As < 5 ppm, Sb < 10 ppm, Hg < 1 ppm, Ni < 10 ppm.

Das unkeramisierte Ausgangsglas, das direkt in den bevorzugten erfindungsgemäßen Zusammensetzungen enthalten oder gegebenenfalls für die Herstellung einer erfindungsgemäß einsetzbaren Glaskeramik verwandt wird, enthält SiO₂ als Netzwerkbildner, vorzugsweise zwischen 35-80 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität abnehmen, und die Verarbeitungstemperatur wird deutlich erhöht, so daß sich die Heißformgebungseigenschaften verschlechtern. Na₂O wird als Flußmittel beim Schmelzen des Glases eingesetzt. Bei Konzentrationen kleiner 5% wird das Schmelzverhalten negativ beeinflußt. Natrium ist Bestandteil der sich bei der Keramisierung bildenden Phasen und muß, sofern hohe kristalline Phasenanteile durch die Keramisierung eingestellt werden sollen, in entsprechend hohen Konzentrationen im Glas enthalten sein. K₂O wirkt als Flußmittel beim Schmelzen des Glases. Außerdem wird Kalium in wässrigen Systemen abgegeben. Liegen hohe Kaliumkonzentrationen im Glas vor, werden kaliumhaltige Phasen wie Kalzium-Silicaten ebenfalls ausgeschieden. Über den P₂O₅-Gehalt kann bei silikatischen Gläsern, Glaskeramiken oder Kompositen die chemische Beständigkeit des Glases und damit die Ionenabgabe in wässrigen Medien eingestellt werden. Bei Phospahtgläsern ist P₂O₅ Netzwerkbilder. Der P₂O₅-Gehalt liegt vorzugsweise zwischen 0 und 80 Gew.-%. Um die Schmelzbarkeit zu verbessern, kann das Glas bis zu 25 Gew.- % B₂O₃ enthalten. Al₂O₃ wird genutzt, um die chemische Beständigkeit des Glases einzustellen.

Zur Verstärkung der antimikrobiellen, insbesondere der antibakteriellen Eigenschaften der Glaskeramik können antimikrobiell wirkende lonen wie z. B. Ag, Au, I, Ce, Cu, Zn in Konzentrationen kleiner 5 Gew.-% enthalten sein.

Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.

Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.

Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.- %, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al ₂O₃: 0-25 Gew.-% undB₂O₃: 0-25 Gew.-% .

Weiterhin können dem Grundglas gemäß obiger Zusammensetzung zur Erzielung weiterer Effekte wie beispielsweise Farbigkeit oder UV-Filterung lonen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO ₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K ₂O: 0-35 Gew.-%, ZnO: 0-10 Gew.-%, Al₂ O₃: 0-25 Gew.-%, B₂O₃: 0- 25 Gew.-%, SnO: 0-5 Gew.-%, CeO₂: 0-3 Gew.- % und Au: 0,001-0,1 Gew.-%.

Besonders bevorzugte erfindungsgemäße Mund- und Zahnpflege- und -reinigungsmittel sind dadurch gekennzeichnet, daß das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und - reinigungsmittel dadurch gekennzeichnet, daß das antimikrobielle Glas Teilchengrößen < 10 µm, vorzugsweise von 0,5 bis 4 µm, besonders bevorzugt von 1 bis 2 µm, aufweist.

Die nachfolgenden Beispiele dienen der Veranschaulichung der vorliegenden Erfindung:

### Beispiele:

Es wurden Zahnpasten folgender Zusammensetzung hergestellt:

| | **E1** | **E2** |
|---|---|---|
| Sorbit (70 % DAB) | 31,0 | 30,0 |
| Fällungskieselsäure : Sident 8 | 10,0 | 10,0 |
| Fällungskieselsäure : Sident 22S | 8,5 | 8,5 |
| Poliertonerde P10 feinst | - | 1,0 |
| Dinatriumphosphat, wasserfrei | 0,1 | 0,1 |
| Trinatriumphosphat, wasserfrei MgS04.7H2O | 0,2 | 0,2 |
| Na-Monofluorophosphat Na2PO3F | - | - |
| Natriumfluorid | 0,32 | 0,32 |
| Polyethylenglycol (MG : 1500) | 1,5 | 1,5 |
| Titandioxid | 0,5 | 0,5 |
| Na-Laurylsulfat | 1,5 | 1,5 |
| Saccharin-Na | 0,1 | 0,1 |
| Aroma | 1,2 | 1,2 |
| Jambu Oleoresin (30 % Spilanthol) | 0,1 | 0,02 |
| Wasser | ad 100 | ad 100 |
| Wasser | 54,28 | 54,06 |
| Sorbit (berechnet als 100 %) | 21,7 | 21,0 |
| Verhältnis Wasser/Sorbit | 2,46 | 2,53 |

| | **E5** | **E6** |
|---|---|---|
| Sorbit (70 % DAB) | 31,0 | 30,0 |
| Glycerin, wasserfrei | 5,0 | 7,5 |
| Ethanol (96%) | - | - |
| Fällungskieselsäure : Sident 8 | 10,0 | 10,0 |
| Fällungskieselsäure : Sident 22S | 8,5 | 8,5 |
| Poliertonerde P10 feinst | - | 1,0 |
| Dinatriumphosphat, wasserfrei | 0,1 | 0,1 |
| Trinatriumphosphat, wasserfrei MgSO4 . 7H2O | 0,2 | 0,2 |
| Na-Monofluorophosphat Na2PO3F | - | - |
| Natriumfluorid | 0,32 | 0,32 |
| Polyethylenglycol (MG : 1500) | 1,5 | 1,5 |
| Titandioxid | 0,5 | 0,5 |
| Na-Laurylsulfat | 1,5 | 1,5 |
| Saccharin-Na | 0,1 | 0,1 |
| Xanthan | 0,4 | 0,4 |
| Aroma | 1,2 | 1,2 |
| Jambu Oleoresin (30 % Spilanthol) | 0,1 | 0,02 |
| Wasser | ad 100 | ad 100 |
| Wasser | 48,88 | 46,16 |
| Sorbit (berechnet als 100 %) + Glycerin | 26,7 | 28,5 |
| Verhältnis Wasser/Sorbit | 1,80 | 1,58 |

Die erfindungsgemäßen Zusammensetzungen wurden von einem Verbraucherpanel aus 20 Verbrauchern als "angenehm prickelnd" bzw. "von warmer Schärfe" beschrieben. Ansonsten analog zusammengesetzte Zusammensetzungen, bei denen das Verhältnis von mehrwertigen Alkoholen zu Wasser < 1,5 : 1 war, wurden als sensorisch unbefriedigender bewertet.

## Patentansprüche

1. Mund- und Zahnpflege- und -reinigungsmittel, enthaltend
a) 12,5 bis 35 Gew.-% mindestens eines mehrwertigen Alkohols aus der Gruppe Sorbit und/oder Glycerin und/oder 1,2-Propylenglycol;
b) Wasser;
c) mindestens eine scharf schmeckende und/oder ein Gefühl von Wärme erzeugende Substanz aus der Gruppe der N-Alkyl-substituierte Amide von ungesättigten Carbonsäuren
d) 0,01 bis 5 Gew.% Fluorverbindung(en),
**dadurch gekennzeichnet, dass** das Gewichtsverhältnis von b) zu a) größer als 1,5 : 1 ist.

2. Mund- und Zahnpflege- und -reinigungsmittel gemäß Anspruch 1, **dadurch gekennzeichnet, dass** 15 bis 30 Gew.-% und insbesondere 20 bis 27,5 Gew.-% mindestens eines mehrwertigen Alkohols aus der Gruppe Sorbit und/oder Glycerin und/oder 1,2-Propylenglycol.-%, jeweils bezogen auf das Gewichts des gesamten Mittels, enthält.

3. Mund- und Zahnpflege- und -reinigungsmittel gemäß Anspruch 1 oder 2, **dadurch gekennzeichnet, dass** es 30 bis 70 Gew.-%, vorzugsweise 36 bis 65 Gew.-%, besonders bevorzugt 41 bis 60 Gew.-% und insbesondere 45 bis 55 Gew.-% Wasser.-%, jeweils bezogen auf das Gewichts des gesamten Mittels, enthält.

4. Mund- und Zahnpflege- und -reinigungsmittel gemäß einem der Ansprüche 1 bis 3, **dadurch gekennzeichnet, dass** es die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,5 Gew.-% und insbesondere von 0,01 bis 0,2 Gew.-%, jeweils bezogen auf das Gewichts des gesamten Mittels, enthält.

5. Mund- und Zahnpflege- und -reinigungsmittel gemäß Anspruch 4, **dadurch gekennzeichnet, dass** das eine N-Alkylsubstituierte Amid von ungesättigten Carbonsäuren
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. *N*-[2-(4-Hydroxy-3-methoxyphenyl)ethyl]-3-(4-hydroxy-3-methoxy-phenyl)- (2*E*)-propensäureamid (trans-Feruloylmethoxytyramin) und/oder
h. *N*-[2- (4-Hydroxy-3-methoxyphenyl)ethyl]-3-(4-hydroxy-3-methoxy-phenyl)-(2*Z*)-propensäureamid (cis-Feruloylmethoxytyramin) und/oder
i. N-[2-(4- Hydroxy-3-methoxyphenyl)ethyl]-3-(4-hydroxy-3-methoxy-phenyl)-propansäureamid (Dihydroferuloylmethoxytyramin) und/oder
j. *N*-[2-(3,4- Dihydroxyphenyl)ethyl]-3-(4-hydroxy-3-methoxy-phenyl)-(2*E*)-propensäureamid (trans-Feruloyldopamin) und/oder
k. *N*-[2-(3,4-Dihydroxyphenyl) ethyl]-3-(4-hydroxy-3-methoxy-phenyl)-(2*Z*)-propensäureamid (cis- Feruloyldopamin) und/oder
l. *N*-[2-(4-Hydroxyphenyl)ethyl]-3-(3,4- dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)- propensäureamid (cis-Caffeoyltyramin) und/oder
n. *N*-[2-(3,4-Dimethoxyphenyl) ethyl]-3-(3,4-dimethoxyphenyl)-(2*E*)-propensäureamid (trans- Rubenamin) und/oder
o. *N*-[2-(3,4-dimethoxyphenyl)ethyl]-3-(3,4- dimethoxy-phenyl)-(2*Z*)-propensäureamid (cis-Rubenamin)
ist.

6. Mund- und Zahnpflege- und -reinigungsmittel gemäß einem der Ansprüche 1 bis 5, **dadurch gekennzeichnet, dass** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ 2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

7. Mund- und Zahnpflege- und -reinigungsmittel nach einem der Ansprüche 1 bis 6, **dadurch gekennzeichnet, dass** es zusätzlich Phosphat(e), vorzugsweise Alkalimetallphosphat(e) und insbesondere Natriumtripolyphoyphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthält.

8. Mund- und Zahnpflege- und -reinigungsmittel nach einem der Ansprüche 1 bis 7, **dadurch gekennzeichnet, dass** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthält.

9. Mund- und Zahnpflege- und -reinigungsmittel nach einem der Ansprüche 1 bis 8, **dadurch gekennzeichnet, dass** es Fluorverbindung(en), insbesondere Natriumfluorid, Kaliumfluorid, Natriummonofluorphosphat, Zinkfluorid, Zinnfluorid und Natriumfluorosilikat, in Mengen von 0,1 bis 2,5 Gew.% und insbesondere von 0,2 bis 1,1 Gew.%, jeweils bezogen auf das gesamte Mittel, enthält.

10. Mund- und Zahnpflege- und -reinigungsmittel nach einem der Ansprüche 1 bis 9, **dadurch gekennzeichnet, dass** es die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthält.

11. Mund- und Zahnpflege- und -reinigungsmittel nach einem der Ansprüche 1 bis 10, **dadurch gekennzeichnet, dass** es 0,2 bis 20 Gew.-%, vorzugsweise 0,4 bis 14 Gew.%, besonders bevorzugt 0,5 bis 3 Gew.-% und insbesondere 0,6 bis 2 Gew.-% mindestens eines bioaktiven Glases enthält.

## Claims

1. Mouthwash and dentifrice and cleansing agent, comprising
a) 12.5 to 35 wt % of at least one polyhydric alcohol from the group sorbitol and/or glycerine and/or 1,2-propylene glycol;
b) water;
c) at least one pungent-tasting substance and/or substance that generates a sensation of warmth from the group of the *N*-alkyl-substituted amides of unsaturated carboxylic acids
d) 0.01 to 5 wt % of compound(s) that contain(s) fluorine,
**characterised in that** the weight ratio of b) to a) is greater than 1.5 : 1.

2. Mouthwash and dentifrice and cleansing agent according to claim 1, **characterised in that** it comprises 15 to 30 wt % and in particular 20 to 27.5 wt % of at least one polyhydric alcohol from the group sorbitol and/or glycerine and/or 1,2-propylene glycol, each relative to the weight of the total agent.

3. Mouthwash and dentifrice and cleansing agent according to claim 1 or 2, **characterised in that** it comprises 30 to 70 wt %, preferably 36 to 65 wt %, particularly preferably 41 to 60 wt % and in particular 45 to 55 wt % water, each relative to the weight of the total agent.

4. Mouthwash and dentifrice and cleansing agent according to one of claims 1 to 3, **characterised in that** it comprises the pungent-tasting substance(s) and/or substance(s) that generate(s) a sensation of warmth in amounts of 0.00001 to 5 wt %, preferably 0.001 to 1 wt %, particularly preferably 0.005 to 0.5 wt % and in particular 0.01 to 0.2 wt %, each relative to the weight of the total agent.

5. Mouthwash and dentifrice and cleansing agent according to claim 4, **characterised in that** the at least one *N*-alkyl-substituted amide of unsaturated carboxylic acids is
a. 2*E*,6*Z*,8*E*-decatrienoic acid *N*-isobutylamide (spilanthol) and/or
b. 2*E*,4*E*,8*Z*-decatrienoic acid *N*-isobutylamide and/or
c. 2*E*,7*Z*,9*E*-undecatrienoic acid *N*-isobutylamide and/or
d. 2*E*,4*Z*-decadienoic acid *N*-isobutylamide (*cis*-pellitorin) and/or
e. 2*E*,4*E*-decadienoic acid *N*-isobutylamide (*trans*-pellitorin) and/or
f. ferulic acid *N*-vanillylamide and/or
g. *N*-[2-(4-hydroxy-3-methoxyphenyl)ethyl]-3-(4-hydroxy-3-methoxyphenyl)-(2*E*)-propenoic acid amide (*trans*-feruloylmethoxytyramine) and/or
h. *N*-[2-(4-hydroxy-3-methoxyphenyl)ethyl]-3-(4-hydroxy-3-methoxyphenyl)-(2*Z*)-propenoic acid amide (*cis*-feruloylmethoxytyramine) and/or
i. *N*-[2-(4-hydroxy-3-methoxyphenyl)ethyl]-3-(4-hydroxy-3-methoxyphenyl)-propanoic acid amide (dihydroferuloylmethoxytyramine) and/or
j. *N*-[2-(3,4-dihydroxyphenyl)ethyl]-3-(4-hydroxy-3-methoxyphenyl)-(2*E*)-propenoic acid amide (*trans*-feruloyldopamine) and/or
k. *N*-[2-(3,4-dihydroxyphenyl)ethyl]-3-(4-hydroxy-3-methoxyphenyl)-(2*Z*)-propenoic acid amide (*cis*-feruloyldopamine) and/or
l. *N*-[2-(4-hydroxyphenyl)ethyl]-3-(3,4-dihydroxyphenyl)-(2*E*)-propenoic acid amide (*trans*-caffeoyltyramine) and/or
m. *N*-[2-(4-hydroxyphenyl)ethyl]-3-(3,4-dihydroxyphenyl)-(2*Z*)-propenoic acid amide (*cis*-caffeoyltyramine) and/or
n. *N*-[2-(3,4-dimethoxyphenyl)ethyl]-3-(3,4-dimethoxyphenyl)-(2*E*)-propenoic acid amide (*trans*-rubenamine) and/or
o. *N*-[2-(3,4-dimethoxyphenyl)ethyl]-3-(3,4-dimethoxyphenyl)-(2*Z*)-propenoic acid amide (*cis*-rubenamine).

6. Mouthwash and dentifrice and cleansing agent according to one of claims 1 to 5, **characterised in that** it additionally comprises abrasive agents, preferably silicic acids, aluminium hydroxide, aluminium oxide, calcium pyrophosphate, chalk, dicalcium phosphate dihydrate (CaHPO₄ 2H₂O), sodium aluminium silicates, in particular zeolite A, organic polymers, in particular polymethacrylates or mixtures of these friction agents, preferably in amounts of 1 to 30 wt %, preferably 2.5 to 25 wt % and in particular 5 to 22 wt %, each relative to the total agent.

7. Mouthwash and dentifrice and cleansing agent according to one of claims 1 to 6, **characterised in that** it additionally comprises phosphate(s), preferably alkali metal phosphate(s) and in particular sodium tripolyphosphate, preferably in amounts of 1 to 10 wt %, particularly preferably 2 to 8 wt % and in particular 3 to 7 wt %, each relative to the total agent.

8. Mouthwash and dentifrice and cleansing agent according to one of claims 1 to 7, **characterised in that** it additionally comprises anti-plaque active substances, preferably methyl, ethyl or propyl *p*-hydroxybenzoate, sodium sorbate, sodium benzoate, bromochlorophene, triclosan, phenyl salicylate, biguanidines e.g. chlorhexidine, thymol, preferably in amounts of 0.1 to 5 wt %, preferably 0.25 to 2.5 wt % and in particular 0.5 to 1.5 wt %, each relative to the total agent.

9. Mouthwash and dentifrice and cleansing agent according to one of claims 1 to 8, **characterised in that** it comprises compound(s) that contain(s) fluorine, in particular sodium fluoride, potassium fluoride, sodium monofluorophosphate, zinc fluoride, tin fluoride and sodium fluorosilicate, in amounts of 0.1 to 2.5 wt % and in particular 0.2 to 1.1 wt %, each relative to the total agent.

10. Mouthwash and dentifrice and cleansing agent according to one of claims 1 to 9, **characterised in that** it comprises substances that increase the insensitivity of the teeth, preferably potassium salts, particularly preferably potassium nitrate and/or potassium citrate and/or potassium chloride and/or potassium bicarbonate and/or potassium oxalate, preferably in amounts of 0.5 to 20 wt %, particularly preferably 1.0 to 15 wt %, further preferably 2.5 to 10 wt % and in particular 4.0 to 8.0 wt %, each relative to the total agent.

11. Mouthwash and dentifrice and cleansing agent according to one of claims 1 to 10, **characterised in that** it comprises 0.2 to 20 wt %, preferably 0.4 to 14 wt %, particularly preferably 0.5 to 3 wt % and in particular 0.6 to 2 wt % of at least one bioactive glass.

## Revendications

1. Agent de nettoyage et de soin dentaire et buccal, contenant
a) 12,5 à 35 % en poids d'au moins un alcool plurivalent choisi dans le groupe sorbitol et/ou glycérine et/ou 1,2-propylène glycol ;
b) de l'eau ;
c) au moins une substance au goût relevé et/ ou produisant une sensation de chaleur choisie dans le groupe des amides N-alkyl-substitués d' acides carboxyliques insaturés
d) 0,01 à 5 % en poids de composé(s) fluoré(s),
**caractérisé en ce que** le rapport massique de b) sur a) est supérieur à 1,5 : 1.

2. Agent de nettoyage et de soin dentaire et buccal selon la revendication 1, **caractérisé en ce qu'**il contient 15 à 30 % en poids et, notamment 20 à 27,5 % en poids d'au moins un alcool plurivalent choisi dans le groupe sorbitol et/ou glycérine et/ou 1,2-propylène glycol, le pourcentage étant à chaque fois rapporté au poids de l'agent dans sa globalité.

3. Agent de nettoyage et de soin dentaire et buccal selon les revendications 1 ou 2, **caractérisé en ce qu'**il contient 30 à 70 % en poids, notamment de 36 à 65 % en poids, de préférence de 41 à 60 % en poids et idéalement de 45 à 55 % en poids d'eau, le pourcentage étant à chaque fois rapporté au poids de l'agent dans sa globalité.

4. Agent de nettoyage et de soin dentaire et buccal selon l'une quelconque des revendications 1 à 3, **caractérisé en ce qu'**il contient la ou les substance(s) au goût relevé et/ ou produisant une sensation de chaleur en des quantités comprises dans une plage de 0,00001 à 5 % en poids, notamment de 0,0005 à 2,5 % en poids, de préférence de 0,001 à 1 % en poids, de manière particulièrement préférée de 0,005 à 0,5 % en poids et en particulier de 0,01 à 0,2 % en poids, le pourcentage étant à chaque fois rapporté au poids de l'agent dans sa globalité.

5. Agent de nettoyage et de soin dentaire et buccal selon la revendication 4, **caractérisé en ce que** le au moins un amide N-alkyl-substitué d'acides carboxyliques insaturés est
a. le N-isobutylamide de l'acide 2E,6Z,8E-décatriénoïque (spilanthol) et/ou
b. le N-isobutylamide de l'acide 2E,4E,8Z-décatriénoïque et/ou
c. le N-isobutylamide de l'acide 2E,7Z,9E-undécatriénoïque et/ou
d. le N-isobutylamide de l'acide 2E,4Z-décadiénoïque (cis-pellitorine) et/ou
e. le N-isobutylamide de l'acide 2E,4E-décadiénoïque (trans-pellitorine) et/ou
f. le N-vanillylamide d'acide férulique et/ou
g. l'amide d'acide N-[2-(4-hydroxy-3-méthoxyphényl)éthyl]-3-(4-hydroxy-3-méthoxy-phényl)-(2E)-propénoïque (trans-féruloylméthoxytyramine) et/ou
h. l'amide d'acide N-[2-(4-hydroxy-3-méthoxyphényl)éthyl]-3-(4-hydroxy-3-méthoxy-phényl)-(2Z)-propénoïque (cis-féruloylméthoxytyramine) et/ou
i. l'amide d'acide N-[2-(4-hydroxy-3-méthoxyphényl)éthyl]-3-(4-hydroxy-3-méthoxy-phényl)-propanoïque (Dihydroféruloylméthoxytyramine) et/ou
j. l'amide d'acide N-[2-(3,4-dihydroxyphényl)éthyl]-3-(4-hydroxy-3-méthoxy-phényl)-(2E)-propénoïque (trans-féruloyldopamine) et/ou
k. l'amide d'acide N-[2-(3,4-dihydroxyphényl)éthyl]-3-(4-hydroxy-3-méthoxy-phényl)-(2Z)-propénoïque (cis-féruloyldopamine) et/ou
l. l'amide d'acide N-[2-(4-hydroxyphényl)éthyl]-3-(3,4-dihydroxyphényl)-(2E)-propénoïque (trans-caféoyltyramine)
m. l'amide d'acide N-[2-(4-hydroxyphényl)éthyl]-3-(3,4-dihydroxyphényl)-(2Z)-propénoïque (cis-caféoyltyramine) et/ou
n. l'amide d'acide N-[2-(3,4-diméthoxyphényl)éthyl]-3-(3,4-diméthoxyphényl)-(2E)-propénoïque et/ou (trans-rubénamine) et/ou
o. l'amide d'acide N-[2-(3,4-diméthoxyphényl)éthyl]-3-(3,4-diméthoxyphényl)- (2Z)-propénoïque (cis-rubénamine).

6. Agent de nettoyage et de soin dentaire et buccal selon l'une quelconque des revendications 1 à 5, **caractérisé en ce qu'**il contient en plus des agents abrasifs, de préférence des acides siliciques, de l'hydroxyde d'aluminium, de l'oxyde d'aluminium, du pyrophosphate de calcium, de la craie, du dicalcium phosphate, dihydrate (CaHPO₄-2H₂O), des silicates d'aluminium et sodium, notamment de la zéolithe A, des polymères organiques, en particulier, des polyméthacrylates ou des mélanges de ces agents de friction, de préférence en des quantités comprises dans une plage de 1 à 30 % en poids, de préférence de 2,5 à 25 % en poids et en particulier de 5 à 22 % en poids, le pourcentage étant à chaque fois rapporté au poids de l'agent dans sa globalité.

7. Agent de nettoyage et de soin dentaire et buccal selon l'une quelconque des revendications 1 à 6, **caractérisé en ce qu'**il contient en plus du (des) phosphate(s), de préférence du(des) phosphate(s) de métal alcalin et notamment du tripolyphosphate de sodium, de préférence en des quantités comprises dans une plage de 1 à 10 % en poids, de manière particulièrement préférée, de 2 à 8 % en poids et en particulier de 3 à 7 % en poids, le pourcentage étant à chaque fois rapporté au poids de l'agent dans sa globalité.

8. Agent de nettoyage et de soin dentaire et buccal selon l'une quelconque des revendications 1 à 7, **caractérisé en ce qu'**il contient en plus des principes actifs antitartres, de préférence de l'ester méthylique, éthylique ou propylique de l'acide p-hydroxybenzoïque, du sorbate de sodium, du benzoate de sodium, du bromochlorophène, du triclosan, de l'ester phénylique de l'acide, salicylique, du biguanide par. ex. de la chlorhexidine, du thymol, de préférence, en des quantités comprises dans une plage de 0,1 à 5 % en poids, de préférence de 0,25 à 2,5 % en poids et en particulier de 0,5 à 1,5 % en poids, le pourcentage étant à chaque fois rapporté au poids de l'agent dans sa globalité.

9. Agent de nettoyage et de soin dentaire et buccal selon l'une quelconque des revendications 1 à 8, **caractérisé en ce qu'**il contient un (des) composé(s) fluoré(s), notamment du fluorure de sodium, du fluorure de potassium, du monofluorophosphate de sodium, du fluorure de zinc, du fluorure d'étain et du fluorosilicate de sodium en des quantités comprises dans une plage de 0,1 à 2,5 % en poids et notamment de 0,2 à 1,1 % en poids, le pourcentage étant à chaque fois rapporté au poids de l'agent dans sa globalité.

10. Agent de nettoyage et de soin dentaire et buccal selon l'une quelconque des revendications 1 à 9, **caractérisé en ce qu'**il contient en plus des substances contre l'hypersensibilité des dents, de préférence des sels de potassium, notamment du nitrate de potassium, et/ou du citrate de potassium et/ou du chlorure de potassium et/ou du bicarbonate de potassium et/ou de l'oxalate de potassium, de préférence en des quantités de 0,5 à 20 % en poids, de manière particulièrement préférée de 1,0 à 15 % en poids, et de manière d'avantage de 2,5 à 10 % en poids et en particulier de 4,0 à 8,0 % en poids, le pourcentage étant à chaque fois rapporté au poids de l'agent dans sa globalité.

11. Agent de nettoyage et de soin dentaire et buccal selon l'une quelconque des revendications 1 à 10, **caractérisé en ce qu'**il contient 0,2 à 20 % en poids, notamment de 0,4 à 14 % en poids, de manière particulièrement préférée de 0,5 à 3 % en poids et en particulier de 0,6 à 2 % en poids au moins d'un verre bioactif.
